(19) Europäisches Patentamt
European Patent Office
Office européen des brevets

(11) **EP 4 431 491 A1**

(12) **EUROPEAN PATENT APPLICATION**
published in accordance with Art. 153(4) EPC


(43) Date of publication:
**18.09.2024 Bulletin 2024/38**

(21) Application number: **22890078.3**

(22) Date of filing: **08.11.2022**

(51) International Patent Classification (IPC):
***C07C 265/14*** *(2006.01)* ***C07C 269/00*** *(2006.01)*
***C07C 269/08*** *(2006.01)* ***C07C 271/52*** *(2006.01)*

(52) Cooperative Patent Classification (CPC):
**C07C 271/66; C07C 265/14;** C07C 2601/14

(86) International application number:
**PCT/JP2022/041614**

(87) International publication number:
**WO 2023/080258 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.11.2021 JP 2021182189**
**08.11.2021 JP 2021182192**



(71) Applicant: **Asahi Kasei Kabushiki Kaisha**
**Tokyo 1000006 (JP)**

(72) Inventor: **NAKAOKA Koichi**
**Tokyo 100-0006 (JP)**

(74) Representative: **D Young & Co LLP**
**3 Noble Street**
**London EC2V 7BQ (GB)**


# (54) CARBONYL COMPOUND, METHOD FOR PRODUCING CARBONYL COMPOUND, METHOD FOR PRODUCING ISOCYANATE COMPOUND, AND ISOCYANATE COMPOSITION


(57) The present invention provides a carbonyl compound represented by General Formula (I) (in General Formula (I), $R^{11}$ is an (n11 + n12)-valent organic group, and $R^{12}$ is a monovalent organic group, n11 is an integer of 1 or more and 8 or less, n12 is an integer of 0 or more and 7 or less, and a sum of n11 and n12 is an integer of 2 or more and 8 or less).

[Chem. 1]

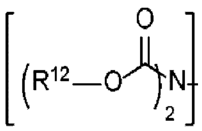


*FIG. 1*

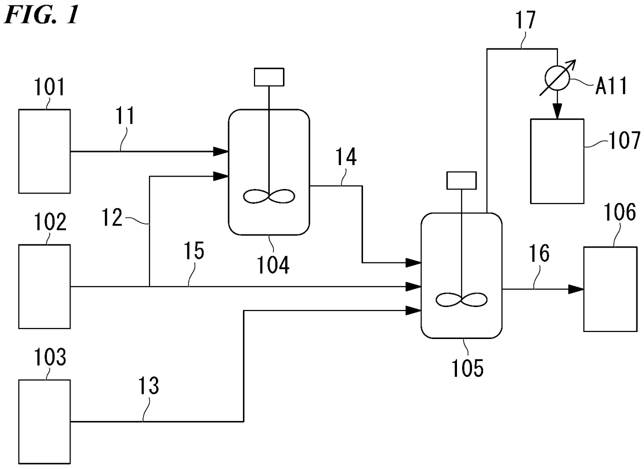


EP 4 431 491 A1

## Description

[Technical Field]

The present invention relates to a carbonyl compound, a method for producing a carbonyl compound, a method for producing an isocyanate compound, and an isocyanate composition.

[Background Art]

An isocyanate has been widely used as a raw material for producing polyurethane foams, coating materials, adhesives, and the like. A major industrial production method for an isocyanate is a reaction between an amine compound and phosgene (phosgene method), and almost all of isocyanates produced in the world are produced by the phosgene method. However, the phosgene method has many problems.

First, a large amount of phosgene is used as a raw material. Phosgene is extremely toxic, and thus special caution is required in handling thereof to prevent workers from being exposed thereto, and a special device for detoxifying the waste is also required. Second, in the phosgene method, since a large amount of highly corrosive hydrogen chloride is produced as a by-product, a process for detoxifying the hydrogen chloride is required, and furthermore, the produced isocyanate contains hydrolyzable chlorine in many cases. Therefore, in a case where an isocyanate produced by the phosgene method is used, there is a case where atmospheric corrosion resistance and heat resistance of the polyurethane product are adversely affected.

From such a background, a method for producing an isocyanate compound which does not use phosgene is desired. As one of the methods for producing an isocyanate compound without using phosgene, a method utilizing pyrolysis of a carbamate compound has been suggested. It has been known for a long time that an isocyanate and a hydroxy compound can be obtained by pyrolysis of a carbamate compound (for example, refer to Non-Patent Document 1). The basic reaction is exemplified by Formula (A).

[Chem. 1]

$$R(NHCOOR')a \rightarrow R(NCO)a + a\ R'OH \qquad (A)$$

(In Formula (A), R is an a-valent organic group, R' is a monovalent organic group, and a is an integer of 1 or more.)

On the other hand, in the pyrolysis reaction of a carbamate compound, various irreversible side reactions such as an unpreferable thermal denaturation reaction of the carbamate compound and a condensation reaction of an isocyanate generated by the pyrolysis are likely to occur together. Examples of the side reaction include a reaction of forming an isocyanurate group represented by Formula (B), a reaction of generating carbodiimides represented by Formula (C), a reaction of forming a uretonimine group represented by Formula (D), a reaction of forming an allophanate group represented by Formula (E), and the like (for example, refer to Non-Patent Documents 1 to 4 and the like).

[Chem. 2]

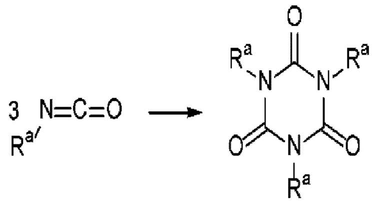
(B)

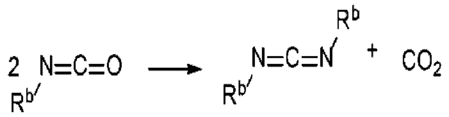
(C)

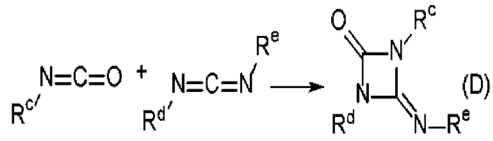
(D)

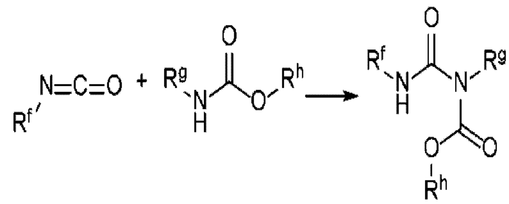
(E)

(In Formulae (B) to (E), $R^a$ to $R^h$ each independently is a monovalent organic group.)

A polyurethane having a urethane bond is mainly produced by a reaction between a bifunctional or higher functional isocyanate and a bifunctional or higher functional alcohol, is a polymer excellent in tensile strength, abrasion resistance, and oil resistance, and is used in a wide range of fields such as a soft foam, a hard foam, an elastomer, an adhesive, a coating material, and a binder. Among these, a polyurethane for which a chain-like or cyclic aliphatic isocyanate is used as a raw material has excellent atmospheric corrosion resistance and light resistance, and is used in fields where appearance quality of a baking coating material, an automobile clear coating material, a coil coating material, and the like is required.

There is a case where a diisocyanate, which is a bifunctional isocyanate is used as the isocyanate, but for the purpose of ensuring the safety of a handling operator by improving physical properties of polyurethane and suppressing a steam pressure, there is a case where a diisocyanate is polymerized by the reaction represented by Formulae (a) to (c) and is used as the isocyanate polymer, for example. In the formula, R represents a divalent organic group, and R' represents a trivalent organic group.

[Chem. 3]

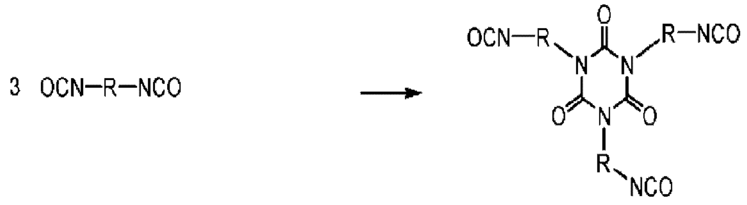

(a)

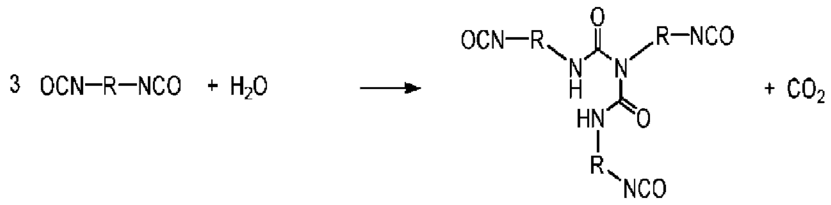

(b)

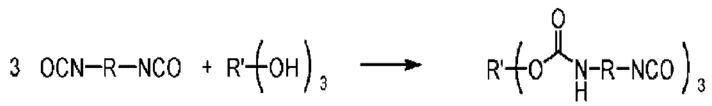

(c)

An isocyanurate type isocyanate polymer is obtained in the reaction represented by Formula (a), a biuret type isocyanate polymer is obtained in the reaction represented by Formula (b), and a urethane type isocyanate polymer is obtained in the reaction represented by Formula (c). The isocyanurate type isocyanate polymer is disclosed in Patent Documents 1 to 4. The biuret type isocyanate polymer is disclosed in Patent Documents 5 to 10. The allophanate type isocyanate polymer is disclosed in Patent Documents 11 and 12.

As described above, in a case where polyurethane is used in a field where appearance quality is required, polyurethane is required to have less coloring. For that purpose, it is important not only that there is no coloration in the polyurethanization reaction, but also that the raw material isocyanate (isocyanate with two or more functional groups) has little coloration. However, generally, the isocyanate tends to be oxidized by oxygen or the like in the air to be degenerated or colored easily. In addition, even in a case where an isocyanate polymer is produced by polymerization of a diisocyanate, the isocyanate tends to be colored due to a catalyst or solvent used in the polymerization reaction.

As a method of suppressing the coloring of an isocyanate, there is a method of producing an isocyanate by sealing the isocyanate with nitrogen gas and blocking the air, and storing thereof, or a method of storing an isocyanate by adding an ultraviolet absorber, an antioxidant, and the like. For example, Patent Document 13 discloses a method of denaturing an isocyanate and then treating the isocyanate with a peroxide to produce a polyisocyanate for a light-colored polyurethane lacquer. In addition, Patent Document 14 examines a method for producing an isocyanate with reduced coloring by bringing an ozone-containing gas into contact with the colored isocyanate. In addition, Patent Document 15 also examines a method for producing an isocyanate with reduced coloring by irradiating a colored isocyanate with light having a wavelength of 200 to 600 nm.

[Citation List]

[Patent Documents]

[Patent Document 1]
Specification of United States Patent No. 4324879
[Patent Document 2]
Specification of United States Patent No. 4412073
[Patent Document 3]
Japanese Unexamined Patent Application, First Publication No. S57-047319
[Patent Document 4]
Japanese Unexamined Patent Application, First Publication No. S63-057577
[Patent Document 5]

Specification of United States Patent No. 3976622
[Patent Document 6]
Specification of United States Patent No. 4176132
[Patent Document 7]
Specification of United States Patent No. 4290969
[Patent Document 8]
Specification of United States Patent No. 4837359
[Patent Document 9]
Specification of United States Patent No. 4983762
[Patent Document 10]
Specification of United States Patent No. 5641851
[Patent Document 11]
Specification of United Kingdom Patent No. 994890
[Patent Document 12]
Japanese Unexamined Patent Application, First Publication No. H07-304724
[Patent Document 13]
Japanese Unexamined Patent Application, First Publication No H02-228317
[Patent Document 14]
Japanese Unexamined Patent Application, First Publication No. H08-291129
[Patent Document 15]
Published Japanese Translation No. 2012-506465 of the PCT International Publication

[Non-Patent Documents]

[Non-Patent Document 1]
Dritter Jahrgang, "186. A. W. Hofmann: Ueber die aromatischen Cyanate.", Berchte der Deutechen Chemischen Gesellschaft, Vol. 3, pp. 653-658, 1870.
[Non-Patent Document 2]
Dyer E et al., "Thermal Degradation of Alkyl N-Phenylcarbomates.", Journal of American Chemical Society, Vol. 81, pp. 2138-2143, 1959.
[Non-Patent Document 3]
Ulrich H et al., "[2+2] Cycloaddition Reactions of Unsymmetrically substituted Carbodiimides.", Journal of Heterocyclic Chemistry, Vol. 24, pp. 1121-1123, 1987.
[Non-Patent Document 4]
Schwetlick K et al., "Kinetics and Catalysis of Consecutive Isocyanate Reactions. Formation of Carbamates, Allophanates and Isocyanurates.", Journal of the Chemical Society, Perkin transactions II, Vol. 2, pp. 395-402, 1995.

[Summary of Invention]

[Technical Problem]

The above-described side reaction causes a decrease in the yield or selection rate of a desired isocyanate compound, and also there is a case where long-term operation becomes difficult due to precipitation of a polymer-like solid substance, clogging of a reactor, and the like particularly in the production of a polyisocyanate. In addition, it is not known whether a compound having a boiling point higher than that of the isocyanate compound, in addition to the compound generated by the above-described side reaction, is produced as a by-product.

The present invention has been made in view of the above-described circumstances, and provides a novel carbonyl compound, a method for producing thereof, and a method for producing an isocyanate compound using the carbonyl compound.

In addition, as described above, various methods have been examined in order to suppress the coloring of the isocyanate, and in the method of storing by adding a compound that is not necessary for a polymerization reaction to an isocyanate, there is a case where the added compound can be a cause of coloring during production of polyurethane and the like.

In addition, in the methods disclosed in Patent Documents 4 to 6, it cannot be said that the coloring is necessarily sufficiently reduced, and there is a demand for an isocyanate with a further reduced coloring. In addition, distillation purification is a common method of purifying a compound, but since the isocyanate is heated during the distillation

purification, there is a case where the coloring of the isocyanate proceeds or denaturation of the isocyanate occurs.

The present invention has been made in view of the above-described circumstances, and provides an isocyanate composition in which coloring is sufficiently suppressed and storage stability is improved.

[Solution to Problem]

That is, the present invention includes the following aspects.

(1) A carbonyl compound represented by General Formula (I).

[Chem. 4]

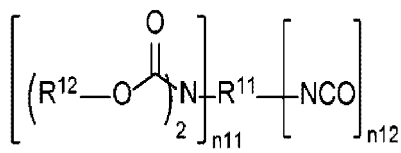

(I)

(In General Formula (1), $R^{11}$ is an (n11 + n12)-valent organic group, and $R^{12}$ is a monovalent organic group. n11 is an integer of 1 or more and 8 or less, n12 is an integer of 0 or more and 7 or less, and a sum of n11 and n12 is an integer of 2 or more and 8 or less).

(2) The carbonyl compound according to (1), in which $R^{11}$ is a di- or higher valent and tetra- or lower valent aliphatic hydrocarbon group having 1 or more and 20 or less carbon atoms, or a di- or higher valent and tri- or lower valent aromatic hydrocarbon group having 6 or more and 20 or less carbon atoms, which may have 1 or more and 4 or less ester groups or nitrogen atoms, and

$R^{12}$ is a monovalent aromatic hydrocarbon group having 6 or more and 20 or less carbon atoms, which may contain an oxygen atom.

(3) The carbonyl compound according to (1) or (2), in which $R^{11}$ is a di- or higher valent and tetra- or lower valent aliphatic hydrocarbon group having 5 or more and 15 or less carbon atoms, or a di- or higher valent and tri- or lower valent aromatic hydrocarbon group having 6 or more and 15 or less carbon atoms, which may have 1 or more and 2 or less ester groups,

$R^{12}$ is a monovalent aromatic hydrocarbon group having 6 or more and 15 or less carbon atoms, which may contain an oxygen atom,
$n^{11}$ is an integer of 1 or more and 4 or less,
$n^{12}$ is an integer of 0 or more and 3 or less, and
a sum of $n^{11}$ and $n^{12}$ is an integer of 2 or more and 4 or less.

(4) A method for producing the carbonyl compound according to any one of (1) to (3), the production method including:

mixing one or more compounds selected from the group consisting of an isocyanate compound and a carbamate compound with
one or more compounds selected from the group consisting of a carbonate and a hydroxy compound, and
heating the mixture to synthesize the carbonyl compound.

(5) The production method according to (4), in which the isocyanate compound is a compound represented by General Formula (II).

[Chem. 5]

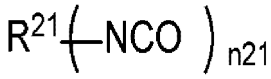

(II)

(In General Formula (II), $R^{21}$ is an n21-valent organic group, and satisfies a relational formula: $R^{21} = R^{11}$. n21 is an integer of 2 or more and 8 or less, and satisfies a relational formula: n21 = n11 + n12.)
(6) The production method according to (4) or (5), in which the carbamate compound is a compound represented by General Formula (III).

[Chem. 6]

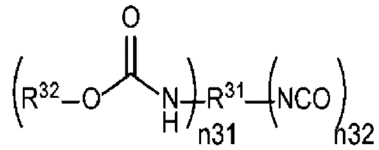
(III)

(In General Formula (III), $R^{31}$ is an (n31 + n32)-valent organic group, and satisfies a relational formula: $R^{31} = R^{11}$. $R^{32}$ is a monovalent organic group, and satisfies a relational formula: $R^{32} = R^{12}$. n31 is an integer of 1 or more and 8 or less, n32 is an integer of 0 or more and 7 or less, and a sum of n31 and n32 is an integer of 2 or more and 8 or less, and satisfies a relational formula: n31 + n32 = n11 + n12.)
(7) The production method according to any one of (4) to (6), in which the carbonate is a compound represented by General Formula (IV).

[Chem. 7]

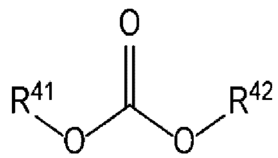
(IV)

(In General Formula (IV), $R^{41}$ and $R^{42}$ are each independently a monovalent organic group, and satisfy a relational formula: $R^{41} = R^{42} = R^{12}$.)
(8) The production method according to any one of (4) to (7), in which the hydroxy compound is a compound represented by General Formula (V).
[Chem. 8]

$R^{51}$-OH (V)

(In General Formula (V), $R^{51}$ is a monovalent organic group, and satisfies a relational formula: $R^{51} = R^{12}$.)
(9) A method for producing an isocyanate compound, the method including: performing distillation purification on a reaction solution including an isocyanate compound represented by General Formula (II) in the presence of the carbonyl compound according to any one of (1) to (3), and continuously recovering the isocyanate compound as a gas-phase component.

[Chem. 9]

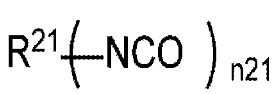
(II)

(In General Formula (II), $R^{21}$ is an n21-valent organic group, and satisfies a relational formula: $R^{21} = R^{11}$. n21 is an integer of 2 or more and 8 or less, and satisfies a relational formula: n21 = n11 + n12.)

(1) An isocyanate composition containing, with respect to a total mass of the isocyanate composition,

97% by mass or more of an isocyanate compound, and
2.0 ppm by mass or more and $1.0 \times 10^4$ ppm by mass or less of a carbonyl compound represented by General

Formula (I),
in which
the isocyanate compound and the carbonyl compound are different compounds from each other.

[Chem. 10]

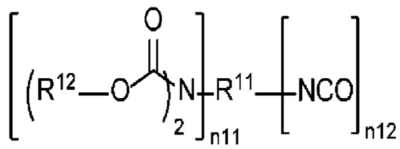

(I)

(In General Formula (I), $R^{11}$ is an (n11 + n12)-valent organic group, and $R^{12}$ is a monovalent organic group. n11 is an integer of 1 or more and 8 or less, n 12 is an integer of 0 or more and 7 or less, and a sum of n11 and n12 is an integer of 2 or more and 8 or less).

(2) The isocyanate compound according to (1), in which $R^{11}$ is a di- or higher valent and tetra- or lower valent aliphatic hydrocarbon group having 1 or more and 20 or less carbon atoms, or a di- or higher valent and tri- or lower valent aromatic hydrocarbon group having 6 or more and 20 or less carbon atoms, which may have 1 or more and 4 or less ester groups or nitrogen atoms, and
$R^{12}$ is a monovalent aromatic hydrocarbon group having 6 or more and 20 or less carbon atoms, which may contain an oxygen atom.
(3) The isocyanate composition according to (1) or (2), in which the isocyanate compound is a compound represented by General Formula (II).

[Chem. 11]

$$R^{21}\left(-NCO\right)_{n21} \quad (II)$$

(In General Formula (II), $R^{21}$ is an n21-valent organic group, and satisfies a relational formula: $R^{21} = R^{11}$, n21 is an integer of 2 or more and 8 or less, and satisfies a relational formula: n21 = n11 + n12.)
(4) The isocyanate composition according to any one of (1) to (3), further containing 2.0 ppm by mass or more and $1.0 \times 10^4$ ppm by mass or less of one or more compounds selected from the group consisting of a carbamate compound and a carbonate with respect to the total mass of the isocyanate composition.
(5) The isocyanate composition according to (4), in which the carbamate compound is a compound represented by General Formula (III).

[Chem. 12]

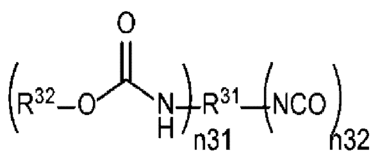

(III)

(In General Formula (III), $R^{31}$ is an (n31 + n32)-valent organic group, and satisfies a relational formula: $R^{31} = R^{11}$. $R^{32}$ is a monovalent organic group, and satisfies a relational formula: $R^{32} = R^{12}$. n31 is an integer of 1 or more and 8 or less, n32 is an integer of 0 or more and 7 or less, and a sum of n31 and n32 is an integer of 2 or more and 8 or less, and satisfies a relational formula: n31 + n32 = n11 + n12.)
(6) The isocyanate composition according to (4) or (5), in which the carbonate is a compound represented by General Formula (IV).

[Chem. 13]

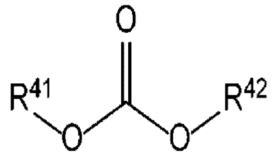

(IV)

(In General Formula (IV), $R^{41}$ and $R^{42}$ are each independently a monovalent organic group, and satisfy a relational formula: $R^{41} = R^{42} = R^{12}$.)

[Advantageous Effects of Invention]

According to the carbonyl compound and the method for producing thereof of the above-described aspect, it is possible to provide a novel carbonyl compound. The method for producing an isocyanate compound of the above-described aspect is a method using the carbonyl compound, and thus it is possible to prevent a by-product during the production of the isocyanate compound from being fixed to a device and to improve a yield of the isocyanate compound.

In addition, according to the isocyanate composition of the above-described aspect, it is possible to provide an isocyanate composition in which coloring is sufficiently suppressed and the storage stability is excellent.

[Brief Description of Drawings]

FIG. 1 is a schematic configuration diagram showing a device for producing a carbamate compound used in examples.
FIG. 2 is a schematic configuration diagram showing a pyrolysis reaction device used in examples.
FIG. 3 is a schematic configuration diagram showing a light-boiling separator used in examples.
FIG. 4 is a schematic configuration diagram showing a high-boiling separator used in examples.
FIG. 5 is a graph showing NMR spectra of carbamate compounds (I-1a) to (I-1c) produced in Example 1-1.
FIG. 6A is a graph showing NMR spectra of carbamate compounds (I-3a) to (I-3c) produced in Example 1-7.
FIG. 6B is a graph showing results of gas chromatography-mass spectrometry of carbamate compounds (I-3a) to (I-3c) produced in Example 1-7.

[Description of Embodiments]

A suitable embodiment of the present invention will be described below. The present invention is not limited to the following embodiments, and can be variously modified and implemented within the scope of the the invention.

In the present specification, in a case where the IUPAC regulation and the Nomenclature regulation stipulated by IUPAC also shown below (excluding cases particularly citing IUPAC recommendations or the like of other years) are indicated, it means citing "Organic Chemistry and Biochemical Naming Method" (Revised Second Edition, 1992, Published by Nankodo Co., Ltd., Japan) in which an edition including all of organic chemistry and biochemistry regulations published as a supplement of "Chemistry Field" in 1980 and all Japanese translated regulations is added with all revisions and recommendations thereafter, based on Recommendations 1979. The term "organic" refers to a general group of compounds as a subject of a naming method disclosed in the naming method. The subject may be a subject described in recommendations issued in 1993. However, the "organic" compounds as a subject of Nomenclature also include organometallic compounds and metal complexes. In the present embodiment, unless otherwise specified, the terms "organic group", "substituent", and the like mean a group formed of atoms not including metal atoms and/or metalloids. In addition, in the present embodiment, preferably, "organic compound", "organic group", or "substituent" formed of an atom selected from H (hydrogen atom), C (carbon atom), N (nitrogen atom), O (oxygen atom), S (sulfur atom), Cl (chlorine atom), Br (bromine atom), and 1 (iodine atom) is used.

In the following description, the terms "aliphatic" and "aromatic" are frequently used. According to the above-described IUPAC regulations, organic compounds are classified into aliphatic compounds and aromatic compounds. The aliphatic compound is defined as a group according to the aliphatic compound based on the 1995 IUPAC recommendation. In the recommendation, the aliphatic compound is defined as "acyclic or cyclic, saturated or unsaturated carbon compounds, excluding aromatic compounds". In addition, the "aliphatic compound" used in the description of the present embodiment is a compound containing all of saturated and unsaturated, and chain-like and cyclic compounds, and refers to the above-described "organic compound", "organic group", or "substituent" formed of an atom selected

from the group consisting of halogen atoms of H (hydrogen atom); C (carbon atom); N (nitrogen atom); O (oxygen atom; S (sulfur atom); Si (silicon atom); Cl (chlorine atom), Br (bromine atom), or I (iodine atom).

In a case where an aromatic group such as aralkyl group is bound to an aliphatic group, there is a case where this is denoted as "aliphatic group substituted with an aromatic group" or "group consisting of an aliphatic group to which an aromatic group is bound" in that way. This is based on the reactivity in the present embodiment, and this is because the properties of a group such as an aralkyl group relating to the reaction are extremely similar to the aliphatic reactivity, not to the aromatic reactivity. In addition, there is a case where a non-aromatic reactive group that includes an aralkyl group, an alkyl group, and the like is denoted as "aliphatic group which may be substituted with an aromatic group", "aliphatic group to which an aromatic group may be bound", or the like.

In a case of describing a general formula of the compound used in the present specification, the definition according to the above-described Nomenclature regulation of the IUPAC is used, but there is a case where common names are used for the name of a specific group and the name of the exemplified compound. In addition, in a case where the number of atoms, the number of substituents, and the number are described in the present specification, these all represent an integer.

In the present specification, the "active hydrogen" refers to a hydrogen atom (excluding aromatic hydroxy group) to which an oxygen atom, a sulfur atom, a nitrogen atom, a silicon atom, or the like is bound, and a hydrogen atom of a terminal methine group. The "active hydrogen" is a hydrogen contained in an atomic group such as -OH group, -C (= O) OH group, -C (= O) H group, -SH group, -SO3H group, -SO2H group, - SOH group, -NH2 group, -NH- group, -SiH group, and -C=CH group.

In addition, examples of the compound having a hydroxy group (-OH group) include an alcohol and an aromatic hydroxy compound.

The "alcohol" in the present specification is "compounds to which a hydroxy group is bound to a saturated carbon atom (compounds in which a hydroxy group, -OH, is attached to a saturated carbon atom: R3COH)", described in the IUPAC definition (Rule C-201), and does not include an aromatic hydroxy compound to which a hydroxy group is bound to an aromatic ring.

The "aromatic hydroxy compound" in the present specification is phenols described in the IUPAC definition (Rule C-202), "compounds having one or more hydroxy groups attached to a benzene or other arene ring)".

<<carbonyl compound>>

The carbonyl compound of the present embodiment is a compound represented by General Formula (I) (hereinafter, referred to as "carbonyl compound (I)" in some cases).

[Chem. 14]

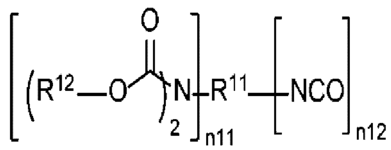

(I)

(In General Formula (1), $R^{11}$ is an (n11 + n12)-valent organic group, and $R^{12}$ is a monovalent organic group. n11 is an integer of 1 or more and 8 or less, n12 is an integer of 0 or more and 7 or less, and a sum of n11 and n12 is an integer of 2 or more and 8 or less).

The inventors of the present invention found that the carbonyl compound (I) is generated in the production of an isocyanate compound by a pyrolysis reaction of a carbamate compound. In addition, the inventors found that in a case of producing an isocyanate compound from a carbamate compound by pyrolysis, by causing carbonyl compound (I) to coexist, it is possible to prevent a by-product having a higher boiling point than that of the isocyanate compound (hereinafter, referred to as "by-product having high-boiling point" in some cases) from being fixed to a device in the pyrolysis step or purification step, to efficiently recover the isocyanate compound, and to improve a yield of the isocyanate compound, thereby completing the present invention.

[$R^{11}$]

$R^{11}$ is an (n11 + n12)-valent organic group, that is, a di- or higher valent and octa- or lower valent organic group. Among these, $R^{11}$ is preferably a di- or higher valent and tetra- or lower valent aliphatic hydrocarbon group having 1 or more and 20 or less carbon atoms, or di- or higher valent and tri- or lower valent aromatic hydrocarbon group having 6

or more and 20 or less carbon atoms, which may have 1 or more and 4 or less ester groups or nitrogen atoms.

Examples of the aliphatic hydrocarbon group in $R^{11}$ preferably include an alkylene group, an alkanetriyl group, a cycloalkyl group, a cycloalkylene group, or a cycloalkanetriyl group, or a group formed of the alkyl group, the alkylene group, the alkanetriyl group, the cycloalkyl group,the cycloalkylene group, or the cycloalkanetriyl group, and more preferably include a linear or branched alkylene group or alkanetriyl group, a cycloalkylene group, or a cycloalkanetriyl group, or a group formed of the alkylene group, the alkanetriyl group, the cycloalkyl group, the cycloalkylene group, or the cycloalkanetriyl group.

Examples of the linear or branched alkylene group include a methylene group, an ethylene group, a propylene group, a trimethylene group, a pentylene group, an n-hexylene group, a decamethylene group, and the like.

Examples of the cycloalkylene group include a cyclobutylene group, a cyclohexene group, and the like.

Examples of the linear or branched alkanetriyl group include a hexanetriyl group, a nonanetriyl group, a decantriyl group, and the like.

Examples of the cycloalkanetriyl group include a cyclopropanetriyl group, a cyclobutanetriyl group, a cyclopentanetriyl group, a cyclohexanetriyl group, and the like.

The aromatic hydrocarbon group in $R^{11}$ is preferably a group having a substituted or unsubstituted aromatic ring having 6 or more and 13 or less carbon atoms. Examples of the substituent include an alkyl group, an aryl group, an aralkyl group, and the like. The aromatic ring may be an aromatic hydrocarbon ring, or may be a heteroaromatic ring, and specific examples thereof include a benzene ring, a naphthalene ring, a pyridine ring, and the like.

In a case where the aliphatic hydrocarbon group or the aromatic group in $R^{11}$ has 1 or more and 4 or less ester groups, $R^{11}$ is preferably a group obtained by removing a primary amino group at a terminal of an amine compound having an ester group obtained by reacting a carboxy group of an amino acid with a hydroxy compound.

Specific examples of the amine compound having an ester group referred to herein include acrylic acid-2-aminoethyl ester, 2-methyl-acrylic acid-2-aminoethyl ester, acrylic acid-2-aminopropyl ester, 2-methyl-acrylic acid-2-aminopropyl ester, acrylic acid-3-aminopropyl ester, 2-methyl-acrylic acid-3-aminopropyl ester, acrylic acid-4-aminobutyl ester, 2-methyl-acrylic acid-4-aminobutyl ester, acrylic acid-5-aminopentyl ester, 2-methyl-acrylic acid-5-aminopentyl ester, acrylic acid-6-aminohexyl ester, 2-methyl-acrylic acid-6-aminohexyl ester, acrylic acid-8-aminoctyl ester, 2-methyl-acrylic acid-8-aminoctyl ester, acrylic acid-10-aminodecyl ester, 2-methyl-acrylic acid-10-aminodecyl ester, acrylic acid-11-aminondecyl ester, 2-methyl-acrylic acid-11-aminodecyl ester, acrylic acid-12-aminododecyl ester, 2-methyl-acrylic acid-12-aminododecyl ester, lysine methyl ester diamine, lysine ethyl ester diamine, 2-aminoethyl-2,5-diaminopentanoate, 2-aminoethyl-2,6-diaminohexanoate, bis(2-aminoethyl)-2-aminobutanedioate, bis(2-aminoethyl)-2-aminopentanedioate, tris(2-aminoethyl)hexane-1,3,6-tricarboxylate, and the like.

Examples of the amino acid used in the production of the amine compound having an ester group include lysine, alanine, arginine, asparagine, glutamine, glycine, aspartic acid, glutamic acid, ornithine, histidine, isoleucine, leucine, methionine, phenylalanine, tryptophan, valine, and the like. Among these, as the amino acid, lysine, arginine, glycine, aspartic acid, glutamic acid, or ornithine is preferable, and lysine, arginine, glycine, aspartic acid, or glutamic acid is more preferable.

Examples of the hydroxy compound used in the production of the amine compound having an ester group include alcohols and an aromatic hydroxy compound.

Examples of the alcohols include methyl alcohol, ethanol, propyl alcohol, butyl alcohol, amyl alcohol, hexyl alcohol, heptyl alcohol, octyl alcohol, nonyl alcohol, decyl alcohol, undecyl alcohol, lauryl alcohol, dotesyl alcohol, stearyl alcohol, eicosyl alcohol, allyl alcohol, crotyl alcohol, propargyl alcohol,cyclopentanol, cyclohexanol, benzyl alcohol, cinnamyl alcohol, ethylene glycol, diethylene glycol, polyethylene glycol, propylene glycol, polypropylene glycol, 1,3-butanediol, 1,4-butanediol, hydrogenated bisphenol A, neopentyl glycol, glycerin, trimethylolpropane, pentaerythritol, ethanolamine, propanolamine (1-amino-2-propanol), dimethanolamine, diethanolamine, dipropanolamine, 1-amino-2-butanol, and the like.

Examples of the aromatic hydroxy compound include monophenols such as phenol (cobaltic acid), 2-methoxyphenol, cresol, xylenol, carbachlor, motyl, naphthol, polyhydric phenols such as catechol, resorcin, hydroquinone, bisphenol A, bisphenol F, pyrogallol, and floroglucinol.

In a case where the aliphatic hydrocarbon group or the aromatic group in $R^{11}$ has 1 or more and 4 or less nitrogen atoms, $R^{11}$ is preferably an organic group obtained by removing an amino group at a terminal from an amine compound having 1 or more and 4 or less secondary or tertiary amines other than a terminal of an aliphatic hydrocarbon group or an aromatic group. Specific examples of the amine compound having 1 or more and 4 or less secondary or tertiary amines other than the terminal of the aliphatic hydrocarbon group or the aromatic group referred to herein include 2-(dimethylamino)ethyleneamine, 2-(diethylamino)ethyleneamine, 2-(diisopropylamino)ethyleneamine, 2-(cyclohexylamino)ethyleneamine, 3-(cyclohexylamino)propylamine, 3-(diethylamino)propylamine,3-(dimethylamino)propylamine, diethylenetriamine, diisopropyltriamine, bis-(3-aminopropyl)methyleneamine, 3-(2-aminoethylamino)propylamine, N,N'-bis(3-aminopropyl)ethylenediamine, 1-(3-aminopropyl)imidazole, trisaminoethylamine, trisaminopropylamine, and the like. Among these, the amine compound is preferably an amine compound which has a tertiary amine other than 1 or

more and 2 or less terminals and has an aliphatic hydrocarbon group or an aromatic group, and is more preferably an amine compound which has a tertiary amine other than 1 terminal and has an aliphatic hydrocarbon group or an aromatic group.

Among these, $R^{11}$ is preferably a group represented by any one of Formulae (Ia-1) to (Ia-24), and more preferably a group represented by Formulae (Ia-1), (Ia-2), (Ia-3), (Ia-14), (Ia-18), or (Ia-19). In each of the formulae, the wavy line indicates an atomic bonding.

[Chem. 15]

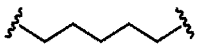
(Ia-1)

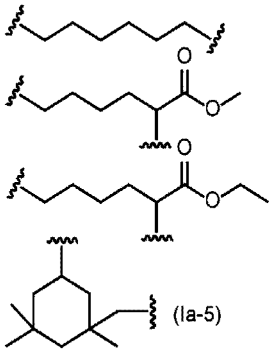
(Ia-2)
(Ia-3)
(Ia-4)
(Ia-5)

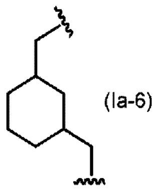
(Ia-6)

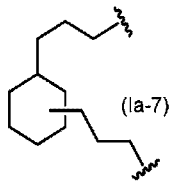
(Ia-7)

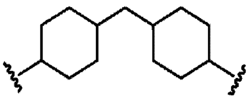
(Ia-8)

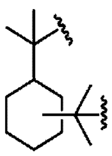
(Ia-9)

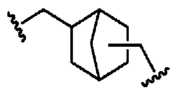
(Ia-10)

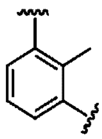
(Ia-11)

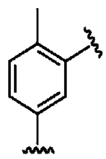
(Ia-12)

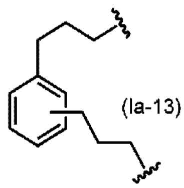
(Ia-13)

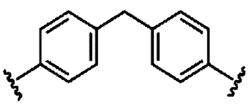
(Ia-14)

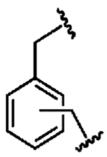
(Ia-15)

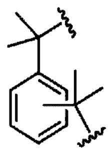
(Ia-16)

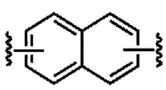
(Ia-17)

[Chem. 16]

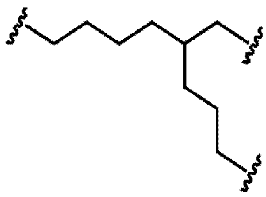

(Ia-18)

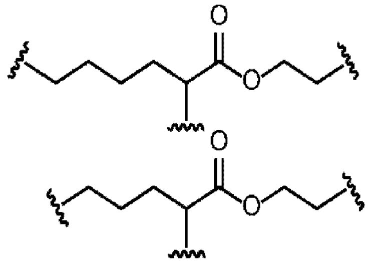

(Ia-19)

(Ia-20)

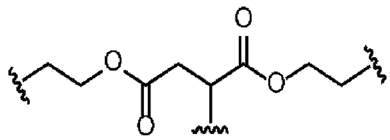

(Ia-21)

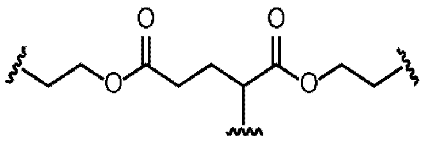

(Ia-22)

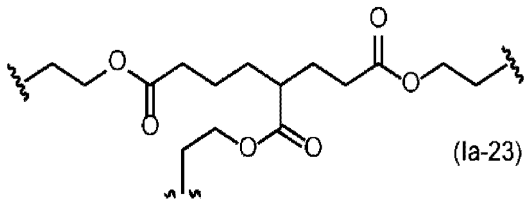

(Ia-23)

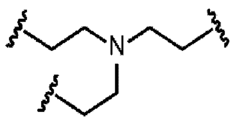

(Ia-24)

[$R^{12}$]

$R^{12}$ is a monovalent organic group, and is preferably an aliphatic hydrocarbon group having 1 or more and 20 or less carbon atoms or an aromatic hydrocarbon group having 6 or more and 20 or less carbon atoms, which may contain an oxygen atom.

Examples of the aliphatic hydrocarbon group in $R^{12}$ include an alkyl group of a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), an undecyl group (each isomer), a dodecyl group (each isomer), a tridecyl group (each isomer), a tetradecyl group (each isomer), a pentadecyl group (each isomer), a hexadecyl group (each isomer), a heptadecyl group (each isomer), an octadecyl group (each isomer), a nonadecyl (each isomer), and an eicosyl group (each isomer); a cycloalkyl group such as a cyclopentyl group, a cyclohexyl group, a cycloheptyl group, a cyclooctyl group, a cyclononyl group, or a cyclodecyl group; and the like.

Examples of the aliphatic hydrocarbon group which may have an oxygen atom in $R^{12}$ include an alkoxyalkyl group such as a methoxymethyl group, a methoxyethyl group (each isomer), a methoxypropyl group (each isomer), a methoxybutyl group (each isomer), a methoxypentyl group (each isomer), a methoxyhexyl group (each isomer), a methoxyheptyl group (each isomer), a methoxyoctyl group (each isomer), a methoxynonyl group (each isomer), a methoxydecyl group (each isomer), a methoxyundecyl group (each isomer), a methoxydodecyl group (each isomer), a methoxytridecyl group (each isomer), a methoxytetradecyl group (each isomer), a methoxypentadecyl group (each isomer), a methoxyhexadecyl group (each isomer), a methoxyheptadecyl group (each isomer), a methoxyoctadecyl group (each isomer), a methoxynonadecyl (each isomer), an ethoxymethyl group, an ethoxyethyl group (each isomer), an ethoxypropyl group (each isomer), an ethoxybutyl group (each isomer), an ethoxypentyl group (each isomer), an ethoxyhexyl group (each isomer), an ethoxyheptyl group (each isomer), an ethoxyoctyl group (each isomer), an ethoxynonyl group (each isomer), an ethoxydecyl group (each isomer), an ethoxyundecyl group (each isomer), an ethoxydodecyl group (each isomer), an ethoxytridecyl group (each isomer), an ethoxytetradecyl group (each isomer), an ethoxypentadecyl group (each isomer), an ethoxyhexadecyl group (each isomer), an ethoxyheptadecyl group (each isomer), an ethoxyoctadecyl group (each isomer), a propyloxymethyl group (each isomer), a propyloxyethyl group (each isomer), a propyloxypropyl group (each isomer), a propyloxybutyl group (each isomer), a propyloxypentyl group (each isomer), a propyloxyhexyl group (each isomer), a propyloxyheptyl group (each isomer), a propyloxyoctyl group (each isomer), a propyloxynonyl group (each isomer), a propyloxydecyl group (each isomer), a propyloxyundecyl group (each isomer), a propyloxidodecyl group (each isomer), a propyloxytridecyl group (each isomer), a propyloxytetradecyl group (each isomer), a propyloxypentadecyl group (each isomer), a propyloxyhexadecyl group (each isomer), a propyloxyheptadecyl group (each isomer), a butyloxymethyl group (each isomer), a butyloxyethyl group (each isomer), a butyloxypropyl group (each isomer), a butyloxybutyl group (each isomer), a butyloxypentyl group (each isomer), a butyloxyhexyl group (each isomer), a butyloxyheptyl group (each isomer), a butyloxyoctyl group (each isomer), a butyloxynonyl group (each isomer), a butyloxydecyl group (each isomer), a butyloxyundecyl group (each isomer), a butyloxidodecyl group (each isomer), a butyloxytridecyl group (each isomer), a butyloxytetradecyl group (each isomer), a butyloxypentadecyl group (each isomer), a butyloxyhexadecyl group (each isomer), a pentyloxymethyl group (each isomer), a pentyloxyethyl group (each isomer), a pentyloxypropyl group (each isomer), a pentyloxybutyl group (each isomer), a pentyloxypentyl group (each isomer), a pentyloxyhexyl group (each isomer), a pentyloxyheptyl group (each isomer), a pentyloxyoctyl group (each isomer), a pentyloxynonyl group (each isomer), a pentyloxydecyl group (each isomer), a pentyloxyundecyl group (each isomer), a pentyloxidodecyl group (each isomer), a pentyloxytridecyl group (each isomer), a pentyloxytetradecyl group (each isomer), a pentyloxypentadecyl group (each isomer), a hexyloxymethyl group (each isomer), a hexyloxyethyl group (each isomer), a hexyloxypropyl group (each isomer), a hexyloxybutyl group (each isomer), a hexyloxypentyl group (each isomer), a hexyloxyhexyl group (each isomer), a hexyloxyheptyl group (each isomer), a hexyloxyoctyl group (each isomer), a hexyloxynonyl group (each isomer), a hexyloxydecyl group (each isomer), a hexyloxyundecyl group (each isomer), a hexyloxidodecyl group (each isomer), a hexyloxytridecyl group (each isomer), a hexyloxytetradecyl group (each isomer), a heptyloxymethyl group (each isomer), a heptyloxyethyl group (each isomer), a heptyloxypropyl group (each isomer), a heptyloxybutyl group (each isomer), a heptyloxypentyl group (each isomer), a heptyloxyhexyl group (each isomer), a heptyloxyheptyl group (each isomer), a heptyloxyoctyl group (each isomer), a heptyloxynonyl group (each isomer), a heptyloxydecyl group (each isomer), a heptyloxyundecyl group (each isomer), a heptyloxidodecyl group (each isomer), a heptyloxytridecyl group (each isomer), an octyloxymethyl group (each isomer), an octyloxyethyl group (each isomer), an octyloxypropyl group (each isomer), an octyloxybutyl group (each isomer), an octyloxypentyl group (each isomer), an octyloxyhexyl group (each isomer), an octyloxyheptyl group (each isomer), an octyloxyoctyl group (each isomer), an octyloxynonyl group (each isomer), an octyloxydecyl group (each isomer), an octyloxyundecyl group (each isomer), an octyloxidodecyl group (each isomer), a nonyloxymethyl group (each isomer), a nonyloxyethyl group (each isomer), a nonyloxypropyl group (each isomer), a nonyloxybutyl group (each isomer), a nonyloxypentyl group (each isomer), a nonyloxyhexyl group (each isomer), a nonyloxyheptyl group (each isomer), a nonyloxyoctyl group (each isomer), a nonyloxynonyl group (each isomer), a nonyloxydecyl group (each isomer), a nonyloxyundecyl group (each isomer), a decyloxymethyl group (each isomer), a decyloxyethyl group (each isomer), a decyloxypropyl group (each isomer), a decyloxybutyl group (each isomer), a decyloxypentyl group (each isomer), a decyloxyhexyl group (each isomer), a decyloxyheptyl group (each isomer), a decyloxyoctyl group (each isomer), a decyloxynonyl group (each isomer), a decyloxydecyl group (each isomer), an undecyloxymethyl group (each isomer), an undecyloxyethyl group (each isomer), an undecyloxypropyl group (each isomer), an undecyloxybutyl group (each isomer), an undecyloxypentyl group (each isomer), an undecyloxyhexyl group (each isomer), an undecyloxyheptyl group (each isomer), an undecyloxyoctyl group (each isomer), an undecyloxynonyl group (each isomer), a dodecyloxymethyl group (each isomer), a dodecyloxyethyl group (each isomer), a dodecyloxypropyl group (each isomer), a dodecyloxybutyl group (each isomer), a dodecyloxypentyl group (each isomer), a dodecyloxyhexyl group (each isomer), a dodecyloxyheptyl group (each isomer), a dodecyldecyloxyoctyl group (each isomer), a tridecyloxymethyl group (each isomer), a tridecyloxyethyl group (each isomer), a tridecyloxypropyl group (each isomer), a tridecyloxybutyl group (each isomer), a tridecyloxypentyl group (each isomer), a tridecyloxyhexyl group (each isomer), a tridecyloxyheptyl group (each isomer), a tetradecyloxymethyl group (each isomer), a tetradecyloxyethyl group (each isomer), a tetradecyloxypropyl group (each isomer), a tetradecyloxybutyl group (each isomer), a tetradecyloxypentyl group (each isomer), a tetradecyloxyhexyl group (each isomer), a pentadecyloxymethyl group, a pentadecyloxyethyl group (each isomer), a pentadecyloxypropyl group (each isomer),

a pentadecyloxybutyl group (each isomer), a pentadecyloxypentyl group (each isomer), a hexadecyloxymethyl group (each isomer), a hexadecyloxyethyl group (each isomer), a hexadecyloxypropyl group (each isomer), a hexadecyloxybutyl group (each isomer), a heptadecyloxymethyl group (each isomer), a heptadecyloxyethyl group (each isomer), a heptadecyloxypropyl group (each isomer), an octadecyloxymethyl group (each isomer), and an octadecyloxyethyl group (each isomer).

Examples of the aromatic hydrocarbon group om $R^{12}$ include an aryl group such as a phenyl group, a naphthyl group, an anthryl group, a pyrenyl group, and a phenanthryl group; a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethyl heptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a cumylphenyl group (each isomer), and the like.

Examples of the aromatic hydrocarbon group which may have an oxygen atom in $R^{12}$ include alkoxyaryl groups such as a methoxyphenyl group (each isomer) and an ethoxyphenyl group (each isomer).

Among these, $R^{12}$ is preferably a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each

isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a cumylphenyl group (each isomer), a methoxyphenyl group (each isomer), or an ethoxyphenyl group (each isomer). In addition, as $R^{12}$, a phenyl group, a cumylphenyl group (each isomer), a methoxyphenyl group (each isomer), or an ethoxyphenyl group (each isomer) is more preferable.

[n11 and n12]

n11 is an integer of 1 or more and 8 or less.

n12 represents the number of isocyanate groups, and is an integer of 0 or more and 7 or less.

The sum (n11 + n12) of n11 and n12 is an integer of 2 or more and 8 or less, preferably an integer of 2 or more and 6 or less, more preferably an integer of 2 or more and 5 or less, and further more preferably an integer of 3 or more and 4 or less.

Generally, the larger the value of (n11 + n12), the more the molecular weight of the carbonyl compound (I) increases and the boiling point also increases, thereby facilitating the separation from the isocyanate. On the other hand, in the production of the carbonyl compound (I), when an isocyanate group having high reactivity is heated, a denaturation reaction is conceived, and this becomes a cause of fixing to the device or clogging. Therefore, from the viewpoint of the production efficiency of the carbonyl compound (I), (n11 + n12) is preferably 6 or less, (n11 + n12) is more preferably 5 or less, and (n11 + n12) is further more preferably 4 or less.

Examples of preferable carbonyl compounds (1) include compounds represented by Formulae (I-1a) to (I-24) (hereinafter, also referred to as "carbonyl compound (I-1a)" in some cases) and the like. In addition, carbonyl compounds (I-1a) to (I-1c), carbonyl compounds (I-2a) to (I-2c), carbonyl compounds (I-3a) to (I-3c), carbonyl compounds (I-4a) to (I-4c), carbonyl compounds (I-5a) to (I-5c), carbonyl compounds (I-6a) to (I-6c), carbonyl compounds (I-7a) to (I-7b), carbonyl compounds (I-8a) to (I-8b), carbonyl compounds (I-9a) to (I-9b), carbonyl compounds (I-10a) to (I-10b), carbonyl compounds (I-11a) to (I-11b), carbonyl compounds (I-12a) to (I-12b), carbonyl compounds (I-22a) to (I-22b), or carbonyl compounds (I-24a) to (I-24b) can be a mixture or single compounds.

[Chem. 17]

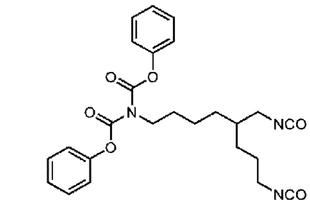

(I - 1a)

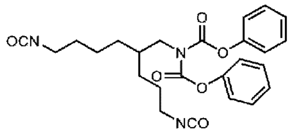

(I - 1b)

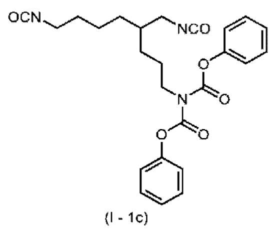

(I - 1c)

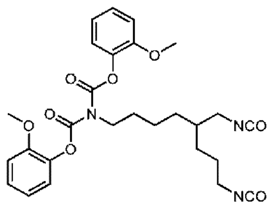

(I - 2a)

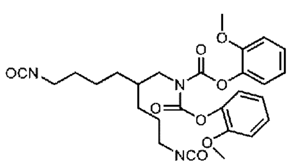

(I - 2b)

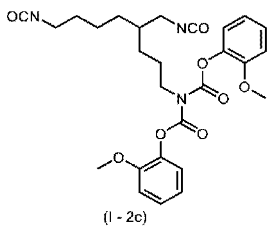

(I - 2c)

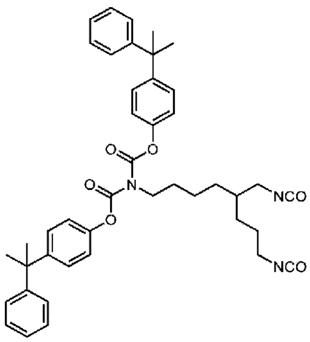

(I - 3a)

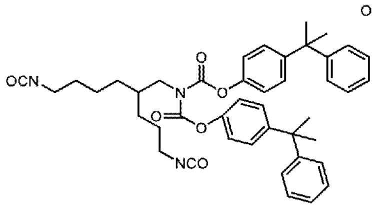

(I - 3b)

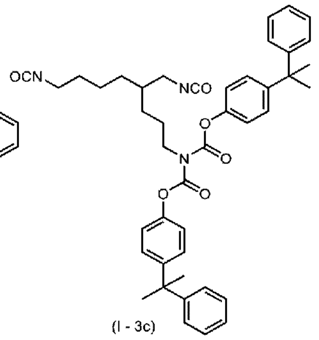

(I - 3c)

[Chem. 18]

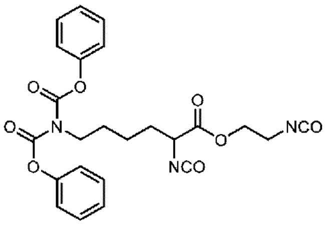
(I - 4a)

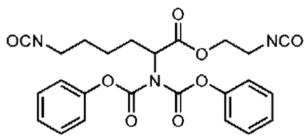
(I - 4b)

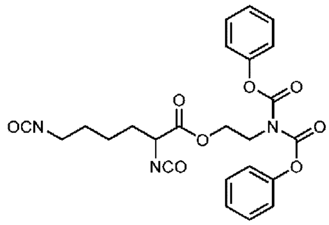
(I - 4c)

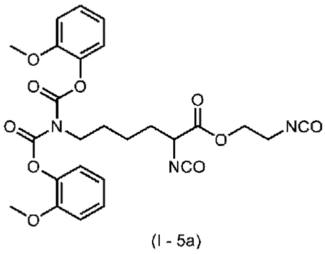
(I - 5a)

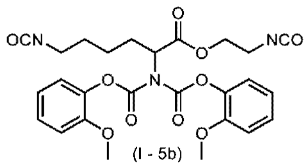
(I - 5b)

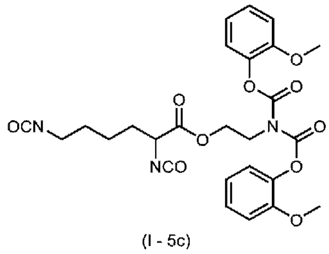
(I - 5c)

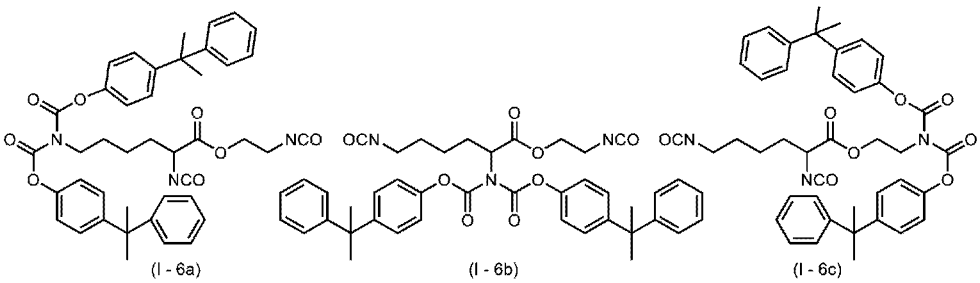
(I - 6a) (I - 6b) (I - 6c)

[Chem. 19]

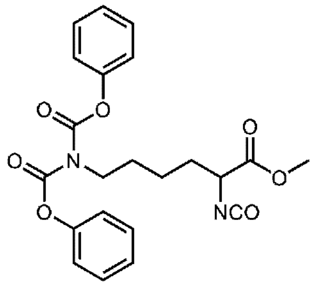

(I - 7a)

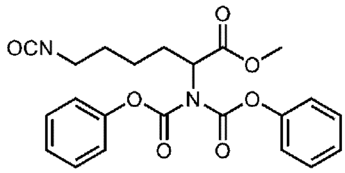

(I - 7b)

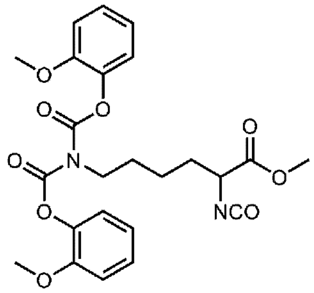

(I - 8a)

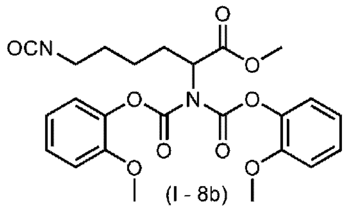

(I - 8b)

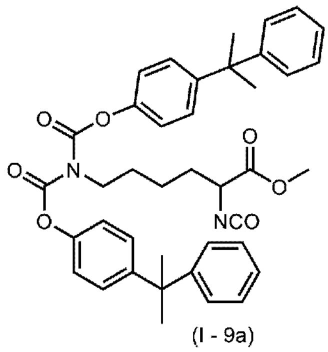

(I - 9a)

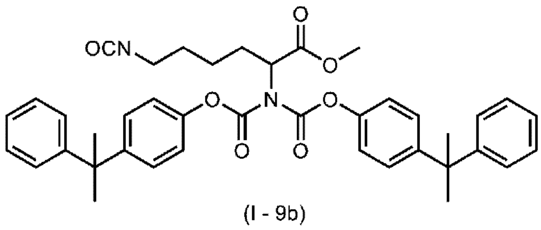

(I - 9b)

[Chem. 20]

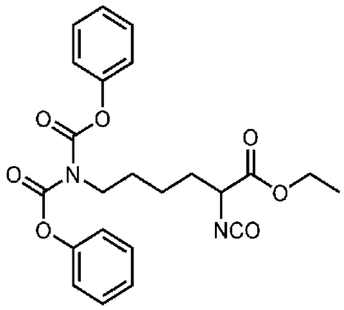

(I - 10a)

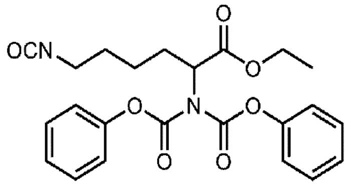

(I - 10b)

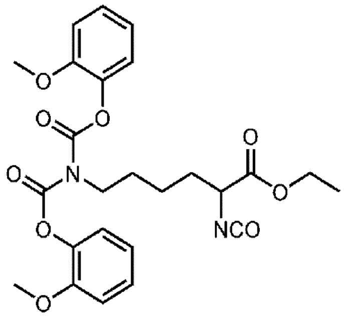

(I - 11a)

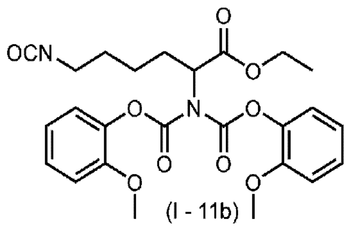

(I - 11b)

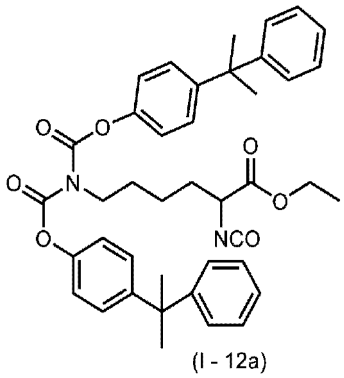

(I - 12a)

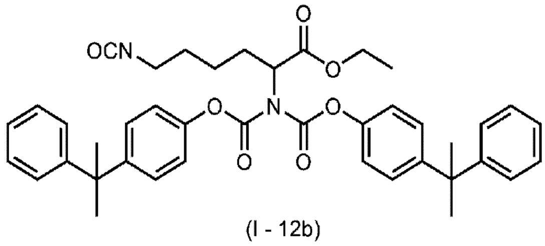

(I - 12b)

[Chem. 21]

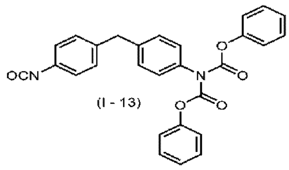

(I - 13)

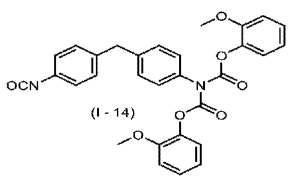

(I - 14)

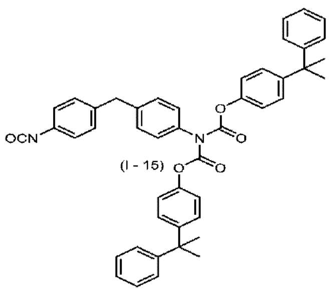

(I - 15)

[Chem. 22]

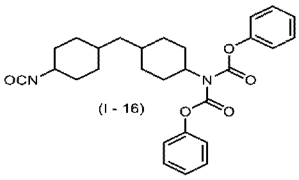
(I - 16)

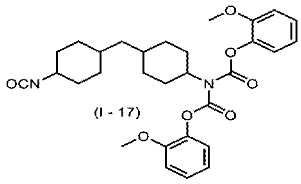
(I - 17)

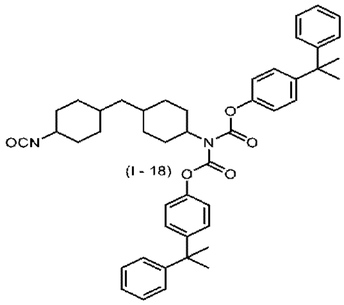
(I - 18)

[Chem. 23]

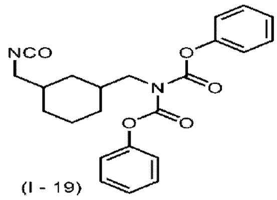
(I - 19)

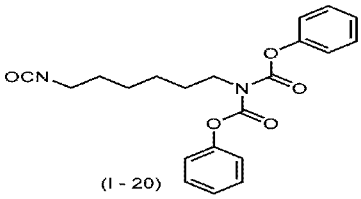
(I - 20)

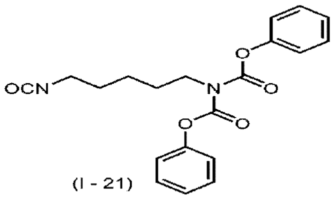
(I - 21)

[Chem. 24]

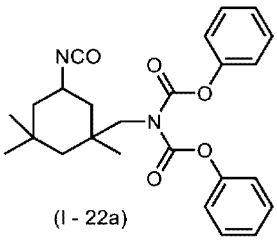
(I - 22a)

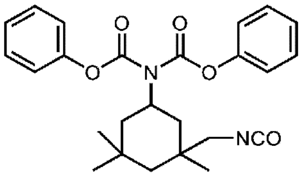
(I - 22b)

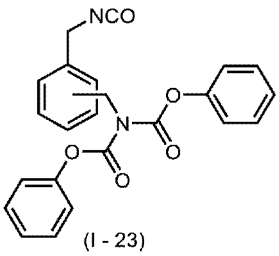
(I - 23)

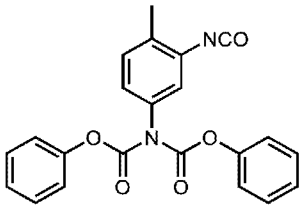
(I - 24a)

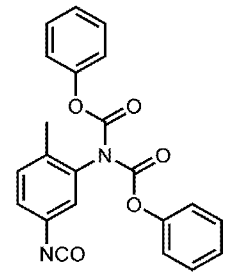
(I - 24b)

<<Method for producing carbonyl compound>>

A method for producing a carbonyl compound of the present embodiment includes mixing one or more compounds selected from the group consisting of an isocyanate compound and a carbamate compound with

one or more compounds selected from the group consisting of a carbonate and a hydroxy compound, and heating thereof to synthesize the carbonyl compound.

The inventors of the present found that the carbonyl compound (I) is generated in the production of an isocyanate compound by a pyrolysis reaction of a carbamate compound.

Therefore, examples of the method for producing a carbonyl compound of the present embodiment include 1) a production method by a pyrolysis reaction of a carbamate compound; and 2) a method for mixing one or more compounds selected from the group consisting of an isocyanate compound and a carbamate compound with one or more compounds selected from the group consisting of a carbonate and a hydroxy compound, and heating thereof.

<Production method by pyrolysis reaction>

In a case where a carbonyl compound is produced by a pyrolysis reaction, as a side reaction, there occurs a reaction of forming an isocyanurate group represented by Formula (B), a reaction of generating carbodiimides represented by Formula (C), a reaction of forming a uretonimine group represented by Formula (D), and a reaction of forming an allophanate group represented by Formula (E). These side reactions occur due to a reaction between isocyanates, between carbamate compounds, or between an isocyanate compound and a carbamate compound. With respect to this, the generation reaction of the carbonyl compound (I) is carried out by a reaction between one or more compounds selected from the group consisting of an isocyanate compound and a carbamate compound and one or more compounds selected from the group consisting of a carbonate and a hydroxy compound. Therefore, in the pyrolysis reaction of the carbamate compound, by increasing a use amount of the carbonate, or carrying out the pyrolysis reaction under reflux conditions to prevent evaporation of the excess carbonate, a generation ratio of the carbonyl compound (I) can be relatively increased.

The carbonyl compound (I) is presumed to be generated by a reaction mechanism represented by Formula (F), (G), or (H).

[Chem. 25]

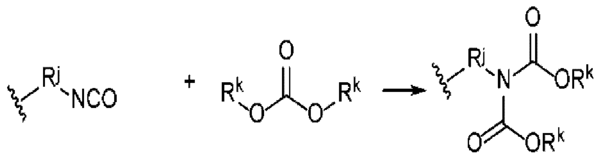
(F)

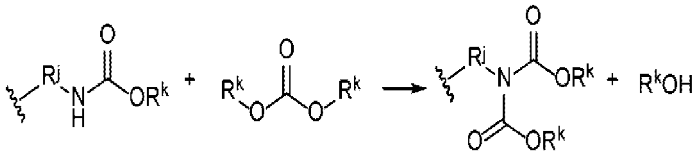
(G)

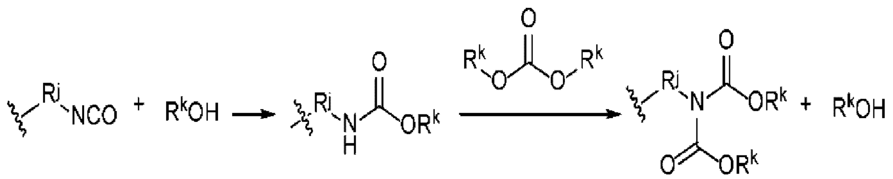
(H)

(In Formulae (F) to (H), $R^j$ is a di- or higher valent organic group. $R^k$ is a monovalent organic group.)

In the pyrolysis reaction, it is preferable that the use amount (molar amount) of the carbonate as a solvent be large from the viewpoint of suppressing a side reaction, but in consideration of the size of the reactor, the use amount is preferably 0.001 times or more and 100 times or less, more preferably 0.01 times or more and 80 times or less, and further more preferably 0.1 times or more and 50 times or less, in terms of a stoichiometric ratio with respect to the carbamate compound.

In a case of producing a carbamate compound by a method to be described later, in a case where the carbamate compound contains a carbonate, the carbonate may be used as it is, or a carbonate may be newly added to the carbamate compound. In addition, the carbonate may be supplied to the reactor before the start of the reaction, may be supplied during the reaction, or may be supplied to the reactor in both cases. Among these, it is preferable that the carbonate be supplied to the reactor before the reaction.

A reaction temperature is usually 100°C or higher and 400°C or lower, and a high temperature is preferable to increase the reaction rate. On the other hand, since there is a case where the above-described side reaction occurs due to one or more compounds selected from the group consisting of a carbamate compound and an isocyanate compound which is a product, at a high temperature, the reaction temperature is preferably 130°C or higher and 300°C or lower, and more preferably 150°C or higher and 280°C or lower. To keep the reaction temperature constant, known cooling devices and heating devices may be installed in the reactor.

In addition, a reaction pressure varies depending on the type of the compound used or the reaction temperature, but the reaction pressure may be any one of reduced pressure, normal pressure, or pressurized pressure, and the reaction pressure is usually in a range of 20 Pa or more and $1 \times 10^6$ Pa or less.

A reaction time (retention time in the case of a continuous method) is not particularly limited, but is usually 0.001 hours or longer and 100 hours or shorter, preferably 0.01 hours or longer and 50 hours or shorter, and more preferably 0.1 hours or longer and 10 hours or shorter.

A catalyst can be used, and a use amount of the catalyst is preferably 0.01% by mass or more and 30% by mass or less, and more preferably 0.5% by mass or more and 20% by mass or less, with respect to the mass of the carbamate compound.

Examples of the catalyst include organic metal catalysts such as dibutyltin dilaurate, lead octylate, and stannous octoate; and amines such as 1,4-diazabicyclo[2,2,2]octane, triethylenediamine, and triethylamine. Among these, organic metal catalysts such as dibutyltin dilaurate, lead octylate, and stanaoctoate are suitable. These compounds may be used alone or a mixture of two or more types thereof may be used.

As described above, the pyrolysis reaction is a reaction of generating the corresponding isocyanate compound, a hydroxy compound, and a carbonyl compound from the carbamate compound. To efficiently obtain the carbonyl compound, in the pyrolysis reaction, the isocyanate compound, the carbonate, and the carbamate compound need to be heated in a coexisting state. Therefore, to efficiently obtain a carbonyl compound in the pyrolysis reaction, it is preferable to extract a hydroxy compound, which is the product in the pyrolysis reaction, as a gas-phase component from the pyrolysis reaction system by, for example, distillation or the like, to separate the hydroxy compound from the carbonate and the isocyanate compound under conditions of reflux and the like, and to cause the carbonate and the isocyanate to exist as liquid-phase components.

After the pyrolysis reaction, a light boiling component may be distilled off, and the distillation method is not particularly limited as long as the light boiling component can be separated as a gas-phase component. The "light boiling component (light-boiling-point component)" referred to herein refers to a component having a boiling point lower than that of the carbonyl compound, and varies depending on the type of the compound serving as a raw material for producing the carbonyl compound, but is mainly one or more compounds selected from the group consisting of a carbonate used in the pyrolysis step, an isocyanate compound generated by the pyrolysis reaction, and a hydroxy compound.

A pressure at a time of distilling off the light boiling component varies depending on the type of the compound or the reaction temperature, but the pressure may be any of reduced pressure, normal pressure, and pressurized pressure as long as the carbonyl compound and the light boiling component can be separated, and is preferably 20 Pa or more and $1 \times 10^6$ Pa or less, more preferably 20 Pa or more and $1 \times 10^4$ Pa or less, further more preferably 20 Pa or more and $1 \times 10^3$ Pa or less, and particularly preferably 20 Pa or more and $1 \times 10^2$ Pa or less.

An operation time (retention time in the case of a continuous method) at the time of distilling off the light boiling component is not particularly limited as long as the carbonyl compound and the light boiling component can be separated, and, from the viewpoint of suppressing a side reaction between the carbonyl compound, is preferably 5 seconds or longer and 100 hours or shorter, more preferably 10 seconds or longer and 50 hours or shorter, and further more preferably 20 seconds or longer and 10 hours or shorter.

A temperature at the time of distilling off the light boiling component is not particularly limited as long as the carbonyl compound is stable and the carbonyl compound and the light boiling component can be separated, and from the viewpoint of suppressing denaturation of the carbonyl compound, the temperature is preferably 20°C or higher and 300°C or lower, more preferably 30°C or higher and 280°C or lower, and further more preferably 40°C or higher and 250°C or lower.

<Production method by heating mixture>

As can be seen from the fact that the reaction mechanism of the carbonyl compound is represented by Formula (F), (G), or (H), the carbonyl compound (I) is obtained by heating a mixture of the isocyanate compound (II) and the carbonate (IV), a mixture of the carbamate compound (III) or the carbamate compound (VI) and the carbonate (IV), a mixture of the isocyanate compound (II), the hydroxy compound (V), and the carbonate (IV), a mixture of the isocyanate compound (II), the carbamate compound (III) or the carbamate compound (VI), and the carbonate (IV), or a mixture of the isocyanate compound (II), the carbamate compound (III) or the carbamate compound (VI), the carbonate (IV), and the hydroxy compound (V) (hereinafter, these mixtures may be collectively referred to as simply "raw material mixture" in some cases).

In the raw material mixture, a blending amount (molar amount) of the carbonate is preferably large from the viewpoint of suppressing a side reaction, but in consideration of the size of the reactor and the like, the blending amount is preferably 0.001 times or more and 100 times or less, more preferably 0.01 times or more and 80 times or less, and further more preferably 0.1 times or more and 50 times or less, in terms of the stoichiometric ratio with respect to the carbamate compound.

A heating temperature is usually 100°C or higher and 400°C or lower, and a high temperature is preferable to increase the reaction rate. On the other hand, since there is a case where the above-described side reaction occurs due to one or more compounds selected from the group consisting of a carbamate compound and an isocyanate

compound which is a product, at a high temperature, the reaction temperature is preferably 130°C or higher and 300°C or lower, and more preferably 150°C or higher and 280°C or lower. To keep the reaction temperature constant, known cooling devices and heating devices may be installed in the reactor.

A pressure at the time of heating varies depending on the type of the compound used or the reaction temperature, but the pressure may be any one of reduced pressure, normal pressure, or pressurized pressure, and the pressure is usually in a range of 20 Pa or more and $1 \times 10^6$ Pa or less.

A heating time (retention time in the case of a continuous method) is not particularly limited, and is usually 0.001 hours or longer and 100 hours or shorter, preferably 0.01 hours or longer and 50 hours or shorter, and more preferably 0.1 hours or longer and 10 hours or shorter.

In the production of the carbonyl compound by mixing and heating, it is possible to increase a generation rate and a generation amount of the carbonyl compound by bringing the raw material mixture into contact with stainless steel and heating the raw material mixture. As the stainless steel, any shape can be used as long as the stainless steel is made of SUS316 or SUS304, and for example, a filling material or a metal piece is preferably used. The filling material is not particularly limited, but DIXON Packing, Mc MAHON Packing, Coil Pack, MESH RING, CANNON Packing, HELI PACK, RASCHIG RING, PRICKLE RING, or the like is used.

In a case where a volume of the raw material liquid of the carbonyl compound is denoted as V and a surface area of the stainless steel is denoted as A, the larger the contact area with stainless steel per unit volume of the raw material liquid, the higher the generation rate of the carbonyl compound. A value of A/V is preferably 0.001 $m^2/m^3$ or more and 100,000 $m^2$ /$m^3$ or less, more preferably 0.01 $m^2/m^3$ or more and 50,000 $m^2/m^3$ or less, and further more preferably 0.1 $m^2/m^3$ or more and 10,000 $m^2/m^3$ or less.

A reaction form is not particularly limited, but a reactor that can efficiently mix and heat the raw material mixture or the mixture and stainless steel is preferable, and for example, a method of heating a raw material mixture in a stirring tank or a distillation column made of stainless steel is preferable.

The distillation method is not particularly limited as long as a light boiling component may be distilled off after heating a raw material mixture, and the light boiling component can be separated as a gas-phase component. The "light boiling component (light boiling point component)" referred to herein refers to a component having a boiling point lower than that of the carbonyl compound and varies depending on the type of the compound serving as a raw material for producing the carbonyl compound, but is mainly one or more compounds selected from the group consisting of a carbonate contained in the raw material mixture, an isocyanate compound generated by the pyrolysis reaction, and a hydroxy compound.

A pressure at a time of distilling off the light boiling component varies depending on the type of the compound or the reaction temperature, but the pressure may be any of reduced pressure, normal pressure, and pressurized pressure as long as the carbonyl compound and the light boiling component can be separated, and is preferably 20 Pa or more and $1 \times 10^6$ Pa or less, more preferably 20 Pa or more and $1 \times 10^4$ Pa or less, further more preferably 20 Pa or more and $1 \times 10^3$ Pa or less, and particularly preferably 20 Pa or more and $1 \times 10^2$ Pa or less.

An operation time (retention time in the case of a continuous method) at the time of distilling off the light boiling component is not particularly limited as long as the carbonyl compound and the light boiling component can be separated, and, from the viewpoint of suppressing a side reaction between the carbonyl compound, is preferably 5 seconds or longer and 100 hours or shorter, more preferably 10 seconds or longer and 50 hours or shorter, and further more preferably 20 seconds or longer and 10 hours or shorter.

A temperature at the time of distilling off the light boiling component is not particularly limited as long as the carbonyl compound is stable and the carbonyl compound and the light boiling component can be separated, and from the viewpoint of suppressing denaturation of the carbonyl compound, the temperature is preferably 20°C or higher and 300°C or lower, more preferably 30°C or higher and 280°C or lower, and further more preferably 40°C or higher and 250°C or lower.

Subsequently, various raw materials used in the method for producing a carbonyl compound of the present embodiment will be described in detail below.

<Isocyanate compound>

As the isocyanate compound, a compound represented by General Formula (II) (hereinafter, referred to as "isocyanate compound (II)" in some cases) is preferably used.

[Chem. 26]

$$R^{21}\left(-NCO\right)_{n21} \qquad (II)$$

(In General Formula (II), $R^{21}$ is an n21-valent organic group, and satisfies a relational formula: $R^{21} = R^{11}$. n21 is an integer of 2 or more and 8 or less, and satisfies a relational formula: n21 = n11 + n12.)

[$R^{21}$]

$R^{21}$ is an n21-valent organic group, and satisfies a relational formula: $R^{21} = R^{11}$. That is, $R^{21}$ is the same as $R^{11}$.

In a case where $R^{21}$ is an aliphatic hydrocarbon group, specific examples of the isocyanate compound (II) include aliphatic diisocyanates, aliphatic triisocyanates, substituted cyclic aliphatic polyisocyanates, and the like.

Examples of the aliphatic diisocyanates include diisocyanatoethane, diisocyanatopropane (each isomer), diisocyanatobutane (each isomer), diisocyanatopentane (each isomer), diisocyanatohexane (each isomer), diisocyanatodecane (each isomer), and the like.

Examples of the aliphatic triisocyanates include triisocyanatohexane (each isomer), 4-isocyanatomethyl-1,8-octamethylene diisocyanate, triisocyanatononane (each isomer), triisocyanatodecane (each isomer), and the like.

Examples of the substituted cyclic aliphatic polyisocyanates include diisocyanatocyclobutane (each isomer), diisocyanatocyclohexane (each isomer), 3-isocyanatomethyl-3,5,5-trimethylcyclohexyl isocyanate (also referred to as isophorone diisocyanate) (each isomer), 1,3-bis(isocyanatomethyl)cyclohexane (at least one isomer of a cis-form and a trans-form), methylenebis(cyclohexylisocyanate) (also referred to as dicyclohexylmethane diisocyanate) (each isomer), and the like.

In a case where $R^{21}$ is an aromatic group, specific examples of the isocyanate compound (II) include aromatic diisocyanates, aromatic triisocyanates, and the like.

Examples of the aromatic diisocyanates include diisocyanatobenzene (each isomer), diisocyanatotoluene (each isomer), bis(isocyanatophenyl)methane (each isomer), diisocyanatomethylene (each isomer), diisocyanatobiphenyl (each isomer), diisocyanatodibenzyl (each isomer), bis(isocyanatophenyl)propane (each isomer), bis(isocyanatophenyl) ether (each isomer),bis(isocyanatophenoxyethane) (each isomer), diisocyanatoxylene (each isomer), diisocyanatoanisole (each isomer), diisocyanatophenitol (each isomer), diisocyanatonaphthalene (each isomer), diisocyanatomethylbenzene (each isomer), diisocyanatomethylpyridine (each isomer), diisocyanatomethylnaphthalene (each isomer), diisocyanatodiphenylmethane (each isomer), tetramethylxilicon diisocyanate (each isomer), and the like.

Examples of the aromatic triisocyanates include triisocyanatobenzene (each isomer), triisocyanato-methylbenzene (each isomer), tris(isocyanatopropane-yl)benzene (each isomer), tris(isocyanatopropane-yl)-methylbenzene (each isomer), tris(isocyanatomethyl)-methylbenzene (each isomer), ((isocyanato-phenylene)bis(methylene))bis(isocyanatobenzene) (each isomer), and the like.

In a case where $R^{21}$ is aromatic, the reactivity of the isocyanate group which is directly bound is increased due to an electron-attracting effect of the aromatic group. In addition, as the number of the isocyanate groups directly bound to the same aromatic compound increases, the reactivity is further enhanced due to a mutual electron-attracting action. From the viewpoint of reactivity, the number of the isocyanate groups to $R^{21}$ is preferably 2 or more, and more preferably 3 or more. On the other hand, in a case where the reactivity of the isocyanate increases, a reaction between moisture or the isocyanates and other impurities occurs, and thus the stability of the compound at room temperature and at the time of heating decreases. From the viewpoint of stability of the isocyanate compound, the number of the isocyanate groups which are directly bound to $R^{21}$ is preferably 4 or less, and more preferably 3 or less.

In a case where the aliphatic hydrocarbon group or aromatic group in $R^{21}$ has 1 or more and 4 or less ester groups or nitrogen atoms, specific examples of the isocyanate compound (II) include acrylic acid-2-isocyanato-ethyl ester, 2-methyl-acrylic acid-2-isocyanato-ethyl ester, acrylic acid-2-isocyanato-propyl ester, 2-methyl-acrylic acid-2-isocyanato-propyl ester, acrylic acid-3-isocyanato-propyl ester, 2-methyl-acrylic acid-3-isocyanato-propyl ester, acrylic acid-4-isocyanato-butyl ester, 2-methyl-acrylic acid-4-isocyanato-butyl ester, acrylic acid-5-isocyanato-pentyl ester, 2-methyl-acrylic acid-5-isocyanato-pentyl ester, acrylic acid-6-isocyanato-hexyl ester, 2-methyl-acrylic acid-6-isocyanato-hexyl ester, acrylic acid-8-isocyanato-octyl ester, 2-methyl-acrylic acid-8-isocyanato-octyl ester, acrylic acid-10-isocyanato-decyl ester, 2-methyl-acrylic acid-10-isocyanato-decyl ester, acrylic acid-11-isocyanato-undecyl ester, 2-methyl-acrylic acid-11-isocyanato-undecyl ester, acrylic acid-12-isocyanato-dodecyl ester, 2-methyl-acrylic acid-12-isocyanato-dodecyl ester, lysine methyl ester diisocyanate, lysine ethyl ester diisocyanate, 2-isocyanatoethyl-2,5-diisocyanatopentanoate, 2-isocyanatoethyl-2,6-diisocyanatohexanoate (lysine triisocyanate), bis(2-isocyanatoethyl)-2-isocyanatobutanedioate, bis(2-isocyanatoethyl)-2-isocyanatopentanedioate, tris(2-isocyanatoethyl)hexane-1,3,6-tricarboxylate, trisisocyanatoethylamine, trisisocyanatopropyl amine, and the like.

As the isocyanate compound has an electron-attracting group such as an ester group or a N atom, reactivity of the isocyanate group is improved. Since the reactivity of the isocyanate group is further improved as the number of carbon atoms between the isocyanate group and the ester group or the nitrogen atom is smaller, the number of carbon atoms is preferably 1 or more and 20 or less, more preferably 1 or more and 8 or less, and further more preferably 1 or more and 3 or less. On the other hand, the carbon-carbon or carbon-nitrogen bond between the isocyanate group which is an electron-attracting group and the ester group or the nitrogen atom is easily dissociated, and thus the thermal stability of the compound is impaired in some cases. Therefore, from the viewpoint of the stability of a compound in which an isocyanate group and an ester group or a nitrogen atom coexist, the larger the number of carbon atoms between the isocyanate group and the ester group or the nitrogen atom, the further improved the stability of the compound, and thus the number of carbon atoms is preferably 1 or more, more preferably 2 or more, and further more preferably 3 or more.

The isocyanate compound (II) is often used as a raw material for coating materials, and is required to have a non-coloring property (atmospheric corrosion resistance) when exposed to light such as the sunlight. In a case where $R^{21}$ contains an aromatic group, coloring occurs due to the absorption of light energy when exposed to the sunlight. Therefore, from the viewpoint of atmospheric corrosion resistance, $R^{21}$ preferably has no aromatic group, may have 1 or more and 4 or less ester groups or nitrogen atoms, and is more preferably a di- or higher valent and tetra- or lower valent aliphatic hydrocarbon group having 1 or more and 20 or less carbon atoms.

In addition, specifically, $R^{21}$ is preferably a group represented by any of Formulae (Ia-1) to (Ia-24), and more preferably a group represented by Formulae (Ia-1), (Ia-2), (Ia-3), (Ia-14), (Ia-18), or (Ia-19).

(n21)

n21 represents the number of isocyanate groups, and satisfies a relational formula: n21 = n11 + n12. n21 is an integer of 2 or more and 8 or less, preferably an integer of 2 or more and 6 or less, more preferably an integer of 2 or more and 5 or less, and further more preferably an integer of 3 or more and 4 or less. Generally, a polymer can be obtained by reacting an n21- or higher-valent isocyanate with a dihydroxy compound or a diamine compound having an active hydrogen group. The larger the value of n21, the more the crosslinking points (isocyanate groups) per isocyanate molecule. Therefore, the crosslinking density at the time of polymerization increases, and the curing time can be shortened or the hardness of the polymer can be improved. The expression "the crosslinking density increases" referred to herein means that an average molecular chain length between the crosslinking points decreases. In a case where the number of the isocyanate groups in the isocyanate molecule is 3 or more (n21 is 3 or more), the linear polymer and the polymer can further bind to each other, and a molecular weight of the polymer tends to increase. Particularly, significant reduction of the curing time, or dramatic improvement of physical properties of a polymer such as the hardness can be expected. On the other hand, in the production of isocyanate, when an isocyanate compound having high reactivity is heated, a denaturation reaction is conceived, and this becomes a cause of fixing to the device or clogging. Therefore, the valence of the isocyanate group n21 in the isocyanate compound is preferably 6 or less, more preferably 5 or less, and further more preferably 4 or less.

Examples of the preferable isocyanate compound (II) include 4-isocyanatomethyl-1,8-octamethylene diisocyanate (TTI), 2-isocyanatoethyl-2,6-diisocyanatohexanoate (LTI), lysine methyl ester diisocyanate (LDI), methylenebis(cyclohexyl isocyanate) (HMDI), 1,3-bis(isocyanatomethyl)cyclohexane (HXDI), diisocyanatodiphenylmethane (MDI), and the like.

<Carbamate compound>

As the carbamate compound, a compound represented by General Formula (III) (hereinafter, referred to as "carbamate compound (III)" in some cases) or a compound represented by General Formula (VI) (hereinafter, also referred to as "carbamate compound (VI)" in some cases) is preferably used.

[Chem. 27]

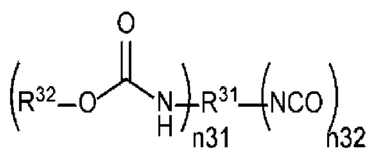

(III)

(In General Formula (III), $R^{31}$ is an (n31 + n32)-valent organic group, and satisfies a relational formula: $R^{31}$ =

$R^{11}$. $R^{32}$ is a monovalent organic group, and satisfies a relational formula: $R^{32} = R^{12}$. n31 is an integer of 1 or more and 8 or less, n32 is an integer of 0 or more and 7 or less, and a sum of n31 and n32 is an integer of 2 or more and 8 or less, and satisfies a relational formula: n31 + n32 = n11 + n12.)

[Chem. 28]

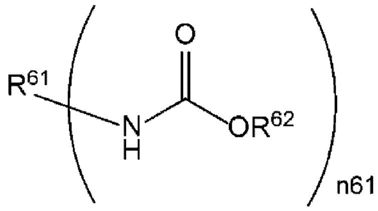

(VI)

(In General Formula (VI), $R^{61}$ is an n61-valent organic group, and satisfies a relational formula: $R^{61} = R^{21}$. $R^{62}$ is a monovalent organic group, and satisfies a relational formula: $R^{62} = R^{12}$. n61 is an integer of 2 or more and 8 or less, and satisfies a relational formula: n61 = n21.)

[Carbamate compound (III)]

(R31)

$R^{31}$ is an (n31 + n32)-valent organic group, and satisfies a relational formula: $R^{31} = R^{11}$. That is, $R^{31}$ is the same as $R^{11}$.

Among these, $R^{31}$ is preferably a di- or higher valent and tetra- or lower valent aliphatic hydrocarbon group having 1 or more and 20 or less carbon atoms, which may have 1 or more and 4 or less ester groups or nitrogen atoms, or a di- or higher valent and tri- or lower valent aromatic hydrocarbon group having 6 or more and 20 or less carbon atoms.

In addition, specifically, $R^{31}$ is preferably a group represented by any of Formulae (Ia-1) to (Ia-24), and more preferably a group represented by Formulae (Ia-1), (Ia-2), (Ia-3), (Ia-14), (Ia-18), or (Ia-19).

($R^{32}$)

$R^{32}$ is a monovalent organic group, and satisfies a relational formula: $R^{32} = R^{12}$. That is, $R^{32}$ is the same as $R^{12}$.

Among these, $R^{32}$ is preferably an aliphatic hydrocarbon group having 1 or more and 20 or less carbon atoms or an aromatic hydrocarbon group having 6 or more and 20 or less carbon atoms, which may contain an oxygen atom.

In addition, as $R^{32}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group

(each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a cumylphenyl group (each isomer), a methoxyphenyl group (each isomer), or an ethoxyphenyl group (each isomer) is preferable. In addition, as $R^{12}$, a phenyl group, a cumylphenyl group (each isomer), a methoxyphenyl group (each isomer), or an ethoxyphenyl group (each isomer) is more preferable.

(n31 and n32)

n31 is an integer of 1 or more and 8 or less.
n32 is an integer of 0 or more and 7 or less.
The sum of n31 and n32 is an integer of 2 or more and 8 or less, and satisfies a relational formula: n31 + n32 = n11 + n12.
Examples of the preferable carbamate compound (III) include compounds represented by Formulae (III-1a) to (III-24b). In addition, carbamate compounds (III-1a) to (III- 1c), carbamate compounds (III-2a) to (III-2c), carbamate compounds (III-3a) to (III-3c), carbamate compounds (III-4a) to (III-4c), carbamate compounds (III-5a) to (III-5c), carbamate compounds (III-6a) to (III-6c), carbamate compounds (III-7a) to (III-7b), carbamate compounds (III-8a) to (III-8b), carbamate compounds (III-9a) to (III-9b), carbamate compounds (III-10a) to (III-10b), carbamate compounds (III-11a) to (111-11b), carbamate compounds (III-12a) to (III-12b), carbamate compounds (III-22a) to (III-22b), or carbamate compounds (III-24a) to (III-24b) can be a mixture, or single compounds.

[Chem. 29]

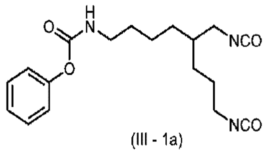
(III - 1a)

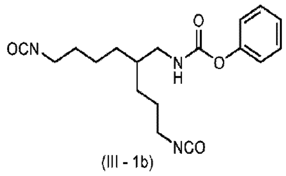
(III - 1b)

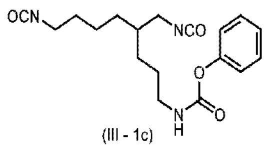
(III - 1c)

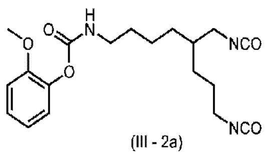
(III - 2a)

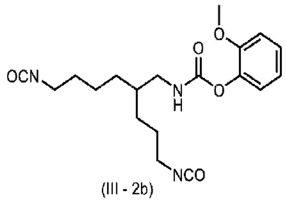
(III - 2b)

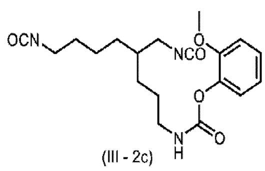
(III - 2c)

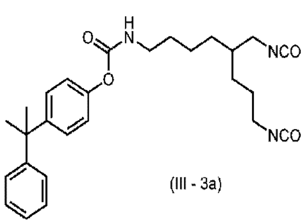
(III - 3a)

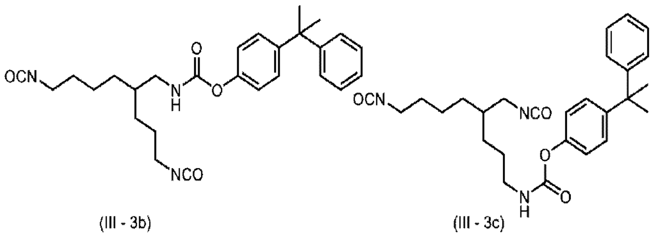
(III - 3b)

(III - 3c)

[Chem. 30]

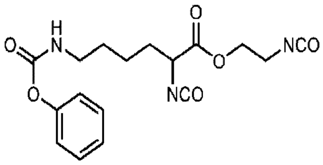
(III - 4a)

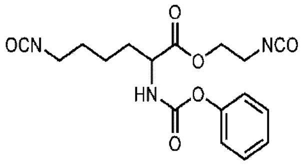
(III - 4b)

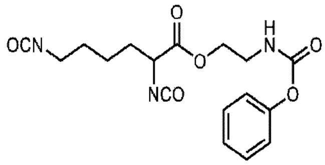
(III - 4c)

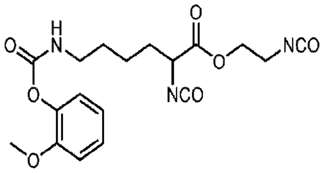
(III - 5a)

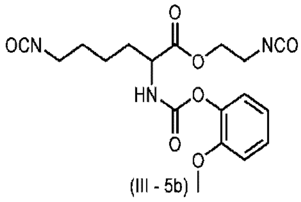
(III - 5b)

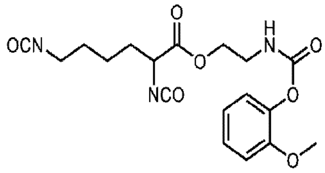
(III - 5c)

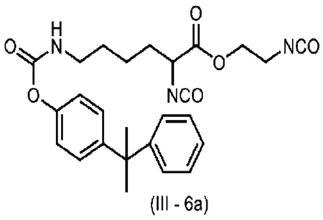
(III - 6a)

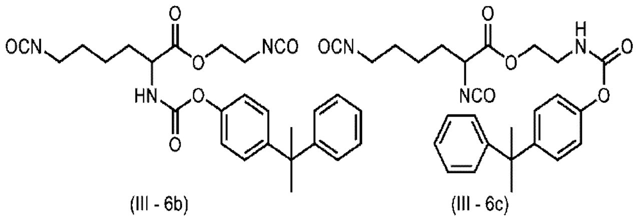
(III - 6b)

(III - 6c)

[Chem. 31]

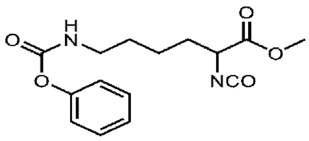
(III - 7a)

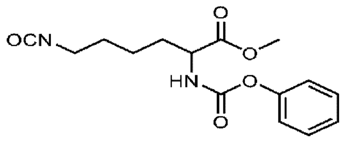
(III- 7b)

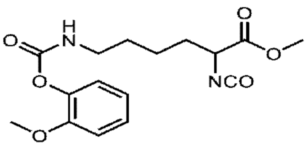
(III - 8a)

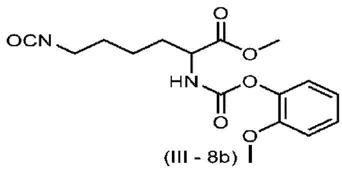
(III - 8b)

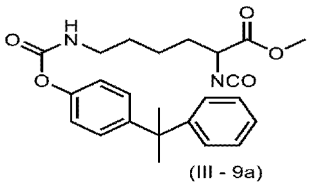
(III - 9a)

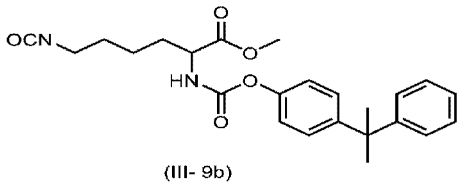
(III- 9b)

[Chem. 32]

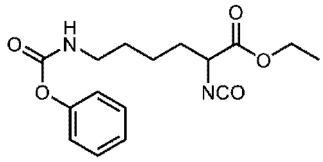

(III - 10a)

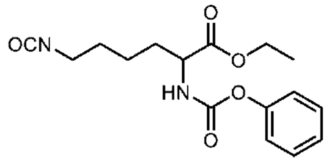

(III - 10b)

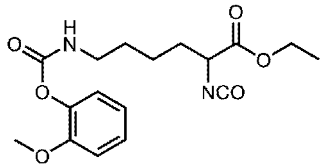

(III - 11a)

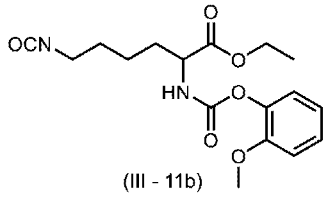

(III - 11b)

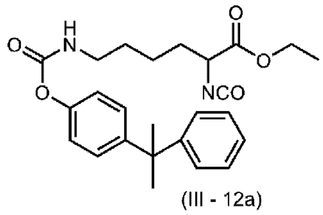

(III - 12a)

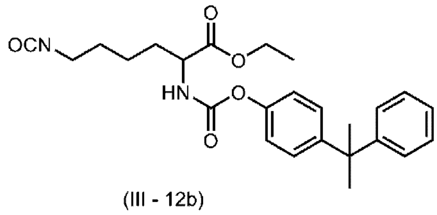

(III - 12b)

[Chem. 33]

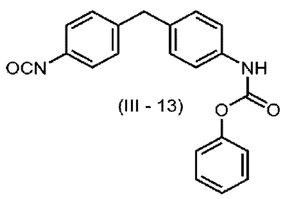

(III - 13)

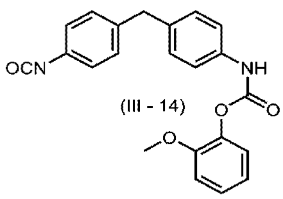

(III - 14)

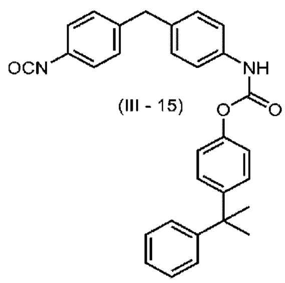

(III - 15)

[Chem. 34]

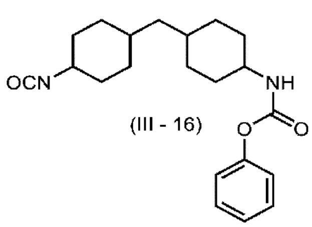

(III - 16)

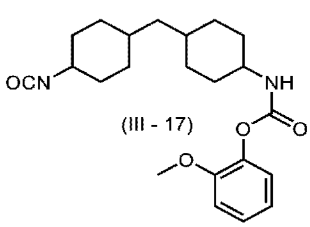

(III - 17)

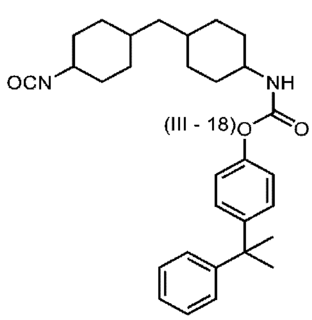

(III - 18)

[Chem. 35]

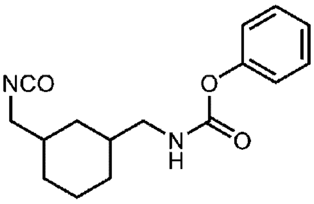
(III - 19)

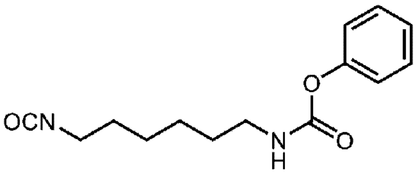
(III - 20)

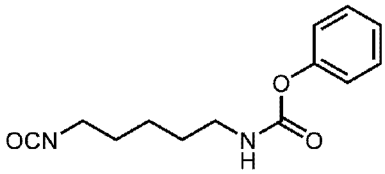
(III - 21)

[Chem. 36]

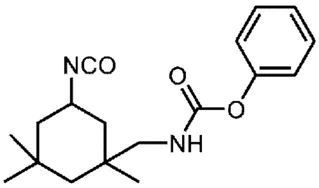
(III - 22a)

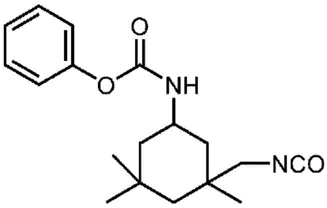
(III - 22b)

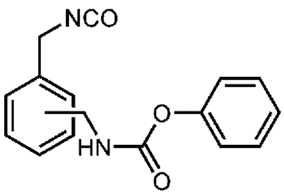
(III - 23)

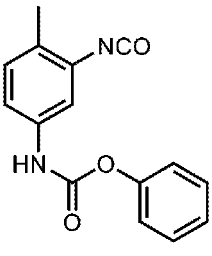
(III - 24a)

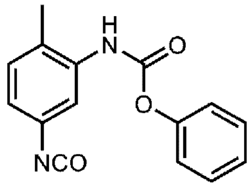
(III - 24b)

[Carbamate compound (VI)]

(R61)

$R^{61}$ is an n61-valent organic group, and satisfies a relational formula: $R^{61} = R^{21}$. That is, $R^{61}$ is the same as $R^{21}$.

Among these, $R^{61}$ is preferably a di- or higher valent and tetra- or lower valent aliphatic hydrocarbon group having 1 or more and 20 or less carbon atoms, or a di- or higher valent and tri- or lower valent aromatic hydrocarbon group having 6 or more and 20 or less carbon atoms, which may have 1 or more and 4 or less ester groups or nitrogen atoms.

In addition, as $R^{61}$, specifically, a group represented by any of Formulae (Ia-1) to (Ia-24) is preferable, and a group represented by Formula (Ia-1), (Ia-2), (Ia-3), (Ia-14), (Ia-18), or (Ia-19) is more preferable.

($R^{62}$)

$R^{62}$ is a monovalent organic group, and satisfies a relational formula: $R^{62} = R^{12}$. That is, $R^{62}$ is the same as $R^{12}$.

Among these, as $R^{62}$, an aliphatic hydrocarbon group having 1 or more and 20 or less carbon atoms or an aromatic hydrocarbon group having 6 or more and 20 or less carbon atoms, which may contain an oxygen atom, is preferable.

In addition, as $R^{62}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each

isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a cumylphenyl group (each isomer), a methoxyphenyl group (each isomer), or an ethoxyphenyl group (each isomer) is preferable. In addition, as $R^{62}$, a phenyl group, a cumylphenyl group (each isomer), a methoxyphenyl group (each isomer), or an ethoxyphenyl group (each isomer) is more preferable.

(n61)

n61 represents the number of carbamate groups and satisfies a relational formula: n61 = n21. n61 is an integer of 2 or more and 8 or less, preferably an integer of 2 or more and 6 or less, more preferably an integer of 2 or more and 5 or less, and further more preferably an integer of 3 or more and 4 or less.

Examples of the preferable carbamate compound (VI) include compounds represented by Formulae (VI-1) to (VI-24) and the like.

[Chem. 37]

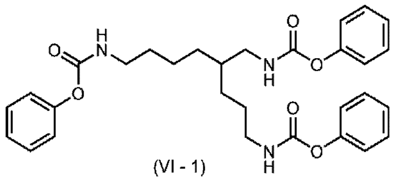

(VI - 1)

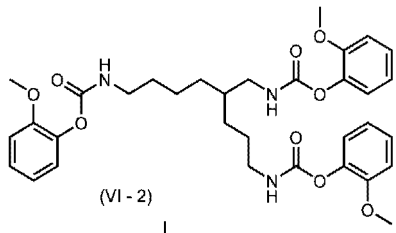

(VI - 2)

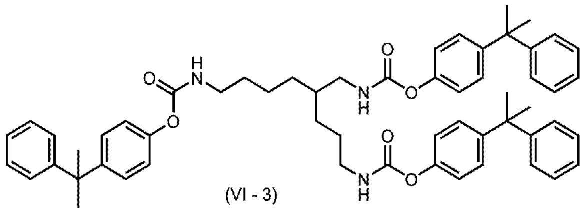

(VI - 3)

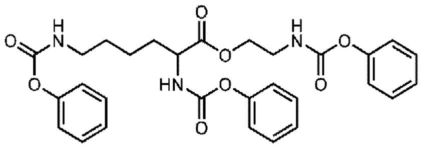

(VI - 4)

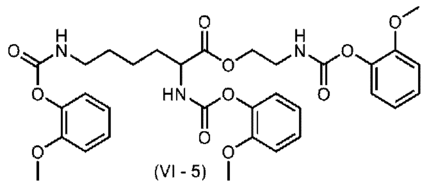

(VI - 5)

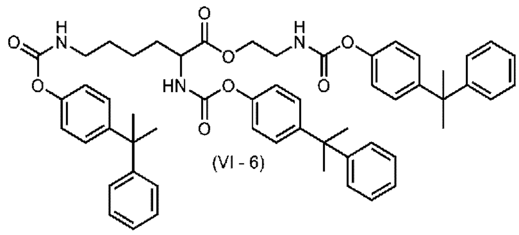

(VI - 6)

[Chem. 38]

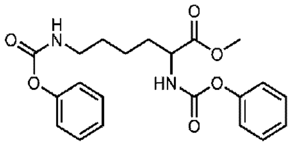
(VI - 7)

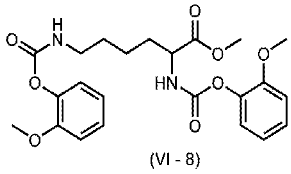
(VI - 8)

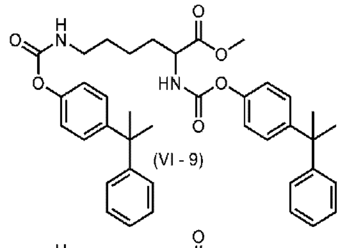
(VI - 9)

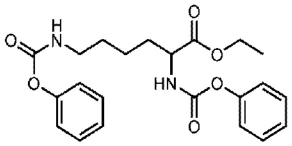
(VI - 10)

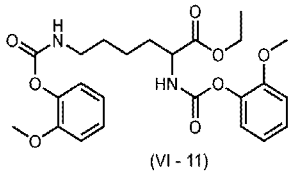
(VI - 11)

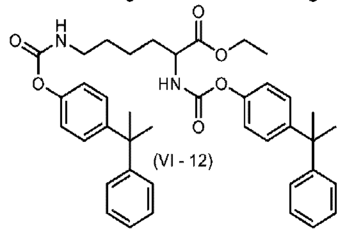
(VI - 12)

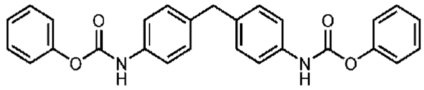
(VI - 13)

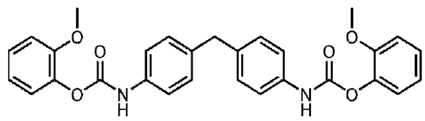
(VI - 14)

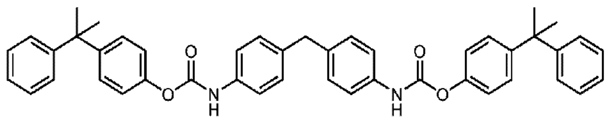
(VI - 15)

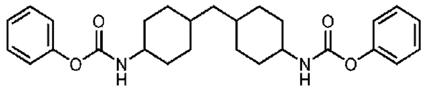
(VI - 16)

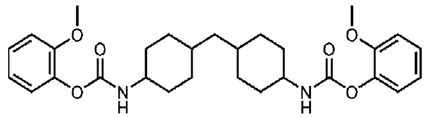
(VI - 17)

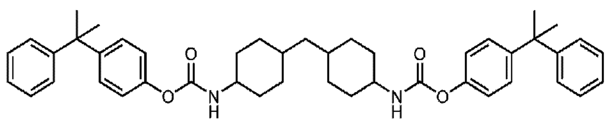
(VI - 18)

[Chem. 39]

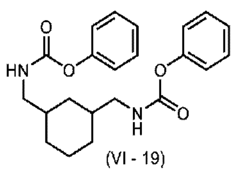
(VI - 19)

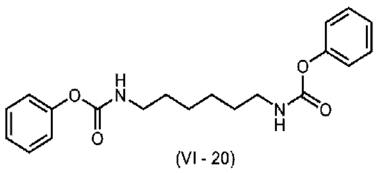
(VI - 20)

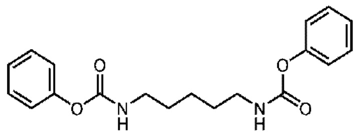
(VI - 21)

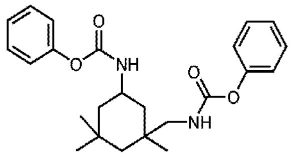
(VI - 22)

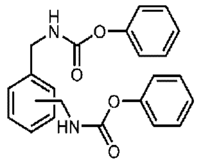
(VI - 23)

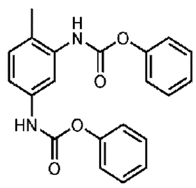
(VI - 24)

<Carbonate>

As the carbonate, a compound represented by General Formula (IV) (hereinafter, referred to as "carbonate (IV)" in some cases) is preferably used.

[Chem. 40]

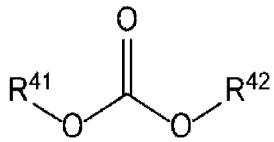

(IV)

(In General Formula (IV), $R^{41}$ and $R^{42}$ are each independently a monovalent organic group, and satisfy a relational formula: $R^{41} = R^{42} = R^{12}$.)

[$R^{41}$ and $R^{42}$]

$R^{41}$ and $R^{42}$ are the same as each other, and satisfy a relational formula: $R^{41} = R^{42} = R^{12}$. That is, examples of $R^{41}$ and $R^{42}$ include the same ones as those exemplified in $R^{12}$ described above.

Among these, $R^{41}$ and $R^{42}$ are preferably substituted or unsubstituted aryl groups.

In addition, as $R^{41}$ and $R^{42}$, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a cumylphenyl group (each isomer), a methoxyphenyl group (each isomer), or an ethoxyphenyl group (each isomer) is more preferable.

In addition, as $R^{41}$ and $R^{42}$, a phenyl group, a methoxyphenyl group (each isomer), or an ethoxyphenyl group (each isomer) is further more preferable.

Examples of the preferable carbonate (IV) include diphenyl carbonate, bis(4-cumylphenyl)carbonate, bis(2-methoxyphenyl)carbonate, and bis(2-ethoxyphenyl)carbonate.

<Hydroxy compound>

As the hydroxy compound, a compound represented by General Formula (V) (hereinafter, referred to as "hydroxy compound (V)" in some cases) is preferably used.

[Chem. 41]

$R^{51}$-OH (V)

(In General Formula (V), $R^{51}$ is a monovalent organic group, and satisfies a relational formula: $R^{51} = R^{12}$.)

[$R^{51}$]

$R^{51}$ is a monovalent organic group, and satisfies a relational formula: $R^{51} = R^{12}$, That is, $R^{51}$ is the same as $R^{12}$.

Among these, as $R^{51}$, an aliphatic hydrocarbon group having 1 or more and 20 or less carbon atoms or an aromatic hydrocarbon group having 6 or more and 20 or less carbon atoms, which may contain an oxygen atom, is preferable.

In addition, as $R^{51}$, specifically, a phenyl group, a naphthyl group, an anthryl group, a pyrenyl group, a phenanthryl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a heptylphenyl group (each isomer), an octylphenyl group (each isomer), a nonylphenyl group (each isomer), a decylphenyl group (each isomer), an undecylphenyl group (each isomer), a dodecylphenyl group (each isomer), a tridecylphenyl group (each isomer), a tetradecylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a methylhexylphenyl group (each isomer), a methylheptylphenyl group (each isomer), a methyloctylphenyl group (each isomer), a methylnonylphenyl group (each isomer), a methyldecylphenyl group (each isomer), a methylundecylphenyl group (each isomer), a methyldodecylphenyl group (each isomer), a methyltridecylphenyl group (each isomer), a diethylphenyl group (each isomer), an ethylpropylphenyl group (each isomer), an ethylbutylphenyl group (each isomer), an ethylpentylphenyl group (each isomer), an ethylhexylphenyl group (each isomer), an ethylheptylphenyl group (each isomer), an ethyloctylphenyl group (each isomer), an ethylnonylphenyl group (each isomer), an ethyldecylphenyl group (each isomer), an ethylundecylphenyl group (each isomer), an ethyldodecylphenyl group (each isomer), a dipropylphenyl group (each isomer), a propylbutylphenyl group (each isomer), a propylpentylphenyl group (each isomer), a propylhexylphenyl group (each isomer), a propylheptylphenyl group (each isomer), a propyloctylphenyl group (each isomer), a propylnonylphenyl group (each isomer), a propyldecylphenyl group (each isomer), a propylundecylphenyl group (each isomer), a dibutylphenyl group (each isomer), a butylpentylphenyl group (each isomer), a butylhexylphenyl group (each isomer), a butylheptylphenyl group (each isomer), a butyloctylphenyl group (each isomer), a butylnonylphenyl group (each isomer), a butyldecylphenyl group (each isomer), a dipentylphenyl group (each isomer), a pentylhexylphenyl group (each isomer), a pentylheptylphenyl group (each isomer), a pentyloctylphenyl group (each isomer), a pentylnonylphenyl group (each isomer), a dihexylphenyl group (each isomer), a hexylheptylphenyl group (each isomer), a hexyloctylphenyl group (each isomer), a diheptylphenyl group (each isomer), a trimethylphenyl group (each isomer), a dimethylethylphenyl group (each isomer), a dimethylpropylphenyl group (each isomer), a dimethylbutylphenyl group (each isomer), a dimethylpentylphenyl group (each isomer), a dimethylhexylphenyl group (each isomer), a dimethylheptylphenyl group (each isomer), a dimethyloctylphenyl group (each isomer), a dimethylnonylphenyl group (each isomer), a dimethyldecylphenyl group (each isomer), a dimethylundecylphenyl group (each isomer), a dimethyldodecylphenyl group (each isomer), a triethylphenyl group (each isomer), a diethylmethylphenyl group (each isomer), a diethylpropylphenyl group (each isomer), a diethylbutylphenyl group (each isomer), a diethylpentylphenyl group (each isomer), a diethylhexylphenyl group (each isomer), a diethylheptylphenyl group (each isomer), a diethyloctylphenyl group (each isomer), a diethylnonylphenyl group (each isomer), a diethyldecylphenyl group (each isomer), a tripropylphenyl group (each isomer), a dipropylmethylphenyl group (each isomer), a dipropylethylphenyl group (each isomer), a dipropylbutylphenyl group (each isomer), a dipropylpentylphenyl group (each isomer), a dipropylhexylphenyl group (each isomer), a dipropylheptylphenyl group (each isomer), a dipropyloctylphenyl group (each isomer), a tributylphenyl group (each isomer), a dibutylmethylphenyl group (each isomer), a dibutylethylphenyl group (each isomer), a dibutylpropylphenyl group (each isomer), a dibutylpentylphenyl group (each isomer), a dibutylhexylphenyl group (each isomer), a cumylphenyl group (each isomer), a methoxyphenyl group (each isomer), or an ethoxyphenyl group (each isomer) is

preferable. In addition, as $R^{51}$, a phenyl group, a cumylphenyl group (each isomer), a methoxyphenyl group (each isomer), or an ethoxyphenyl group (each isomer) is more preferable.

Examples of the preferable hydroxy compound (V) include an aromatic hydroxy compound (hereinafter, referred to as "aromatic hydroxy compound (V-1)" in some cases) represented by General Formula (V-1).

[Chem. 42]

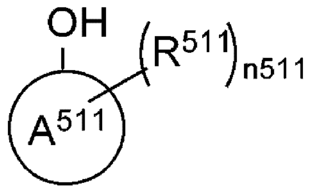

(V-1)

(In General Formula (V-1), a ring $A^{511}$ is an aromatic hydrocarbon ring having 6 or more and 20 or less carbon atoms. $R^{511}$ is a hydrogen atom, an alkyl group having 1 or more and 20 or less carbon atoms, an alkoxy group having 1 or more and 20 or less carbon atoms, an aryl group having 6 or more and 20 or less carbon atoms, an aryloxy group having 6 or more and 20 or less carbon atoms, an aralkyl group having 7 or more and 20 or less carbon atoms, an aralkyloxy group having 7 or more and 20 or less carbon atoms, or a hydroxy group. $R^{511}$ may bind to the ring $A^{511}$ to form a ring structure. In addition, n511 is an integer of 1 or more and 10 or less.)

[$R^{511}$]

Examples of the alkyl group having 1 or more and 20 or less carbon atoms in $R^{511}$ include a methyl group, an ethyl group, a propyl group (each isomer), a butyl group (each isomer), a pentyl group (each isomer), a hexyl group (each isomer), a heptyl group (each isomer), an octyl group (each isomer), a nonyl group (each isomer), a decyl group (each isomer), a dodecyl group (each isomer), an octadecyl group (each isomer), and the like.

Examples of the alkoxy group having 1 or more and 20 or less carbon atoms in $R^{511}$ include a methoxy group, an ethoxy group, a propoxy group (each isomer), a butyloxy group (each isomer), a pentyloxy group (each isomer), a hexyloxy group (each isomer), a heptyloxy group (each isomer), an octyloxy group (each isomer), a nonyloxy group (each isomer), a decyloxy group (each isomer), a dodecyloxy group (each isomer), an octadecyloxy group (each isomer), and the like.

Examples of the aryl group having 6 or more and 20 or less carbon atoms in $R^{511}$ include a phenyl group, a naphthyl group, and the like.

Examples of the aryl group having an alkyl group as a substituent in $R^{511}$ include a methylphenyl group (each isomer), an ethylphenyl group (each isomer), a propylphenyl group (each isomer), a butylphenyl group (each isomer group), a pentylphenyl group (each isomer), a hexylphenyl group (each isomer), a heptylphenyl group (each isomer), an octylphenyl group (each isomer), a nonylphenyl group (each isomer), a decylphenyl group (each isomer), a biphenyl group (each isomer), a dimethylphenyl group (each isomer), a diethylphenyl group (each isomer), a dipropylphenyl group (each isomer), a dibutylphenyl group (each isomer), a dipentylphenyl group (each isomer), a dihexylphenyl group (each isomer), a diheptylphenyl group (each isomer), a terphenyl group (each isomer), a trimethylphenyl group (each isomer), a triethylphenyl group (each isomer), a tripropylphenyl group (each isomer), a tributylphenyl group (each isomer), and the like.

Examples of the aryloxy group having 6 or more and 20 or less carbon atoms in $R^{511}$ include a phenoxy group, a methylphenoxy group (each isomer), an ethylphenoxy group (each isomer), a propylphenoxy group (each isomer), a butylphenoxy group (each isomer), a pentylphenoxy group (each isomer), a hexylphenoxy group (each isomer), a heptylphenoxy group (each isomer), an octylphenoxy group (each isomer), a nonylphenoxy group (each isomer), a decylphenoxy group (each isomer), a phenylphenoxy group (each isomer), a dimethylphenoxy group (each isomer), a diethylphenoxy group (each isomer), a dipropylphenoxy group (each isomer), a dibutylphenoxy group (each isomer), a dipentylphenoxy group (each isomer), a dihexylphenoxy group (each isomer), a diheptylphenoxy group (each isomer), a diphenylphenoxy group (each isomer), a trimethylphenoxy group (each isomer), a triethylphenoxy group (each isomer), a tripropylphenoxy group (each isomer), a tributyl phenoxy group (each isomer), and the like.

Examples of the aralkyl group having 7 or more and 20 or less carbon atoms in $R^{511}$ include a phenylmethyl group, a phenylethyl group (each isomer), a phenylpropyl group (each isomer), a phenylbutyl group (each isomer), a phenylpentyl group (each isomer), a phenylhexyl group (each isomer), a phenylheptyl group (each isomer), a phenyloctyl group (each isomer), a phenylnonyl group (each isomer), and the like.

Examples of the aralkyloxy group having 7 or more and 20 or less carbon atoms in $R^{511}$ include a phenylmethoxy

group, a phenylethoxy group (each isomer), a phenylpropyloxy group (each isomer), a phenylbutyloxy group (each isomer), a phenylpentyloxy group (each isomer), a phenylhexyloxy group (each isomer), a phenylheptyloxy group (each isomer), a phenyloctyloxy group (each isomer), a phenylnonyloxy group (each isomer), and the like.

[$A^{511}$]

The ring $A^{511}$ is an aromatic hydrocarbon ring having 6 or more and 20 or less carbon atoms. The ring $A^{511}$ may be a monocyclic ring, may be a polycyclic ring, or may be a condensed ring.

Specific examples of the ring $A^{511}$ include a benzene ring, a naphthalene ring, an anthracene ring, a phenanthrene ring, an aphthacene ring, a chrysene ring, a pyrene ring, a triphenylene ring, a pentalene ring, an azulene ring, a heptalene ring, an indacene ring, a biphenylene ring, an acenaphthylene ring, an aceanthrylene ring, an acephenanthrylene ring, and the like. Among these, as the ring $A^{511}$, a benzene ring, a naphthalene ring, or an anthracene ring is preferable, and a benzene ring is more preferable.

In addition, these rings may have a substituent other than $R^{511}$ described above. Examples of the substituent other than $R^{511}$ include the same substituents as those exemplified in $R^{511}$. $R^{511}$ and the substituents other than $R^{511}$ consist of different functional groups.

[n511]

n511 indicates the number of substituents $R^{511}$ and is an integer of 1 or more and 10 or less.

In General Formula (V-1), examples of the compound in which the ring $A^{511}$ is a benzene ring include a compound represented by General Formula (V-1-1) (hereinafter, referred to as "hydroxy compound (V-1-1)" in some cases), and the like.

[Chem. 43]

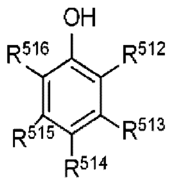

(V-1-1)

(In General Formula (V-1-1), $R^{512}$ to $R^{516}$ are each independently the same as that for $R^{511}$.)

Among these, it is preferable that at least one of $R^{512}$ to $R^{516}$ be a hydrogen atom, and it is more preferable that all of $R^{512}$ to $R^{516}$ be hydrogen atoms.

Examples of the preferable hydroxy compound (V-1-1) include phenol, 2-ethylphenol, 2-propylphenol (each isomer), 2-butylphenol (each isomer), 2-pentylphenol (each isomer), 2-hexylphenol (each isomer), 2-heptylphenol (each isomer), 2-phenylphenol, 2,6-dimethylphenol, 2,4-diethylphenol, 2,6-diethylphenol, 2,4-dipropylphenol (each isomer), 2,6-dipropylphenol (each isomer), 2,4-dibutylphenol (each isomer), 2,4-dipentylphenol (each isomer), 2,4-dihexylphenol (each isomer), 2,4-diheptylphenol (each isomer), 2-methyl-6-ethylphenol, 2-methyl-6-propylphenol (each isomer), 2-methyl-6-butylphenol (each isomer), 2-methyl-6-pentylphenol (each isomer), 2-ethyl-6-propylphenol (each isomer), 2-ethyl-6-butylphenol (each isomer), 2-ethyl-6-pentylphenol (each isomer), 2-propyl-6-butylphenol (each isomer), 2-ethyl-4-methylphenol (each isomer), 2-ethyl-4-propylphenol (each isomer), 2-ethyl-4-butylphenol (each isomer), 2-ethyl-4-pentylphenol (each isomer), 2-ethyl-4-hexylphenol (each isomer), 2-ethyl-4-heptylphenol (each isomer), 2-ethyl-4-octylphenol (each isomer), 2-ethyl-4-phenylphenol (each isomer), 2-ethyl-4-cumylphenol (each isomer), 2-propyl-4-methylphenol (each isomer), 2-propyl-4-ethylphenol (each isomer), 2-propyl-4-butylphenol (each isomer), 2-propyl-4-pentylphenol (each isomer), 2-propyl-4-hexylphenol (each isomer), 2-propyl-4-heptylphenol (each isomer), 2-propyl-4-octylphenol (each isomer), 2-propyl-4-phenylphenol (each isomer), 2-propyl-4-cumylphenol (each isomer), 2-butyl-4-methylphenol (each isomer), 2-butyl-4-ethylphenol (each isomer), 2-butyl-4-propylphenol (each isomer), 2-butyl-4-pentylphenol (each isomer), 2-butyl-4-hexylphenol (each isomer), 2-butyl-4-heptylphenol (each isomer), 2-butyl-4-octylphenol (each isomer), 2-butyl-4-phenylphenol (each isomer), 2-butyl-4-cumylphenol (each isomer), 2-pentyl-4-methylphenol (each isomer), 2-pentyl-4-ethylphenol (each isomer), 2-pentyl-4-propylphenol (each isomer), 2-pentyl-4-butylphenol (each isomer), 2-pentyl-4-hexylphenol (each isomer), 2-pentyl-4-heptylphenol (each isomer), 2-pentyl-4-octylphenol (each isomer), 2-pentyl-4-phenylphenol (each isomer), 2-pentyl-4-cumylphenol (each isomer), 2-hexyl-4-methylphenol (each isomer), 2-hexyl-4-ethylphenol (each isomer), 2-hexyl-4-propylphenol (each isomer), 2-hexyl-4-butylphenol (each isomer), 2-hexyl-4-pentylphenol (each isomer), 2-hexyl-4-heptylphenol (each isomer), 2-hexyl-4-octylphenol (each isomer), 2-hexyl-4-phenylphenol (each isomer), 2-hexyl-4-cumylphenol (each isomer), 2-heptyl-4-methylphenol (each isomer), 2-heptyl-4-ethylphenol (each isomer), 2-heptyl-4-propylphenol (each isomer), 2-heptyl-4-butylphenol (each isomer), 2-heptyl-4-pentylphenol (each isomer), 2-heptyl-4-hexylphenol (each isomer), 2-heptyl-4-octylphenol (each isomer), 2-heptyl-4-phenylphenol (each isomer), 2-heptyl-4-cumylphenol (each isomer), 2,4,6-trimethylphenol, 2,6-dimethyl-4-ethylphenol, 2,6-dimethyl-4-propylphenol (each isomer), 2,6-dimethyl-4-butylphenol (each isomer), 2,6-dimethyl-4-pentylphenol (each isomer), 2,6-dimethyl-4-hexylphenol (each isomer), 2,6-dimethyl-4-phenylphenol, 2,6-dimethyl-4-cumylphenol, 2,4,6-triethylphenol, 2,6-diethyl-4-methylphenol, 2,6-diethyl-4-propylphenol (each isomer), 2,6-diethyl-4-butylphenol (each isomer), 2,6-diethyl-4-pentylphenol (each isomer), 2,6-diethyl-4-hexylphenol (each isomer), 2,6-diethyl-4-phenylphenol, 2,6-diethyl-4-cumylphenol, 2,4,6-tripropylphenol (each isomer), 2,6-dipropyl-4-ethylphenol (each isomer), 2,6-dipropyl-4-methylphenol (each isomer), 2,6-dipropyl-4-butylphenol (each isomer), 2,6-dipropyl-4-pentylphenol (each isomer), 2,6-dipropyl-4-hexylphenol (each isomer), 2,6-dipropyl-4-phenylphenol (each isomer), 2,6-dipropyl-4-cumylphenol (each isomer), 2,4-dimethyl-6-ethylphenol, 2-methyl-4,6-diethylphenol, 2-methyl-4-propyl-6-ethylphenol (each isomer), 2-methyl-4-butyl-6-ethylphenol (each isomer), 2-methyl-4-pentyl-6-ethylphenol (each isomer), 2-methyl-4-hexyl-6-ethylphenol (each isomer), 2-methyl-4-phenyl-6-ethylphenol (each isomer), 2-methyl-4-cumyl-6-ethylphenol (each isomer), 2,4-dimethyl-6-propylphenol (each isomer), 2-methyl-4,6-dipropylphenol (each isomer), 2-methyl-4-ethyl-6-propylphenol (each isomer), 2-methyl-4-butyl-6-propylphenol (each isomer), 2-methyl-4-pentyl-6-propylphenol (each isomer), 2-methyl-4-hexyl-6-propylphenol (each isomer), 2-methyl-4-phenyl-6-propylphenol (each isomer), 2-methyl-4-cumyl-6-propylphenol (each isomer), 2,4-dimethyl-6-butylphenol, 2-methyl-4,6-dibutylphenol, 2-methyl-4-propyl-6-butylphenol (each isomer), 2-methyl-4-ethyl-6-butylphenol (each isomer), 2-methyl-4-pentyl-6-butylphenol (each isomer), 2-methyl-4-hexyl-6-butylphenol (each isomer), 2-methyl-4-phenyl-6-butylphenol (each isomer), 2-methyl-4-cumyl-6-butylphenol (each isomer), 2,4-dimethyl-6-pentylphenol, 2-methyl-4,6-dipentylphenol, 2-methyl-4-propyl-6-pentylphenol (each isomer), 2-methyl-4-butyl-6-pentylphenol (each isomer), 2-methyl-4-ethyl-6-pentylphenol (each isomer), 2-methyl-4-hexyl-6-pentylphenol (each isomer), 2-methyl-4-phenyl-6-pentylphenol (each isomer), 2-methyl-4-cumyl-6-pentylphenol (each isomer), 2,4-dimethyl-6-hexylphenol, 2-methyl-4,6-dihexylphenol, 2-methyl-4-propyl-6-hexylphenol (each isomer), 2-methyl-4-butyl-6-hexylphenol (each isomer), 2-methyl-4-pentyl-6-hexylphenol (each isomer), 2-methyl-4-ethyl-6-hexylphenol (each isomer), 2-methyl-4-phenyl-6-hexylphenol (each isomer), 2-methyl-4-cumyl-6-hexylphenol (each isomer), 2-ethyl-4-methyl-6-propylphenol (each isomer), 2,4-diethyl-6-propylphenol (each isomer), 2-ethyl-4,6-propylphenol (each isomer), 2-ethyl-4-butyl-6-propylphenol (each isomer), 2-ethyl-4-pentyl-6-propylphenol (each isomer), 2-ethyl-4-hexyl-6-propylphenol (each isomer), 2-ethyl-4-heptyl-6-propylphenol (each isomer), 2-ethyl-4-octyl-6-propylphenol (each isomer), 2-ethyl-4-phenyl-6-propylphenol (each isomer), 2-ethyl-4-cumyl-6-propylphenol (each isomer), 2-ethyl-4-methyl-6-butylphenol (each isomer), 2,4-diethyl-6-butylphenol (each isomer), 2-ethyl-4,6-butylphenol (each isomer), 2-ethyl-4-propyl-6-butylphenol (each isomer), 2-ethyl-4-pentyl-6-butylphenol (each isomer), 2-ethyl-4-hexyl-6-butylphenol (each isomer), 2-ethyl-4-heptyl-6-butylphenol (each isomer), 2-ethyl-4-octyl-6-butylphenol (each isomer), 2-ethyl-4-phenyl-6-butylphenol (each isomer), 2-ethyl-4-cumyl-6-butylphenol (each isomer), 2-ethyl-4-methyl-6-pentylphenol (each isomer), 2,4-diethyl-6-pentylphenol (each isomer), 2-ethyl-4,6-pentylphenol (each isomer), 2-ethyl-4-butyl-6-pentylphenol (each isomer), 2-ethyl-4-propyl-6-pentylphenol (each isomer), 2-ethyl-4-hexyl-6-pentylphenol (each isomer), 2-ethyl-4-heptyl-6-pentylphenol (each isomer), 2-ethyl-4-octyl-6-pentylphenol (each isomer), 2-ethyl-4-phenyl-6-pentylphenol (each isomer), 2-ethyl-4-cumyl-6-pentylphenol (each isomer), 2-ethyl-4-methyl-6-hexylphenol (each isomer), 2,4-diethyl-6-hexylphenol (each isomer), 2-ethyl-4,6-hexylphenol (each isomer), 2-ethyl-4-propyl-6-hexylphenol (each isomer), 2-ethyl-4-pentyl-6-hexylphenol (each isomer), 2-ethyl-4-butyl-6-hexylphenol (each isomer), 2-ethyl-4-heptyl-6-hexylphenol (each isomer), 2-ethyl-4-octyl-6-hexylphenol (each isomer), 2-ethyl-4-phenyl-6-hexylphenol (each isomer), 2-ethyl-4-cumyl-6-hexylphenol (each isomer), 2-propyl-4-methyl-6-butylphenol (each isomer), 2,4-dipropyl-6-butylphenol (each isomer), 2-propyl-4,6-butylphenol (each isomer), 2-propyl-4-ethyl-6-butylphenol (each isomer), 2-propyl-4-pentyl-6-butylphenol (each isomer), 2-propyl-4-hexyl-6-butylphenol (each isomer), 2-propyl-4-heptyl-6-butylphenol (each isomer), 2-propyl-4-octyl-6-butylphenol (each isomer), 2-propyl-4-phenyl-6-butylphenol (each isomer), cumylphenol (each isomer), 2-propyl-4-cumyl-6-butylphenol (each isomer), 2,4-dicumylphenol, methoxyphenol (each isomer), ethoxyphenol (each isomer), and the like. Among these, phenol, cumylphenol (each isomer), methoxyphenol (each isomer), or ethoxyphenol (each isomer) is preferable.

<<Method for producing isocyanate compound»

A method for producing an isocyanate compound of the present embodiment includes performing distillation purification on a reaction solution including an isocyanate compound (II) in the presence of the above-described carbonyl compound (I), and continuously recovering the isocyanate compound (II) as a gas-phase component.

In the method for producing an isocyanate compound of the present embodiment, by using the above-described carbonyl compound (I), it is possible to prevent a by-product from being fixed to a device at the time of production of the isocyanate compound (II), and to improve a yield of the isocyanate compound (II).

The method for producing an isocyanate compound of the present embodiment will be described in detail below.

The isocyanate compound (II) is obtained by carrying out a pyrolysis reaction of the carbamate compound (VI) in the presence of the carbonate (IV).

[Pyrolysis step]

In the pyrolysis step, an isocyanate (II) is obtained by carrying out a pyrolysis reaction of the carbamate compound (VI) in the presence of the carbonate (IV) as a solvent. In the pyrolysis reaction, as side reactions represented by Formulae (B) to (E) occur and the generated isocyanurate group, the carbodiimide group, and the allophanate group act as crosslinking points, a high-molecular-weight component is generated, and purification of a solid substance or an increase in the liquid viscosity may occur. As the carbonyl compound (I) is present in the pyrolysis reaction system, the carbonyl compound (I) acts as a good solvent for the above-described high-molecular-weight component, and the carbonyl compound (I) acts as a terminal sealing agent to suppress the increae in the molecular weight of by-products, thereby having an effect of suppressing the generation of solid substances or increase in liquid viscosity during the pyrolysis reaction.

The carbonate (IV) may be supplied to the reactor before starting the reaction, may be supplied during the reaction, or may be supplied to the reactor in both cases. Among these, it is preferable that the carbonate be supplied to the reactor before the reaction.

In the pyrolysis step, it is preferable that a use amount (molar amount) of the carbonate (IV) as the solvent be large from the viewpoint of suppressing side reactions, but in consideration of the size of the reactor, the use amount is preferably 0.001 times or more and 100 times or less, more preferably 0.01 times or more and 80 times or less, and further more preferably 0.1 times or more and 50 times or less, in terms of the stoichiometric ratio with respect to the carbamate compound.

In a case where the carbamate compound contains the carbonate (IV) in a case of producing the carbamate compound by the method to be described later, the carbonate (IV) may be used as it is, or the carbonate (IV) may be newly added to the carbamate compound (VI).

A reaction temperature is usually 100°C or higher and 400°C or lower, and a high temperature is preferable to increase the reaction rate. On the other hand, since there is a case where the above-described side reaction occurs due to one or more compounds selected from the group consisting of a carbamate compound and an isocyanate compound which is a product, at a high temperature, the reaction temperature is preferably 130°C or higher and 300°C or lower, and more preferably 150°C or higher and 280°C or lower. To keep the reaction temperature constant, known cooling devices and heating devices may be installed in the reactor.

In addition, a reaction pressure varies depending on the type of the compound used or the reaction temperature, but the reaction pressure may be any one of reduced pressure, normal pressure, or pressurized pressure, and the reaction pressure is usually in a range of 20 Pa or more and $1 \times 10^6$ Pa or less.

A reaction time (retention time in the case of a continuous method) is not particularly limited, but is usually 0.001 hours or longer and 100 hours or shorter, preferably 0.01 hours or longer and 50 hours or shorter, and more preferably 0.1 hours or longer and 10 hours or shorter.

A catalyst can be used, and a use amount of the catalyst is preferably 0.01% by mass or more and 30% by mass or less, and more preferably 0.5% by mass or more and 20% by mass or less, with respect to the mass of the carbamate compound.

Examples of the catalyst include organic metal catalysts such as dibutyltin dilaurate, lead octylate, and stannous octoate; and amines such as 1,4-diazabicyclo[2,2,2]octane, triethylenediamine, and triethylamine. Among them, an organic metal catalyst such as dibutyltin dilaurate, lead octylate, and stanaoctoate is suitable. These compounds may be used alone or a mixture of two or more types thereof may be used.

As described above, the pyrolysis reaction is a reaction of generating the corresponding isocyanate compound (II) and a hydroxy compound from the carbamate compound (VI), but the pyrolysis reaction is an equilibrium reaction. Therefore, to efficiently obtain the isocyanate compound (II) in the pyrolysis reaction, it is preferable to extract a hydroxy compound, which is the product in the pyrolysis reaction, as a gas-phase component from the pyrolysis reaction system by, for example, a method such as distillation or the like.

[Isocyanate composition preparation step]

Since a reaction solution obtained in the pyrolysis step includes the carbonyl compound (I), the reaction solution may be used as a reaction solution (isocyanate composition) including the isocyanate compound (II) and the carbonyl compound (I) in the purification step to be described later. Alternatively, a composition (isocyanate composition) obtained by mixing the carbonyl compound (I) obtained in the "method for producing a carbonyl compound" with a reaction solution including an isocyanate compound may be prepared and used in the purification step to be described later.

In the purification step to be described later, as the composition of the isocyanate composition, from the viewpoint

of ensuring good distillation operability, a value of {3 × (molar amount of isocyanurate group) + 2 × (molar amount of carbodiimide group) + 3 × (molar amount of uretonimine group) + 2 × (molar amount of allophanate group) [/(molar amount of carbonyl compound) is preferably 0.00001 or more and 80.0 or less, more preferably 0.0001 or more and 40.0 or less, and further more preferably 0.001 or more and 8.0 or less.

[Purification step]

In the purification step, the isocyanate compound is purified from the above-described isocyanate composition. In the purification step, specifically, first, a component (light boiling component) having a boiling point lower than that of the isocyanate compound contained in the isocyanate composition is distilled off (hereinafter, referred to as "light boiling separation"), and then the isocyanate compound is recovered as a gas-phase component, and separated from a component having a boiling point higher than that of the isocyanate compound (high boiling component) (hereinafter, referred to as "high-boiling separation") to purify the isocyanate compound from the isocyanate composition.

(Light boiling separation step)

The distillation method is not particularly limited as long as the light boiling component can be separated as a gas-phase component. The "light boiling component (light boiling point component)" referred to herein refers to a component having a boiling point lower than that of the carbonyl compound, and varies depending on the type of the compound serving as a raw material for producing the isocyanate compound, but is mainly one or more compounds selected from the group consisting of a carbonate used in the pyrolysis step and a hydroxy compound produced as a by-product in the pyrolysis reaction.

A pressure at the time of distilling off the light boiling component varies depending on the type of the compound or the reaction temperature, but the pressure may be any of reduced pressure, normal pressure, and pressurized pressure as long as the isocyanate compound and the light boiling component can be separated, and is preferably 20 Pa or more and $1 \times 10^6$ Pa or less, more preferably 20 Pa or more and $1 \times 10^4$ Pa or less, further more preferably 20 Pa or more and $1 \times 10^3$ Pa or less, and particularly preferably 20 Pa or more and $1 \times 10^2$ Pa or less.

An operation time (retention time in the case of a continuous method) at the time of distilling off the light boiling component is not particularly limited as long as the isocyanate compound and the light boiling component can be separated, and, from the viewpoint of suppressing a side reaction between the isocyanate compound, is preferably 5 seconds or longer and 100 hours or shorter, more preferably 10 seconds or longer and 50 hours or shorter, and further more preferably 20 seconds or longer and 10 hours or shorter.

A temperature at the time of distilling off the light boiling component is not particularly limited as long as the isocyanate compound is stable and the isocyanate compound and the light boiling component can be separated, and, from the viewpoint of suppressing denaturation of the isocyanate compound, is preferably 20°C or higher and 300°C or lower, more preferably 30°C or higher and 280°C or lower, and further more preferably 40°C or higher and 250°C or lower.

(High boiling separation step)

The distillation method is not particularly limited as long as the isocyanate can be separated as a gas-phase component. The "high boiling component (high-boiling-point component)" referred to herein refers to a component having a boiling point higher than that of the isocyanate, and varies depending on the type of the compound serving as a raw material for producing the isocyanate compound, but is mainly a carbonyl compound generated in the pyrolysis step, a carbamate compound (III) (carbato group-containing isocyanate) and a carbamate compound (VI) (carbamate compound serving as a raw material in pyrolysis step), or a compound in which a part of the isocyanate group of the isocyanate compound is transformed into at least one type of functional group selected from the group consisting of a isocyanurate group, a carbodiimide group, a uretonimine group, and an allophanate group (hereinafter, referred to as "isocyanate polymer").

In the high boiling separation step, since a uretonimine group produced as a by-product in the pyrolysis step or the light boiling separation step undergoes a reaction in which the uretonimine group is regenerated to an isocyanate group and a carbodiimide group as shown in Formula (J), there is a case where a recovery rate of the isocyanate compound recovered in the gas phase exceeds 100% by mass.

[Chem. 44]

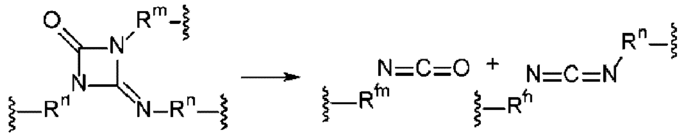

( J )

(In Formula (J), $R^m$ and $R^n$ are each independently a di- or higher valent organic group.)

On the other hand, in the high boiling separation step, the isocyanate compound is distilled off, and thus the high boiling component is thickened and solidified, making it difficult to continue the operation. Therefore, it becomes difficult to recover the isocyanate compound in a high yield. This is caused because the isocyanate (isocyanate polymer), a part of which is transformed into at least one type of functional group selected from the group consisting of an isocyanurate group, a carbodiimide group, a uretonimine group, and an allophanate group, has a high molecular weight, or the carbodiimide group produced by Formula (J) during high boiling separation and the isocyanate group of the isocyanate polymer are bound to form a uretonimine group (reverse reaction of Formula (J), and isocyanate polymers are bound to have a high molecular weight.

Since the carbonyl compound (I) has a higher boiling point than the isocyanate compound and has fewer crosslinking points than the isocyanate polymer, the carbonyl compound (I) acts as a solvent in the high boiling separation step. Alternatively, the carbonyl compound (I) is bound to a carbodiimide group, thereby preventing isocyanate polymers from being bound to each other to have a high molecular weight. Due to the action, the operability in the high boiling separation step is improved, and the isocyanate compound can be recovered in a high yield.

A pressure at the time of separating a high boiling component varies depending on the type of the compound or the reaction temperature, but the pressure may be any of reduced pressure, normal pressure, and pressurized pressure as long as the isocyanate compound and the light boiling component can be separated, and is preferably 0.1 Pa or more and $1 \times 10^6$ Pa or less, more preferably 1 Pa or more and $1 \times 10^4$ Pa or less, and further more preferably 5 Pa or more and $1 \times 10^3$ Pa or less.

An operation time (retention time in the case of a continuous method) at the time of separating the high boiling component is not particularly limited as long as the isocyanate compound and the light boiling component can be separated, and, from the viewpoint of suppressing a side reaction between the isocyanate compound, is preferably 5 seconds or longer and 100 hours or shorter, more preferably 10 seconds or longer and 50 hours or shorter, further more preferably 15 seconds or longer and 10 hours or shorter, particularly preferably 20 seconds or longer and 1 hour or shorter, and most preferably 25 seconds or longer and 10 minutes or shorter.

A temperature at the time of separating the high boiling component is not particularly limited as long as the isocyanate compound is stable and the isocyanate compound and the light boiling component can be separated, and, from the viewpoint of suppressing denaturation of the isocyanate compound, is preferably 20°C or higher and 250°C or lower, more preferably 30°C or higher and 230°C or lower, and further more preferably 40°C or higher and 200°C or lower.

[Device and material]

The materials of the reactor and the manufacturing line in which the pyrolysis step and the purification step are performed may be any known materials as long as the materials do not adversely affect the carbamate compound, the hydroxy compound and the isocyanate compound which are the products, and the carbonate ester which is the solvent, but SUS304, SUS316, SUS316L, and the like are inexpensive and can be preferably used.

The form of the reactor is not particularly limited, and known tank-like or column-like reactors can be used. In the pyrolysis reaction and the subsequent distillation of the light boiling component, a reactor including a line in which a low boiling point mixture containing the produced hydroxy compound is extracted from the reactor as a gas component, and a part or all of the mixture solution including an unreacted carbamate compound or a compound that is not extracted as the gas component is extracted from the reactor in a liquid state is preferably used. Various known methods, such as a method of using a reactor equipped with a stirring tank, a multi-stage stirring tank, a distillation column, a multi-stage distillation column, a multi-tube reactor, a continuous multi-stage distillation column, a filled column, a thin film evaporator, and a reactor having a support therein, a forced circulation reactor, and a reactor including any one of a falling film evaporator, a dropping evaporator, a small flow phase reactor, and a bubbling column, as such a reactor, and a method combining these, are used.

From the viewpoint of separating the hydroxy compound and the isocyanate compound to be generated, a method of using a stirring tank or a multi-stage stirring tank equipped with a distillation column is preferable, and a structure having a large gas-liquid contact area capable of quickly moving a low boiling point component to be generated in a gas phase is preferable.

In the light boiling separation step, in the purification step of an isocyanate composition, a reactor including a line in which a light boiling component is extracted from the reactor as a gas component, and a part or all of a mixture solution including a compound that is not extracted is extracted from the reactor in a liquid state is preferably used. Various known methods, such as a method of using a reactor equipped with a stirring tank, a multi-stage stirring tank, a distillation column, a multi-stage distillation column, a multi-tube reactor, a continuous multi-stage distillation column, a filled column, a thin film evaporator, and a reactor having a support therein, a forced circulation reactor, and a reactor including any one of a falling film evaporator, a dropping evaporator, a small flow phase reactor, and a bubbling column, as such a reactor, and a method combining these, are used.

In addition, in the high boiling separation step, in the purification steps of the isocyanate composition, a reactor including a line in which an isocyanate compound is extracted from the reactor as a gas component, and a part or all of a mixture including a compound that is not extracted is extracted from the reactor in a liquid state is preferably used. Various known methods, such as a method of using a reactor equipped with a stirring tank, a multi-stage stirring tank, a distillation column, a multi-stage distillation column, a multi-tube reactor, a continuous multi-stage distillation column, a filled column, a thin film evaporator, and a reactor having a support therein, a forced circulation reactor, and a reactor including any one of a falling film evaporator, a dropping evaporator, a small flow phase reactor, and a bubbling column, as such a reactor, and a method combining these, are used.

Subsequently, various raw materials used in the method for producing an isocyanate compound of the present embodiment will be described in detail below.

<Method for producing carbamate compound (VI)>

Examples of the carbamate compound (VI) are shown in the above-described "method for producing carbonyl compound".

As the method for producing a carbamate compound (VI), for example, a method for producing a carbamate compound (VI) from a carbonate derivative and an amine compound, or a method for producing a carbamate compound (VI) from a carbonate derivative, a hydroxy compound, and an amine compound is preferable. As the hydroxy compound serving as a raw material for producing the carbamate compound (VI), the same hydroxy compound as the hydroxy compound (V) is preferably used.

[Carbonate derivative]

Examples of the carbonate derivative include urea, carbonate, and the like.

As the carbonate serving as a raw material for producing the carbamate compound (VI), the same carbonate as the carbonate (IV) is preferably used. Among these, as the carbonate derivative, urea, diphenyl carbonate, or dibutyl carbonate is preferable, and urea or diphenyl carbonate is more preferable.

[Amine compound]

As the amine compound, for example, a compound represented by General Formula (VII) (hereinafter, referred to as "amine compound (VII)" in some cases) is preferably used.

[Chem. 45]

$$R^{71}\left(-NH_2\right)_{n71} \qquad (VII)$$

(In General Formula (VII), $R^{71}$ is an n71-valent organic group, and satisfies a relational formula: $R^{71} = R^{61}$. n71 is an integer of 2 or more and 8 or less, and satisfies a relational formula: n71 = n61.)

(R71)

$R^{71}$ is an n71-valent organic group, and satisfies a relational formula: $R^{71} = R^{61}$. That is, $R^{71}$ is the same as $R^{61}$.

Among these, $R^{71}$ is preferably a di- or higher valent and tetra- or lower valent aliphatic hydrocarbon group having 1 or more and 20 or less carbon atoms, or a di- or higher valent and tri- or lower valent aromatic hydrocarbon group having 6 or more and 20 or less carbon atoms, which may have 1 or more and 4 or less ester groups or nitrogen atoms.

In addition, specifically, $R^{71}$ is more preferably a group represented by any of Formulae (Ia-1) to (Ia-24), and

more preferably a group represented by Formulae (Ia-1), (Ia-2), (Ia-3), (Ia-14), (Ia-18), or (Ia-19).

(n71)

n71 represents the number of amino groups, and satisfies a relational formula: n71 = n61. n71 is an integer of 2 or more and 8 or less, preferably an integer of 2 or more and 6 or less, more preferably an integer of 2 or more and 5 or less, and further more preferably an integer of 3 or more and 4 or less.

Examples of preferable amine compounds (VII) include 4-aminomethyl-1,8-octanediamine, 4,4'-diaminodiphenylmethane, lysine β-aminoethyl ester, lysine methyl ester, 4,4'-methylenebis(cyclohexylamine), 1,3-di(aminomethyl)cyclohexane, and the like.

<<Isocyanate composition»

The isocyanate composition of the present embodiment contains

97% by mass or more of an isocyanate compound, and
2.0 ppm by mass or more and 1.0 × $10^4$ ppm by mass or less of a carbonyl compound represented by General Formula (I) (hereinafter, referred to as "carbonyl compound (I)" in some cases), with respect to a total mass of the isocyanate composition.

The isocyanate compound and the carbonyl compound are different compounds from each other.

[Chem. 46]

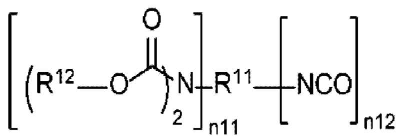

(I)

(In General Formula (I), $R^{11}$ is an (n11 + n12)-valent organic group, and $R^{12}$ is a monovalent organic group. n11 is an integer of 1 or more and 8 or less, n12 is an integer of 0 or more and 7 or less, and a sum of n11 and n12 is an integer of 2 or more and 8 or less).

In general, in a compound containing an unsaturated bond, the unsaturated bond tends to be easily oxidized, and the unsaturated bond compound as a contaminant easily causes coloring. However, the carbonyl compound (I) effectively acts during the storage of the isocyanate composition and exhibits an effect of improving the stability of the isocyanate compound without coloring the isocyanate composition. It is presumed that the effect is exhibited since the carbonyl group of the carbonyl compound (I) has reactivity with water or oxygen and suppresses a denaturation reaction of an isocyanate compound resulting from water or oxygen. In addition, since the carbonyl compound (I) has a large number of unsaturated bonds between carbon and oxygen, the carbonyl compound (I) tends to further exhibit the effect.

To suppress the denaturation reaction of the isocyanate compound, it is preferable to increase a content of the carbonyl compound (I), but in a case where the content is too large, coloring due to the above-described unsaturated bond occurs, and the appearance during use is deteriorated in some cases. Therefore, a lower limit value of the content of the carbonyl compound (1) is 2.0 ppm by mass, preferably 3.0 ppm by mass, more preferably 5.0 ppm by mass, and further more preferably 10 ppm by mass with respect to the total mass of the isocyanate composition. On the other hand, an upper limit value of the content of the carbonyl compound (I) is 1.0 × $10^4$ ppm by mass, preferably 3.0 × $10^3$ ppm by mass, and more preferably 1.0 × $10^3$ ppm by mass, with respect to the total mass of the isocyanate composition.

That is, the content of the carbonyl compound (I) is 2.0 ppm by mass or more and 1.0 × $10^4$ ppm by mass or less, preferably 3.0 ppm by mass or more and 3.0 × $10^3$ ppm by mass or less, more preferably 5.0 ppm by mass or more and 1.0 × $10^3$ ppm by mass or less, and further more preferably 10 ppm by mass or more and 1.0 × $10^3$ ppm by mass or less, with respect to the total mass of the isocyanate composition.

In a case where the content of the carbonyl compound (I) is equal to or more than the lower limit value, the denaturation reaction of the isocyanate compound can be suppressed. On the other hand, in a case where the content of the carbonyl compound (I) is equal to or less than the upper limit value, coloring caused by the unsaturated bond can be suppressed, and the appearance can be favorably maintained.

The content of the isocyanate compound is 97% by mass or more, preferably 98% by mass or more, and more preferably 99% by mass or more, with respect to the total mass of the isocyanate composition. By setting the content of the isocyanate compound to be equal to or more than the lower limit value, a composition sufficiently including the isocyanate compound as a target substance can be obtained. On the other hand, the upper limit is not particularly limited, but can be set to less than 100% by mass.

Hereinafter, each component of the isocyanate composition of the present embodiment will be described in detail.

<Carbonyl compound (I)>

The carbonyl compound (I) is a compound represented by General Formula (I).

[Chem.47]

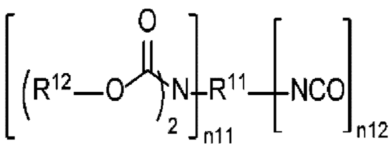

(I)

(In General Formula (I), $R^{11}$ is an (n11 + n12)-valent organic group, and $R^{12}$ is a monovalent organic group. n11 is an integer of 1 or more and 8 or less, n12 is an integer of 0 or more and 7 or less, and a sum of n11 and n12 is an integer of 2 or more and 8 or less).

[$R^{11}$ and $R^{12}$]

$R^{11}$ and $R^{12}$ are as described in «Carbonyl compound» described above.

[n11 and n12]

n11 is an integer of 1 or more and 8 or less.
n12 represents the number of isocyanate groups, and is an integer of 0 or more and 7 or less.

The sum (n11 + n12) of n11 and n12 is an integer of 2 or more and 8 or less, preferably an integer of 2 or more and 6 or less, more preferably an integer of 2 or more and 5 or less, and further more preferably an integer of 3 or more and 4 or less. In general, the larger the value of (n11 + n12), the more the crosslinking points (isocyanate groups) per carbonyl compound molecule increase, and the more the structures that contribute to coloring and prevention of denaturation of the isocyanate, the crosslinking density at the time of polymerization increases, the curing time can be shortened or the hardness of the polymer can be improved, and coloring and denaturation of the isocyanate can be suppressed. The expression "the crosslinking density increases" referred to herein means that an average molecular chain length between the crosslinking points decreases. On the other hand, in the production of a carbonyl compound, when an isocyanate group having high reactivity is heated, a denaturation reaction is conceived, and this becomes a cause of fixing to the device or clogging. Therefore, from the viewpoint of carbonyl compound synthesis, (n11 + n12) is preferably 6 or less, (n11 + n12) is more preferably 5 or less, and (n11 + n12) is further more preferably 4 or less.

Examples of the preferable carbonyl compound (1) include compounds represented by Formulae (I-1a) to (I-24b) (hereinafter, also referred to as "carbonyl compound (I-1a)" in some cases) and the like. In addition, carbonyl compounds (I-1a) to (I-1c), carbonyl compounds (I-2a) to (I-2c), carbonyl compounds (I-3a) to (I-3c), carbonyl compounds (I-4a) to (I-4c), carbonyl compounds (I-5a) to (I-5c), carbonyl compounds (I-6a) to (I-6c), carbonyl compounds (I-7a) to (I-7b), carbonyl compounds (I-8a) to (I-8b), carbonyl compounds (I-9a) to (I-9b), carbonyl compounds (I-10a) to (I-10b), carbonyl compounds (I-11a) to (I-11b), carbonyl compounds (I-12a) to (I-12b), carbonyl compounds (I-22a) to (1-22b), or carbonyl compounds (I-24a) to (I-24b) can be a mixture or single compounds.

In addition, the carbonyl compound (I) may be used alone or in combination of 2 or more thereof. In a case where 2 or more types of the carbonyl compounds (I) are used in combination, since the effects of improving the stability of the isocyanate compound for each carbonyl compound (I) are the same, the carbonyl compounds (I) can be mixed and used at any ratio.

[Chem. 48]

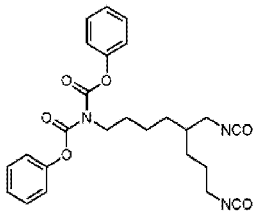

(I - 1a)

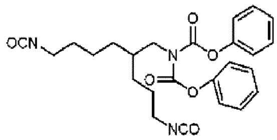

(I - 1b)

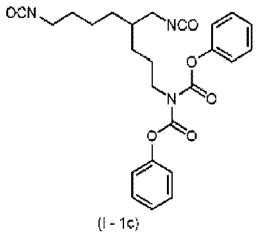

(I - 1c)

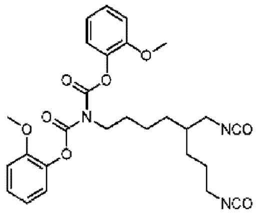

(I - 2a)

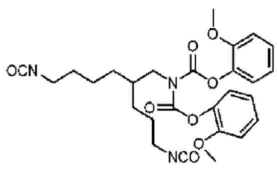

(I - 2b)

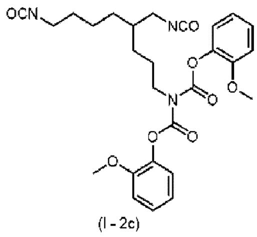

(I - 2c)

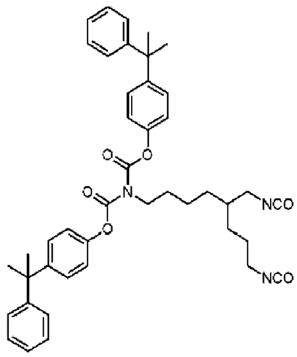

(I - 3a)

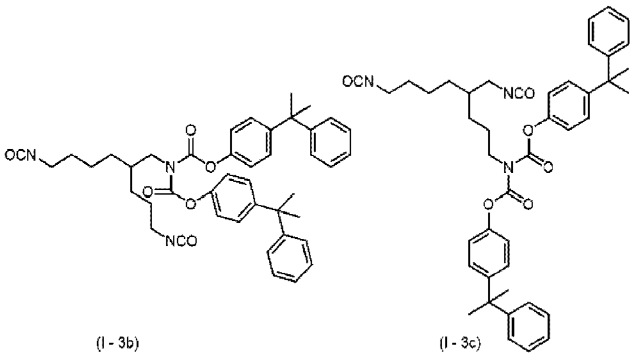

(I - 3b)

(I - 3c)

[Chem. 49]

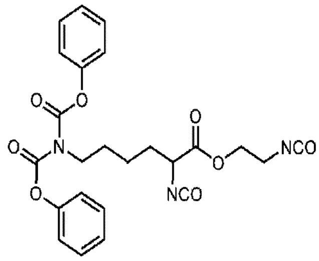

(I - 4a)

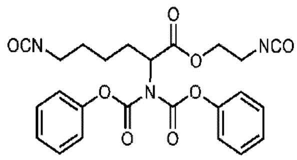

(I - 4b)

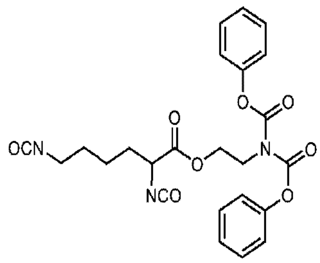

(I - 4c)

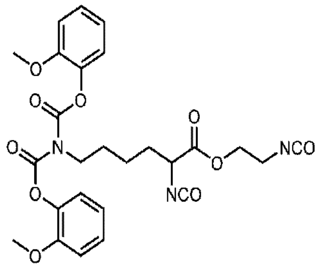

(I - 5a)

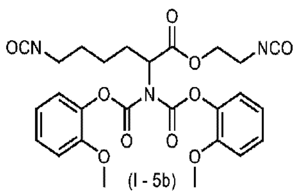

(I - 5b)

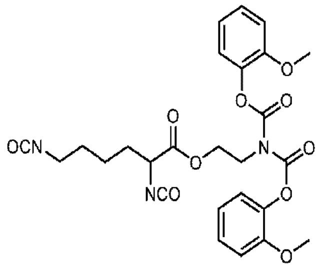

(I - 5c)

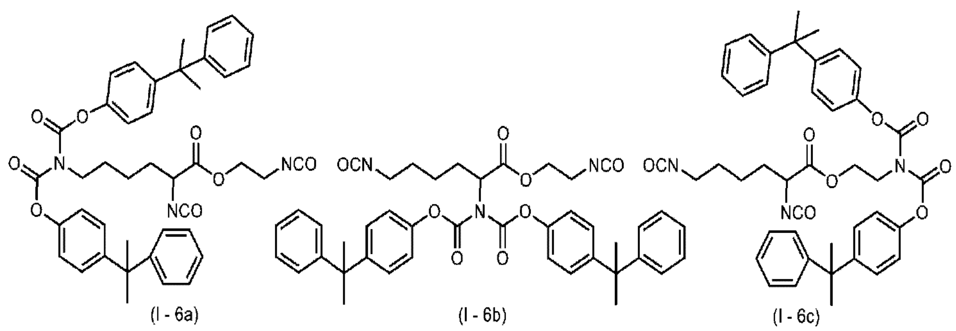

(I - 6a) (I - 6b) (I - 6c)

[Chem. 50]

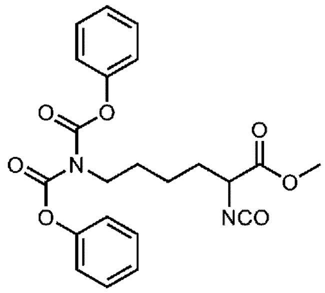

(I - 7a)

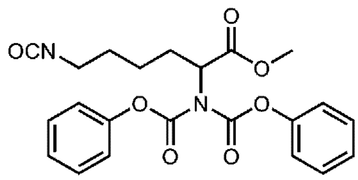

(I - 7b)

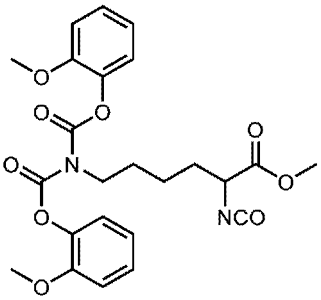

(I - 8a)

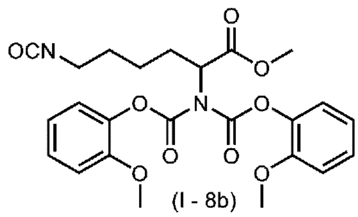

(I - 8b)

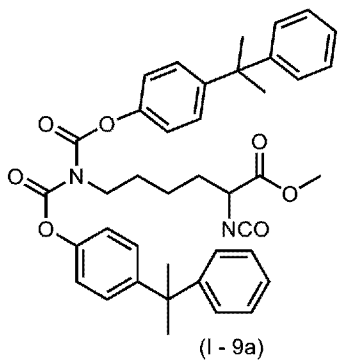

(I - 9a)

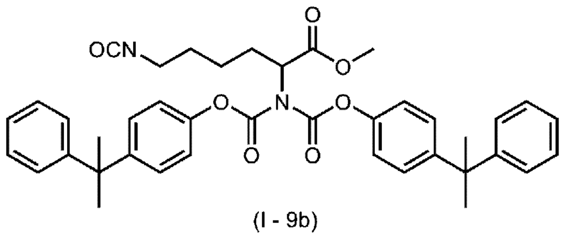

(I - 9b)

[Chem. 51]

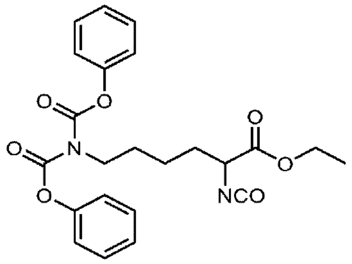

(I - 10a)

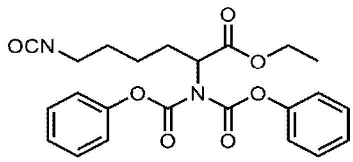

(I - 10b)

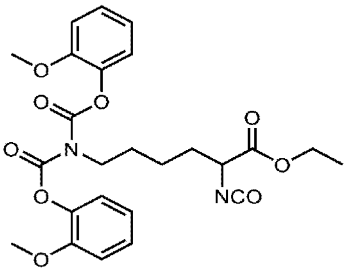

(I - 11a)

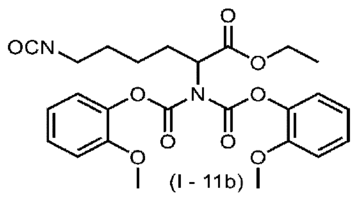

(I - 11b)

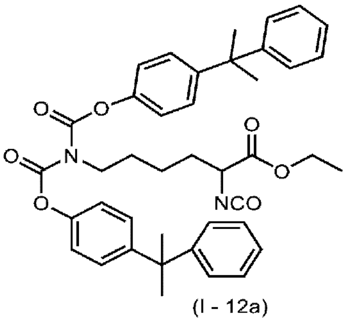

(I - 12a)

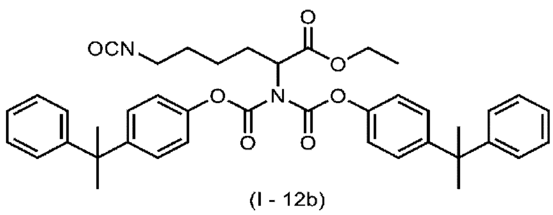

(I - 12b)

[Chem. 52]

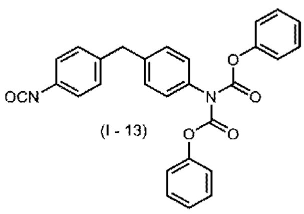

(I - 13)

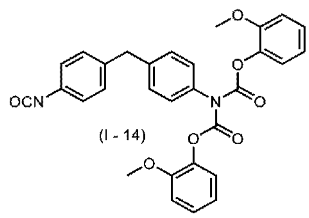

(I - 14)

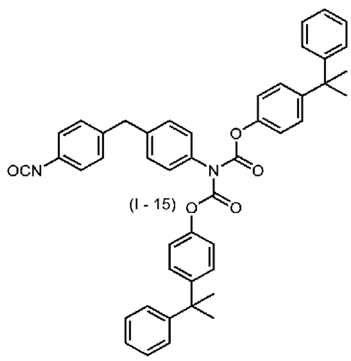

(I - 15)

[Chem. 53]

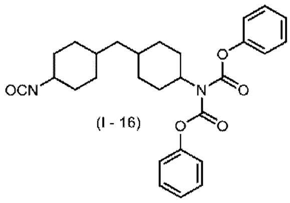
(I - 16)

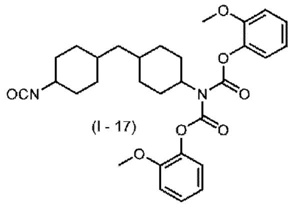
(I - 17)

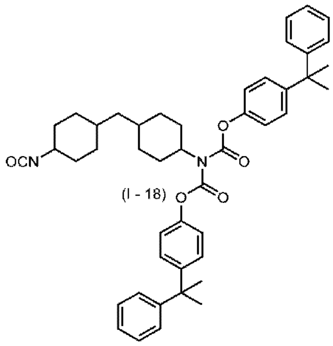
(I - 18)

[Chem. 54]

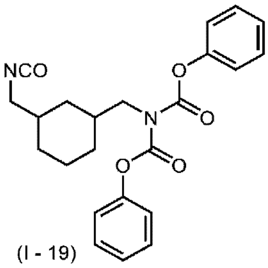
(I - 19)

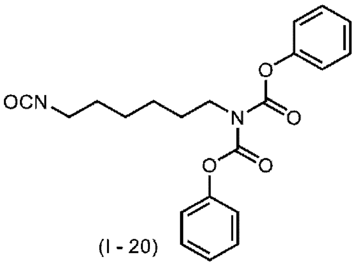
(I - 20)

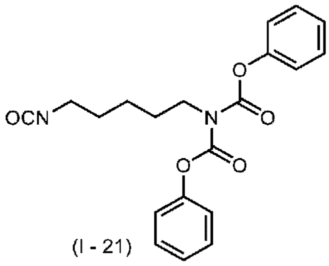
(I - 21)

[Chem. 55]

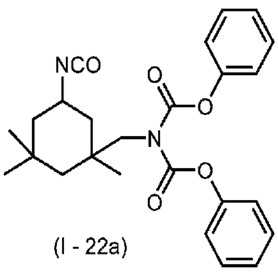
(I - 22a)

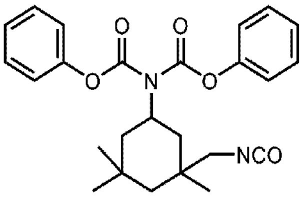
(I - 22b)

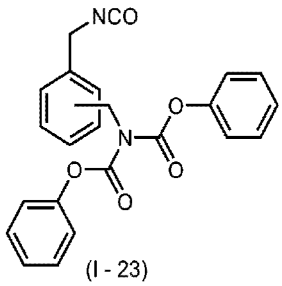
(I - 23)

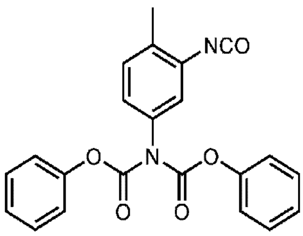
(I - 24a)

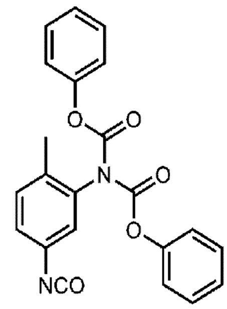
(I - 24b)

<Isocyanate compound>

As the isocyanate compound, a compound represented by General Formula (II) (hereinafter, referred to as "isocyanate compound (II)" in some cases) is preferably used.

[Chem.56]

$$R^{21}\left(-NCO\right)_{n21} \quad (II)$$

(In General Formula (II), $R^{21}$ is an n21-valent organic group, and satisfies a relational formula: $R^{21} = R^{11}$. n21 is an integer of 2 or more and 8 or less, and satisfies a relational formula: n21 = n11 + n12.)

[$R^{21}$, n21]

$R^{21}$ and n21 are as described in <Isocyanate compound> in <<Method for producing carbonyl compound>> described above.

Examples of the preferable isocyanate compound (II) include 4-isocyanatomethyl-1,8-octamethylene diisocyanate (TTI), 2-isocyanatoethyl-2,6-diisocyanatohexanoate (LTI), lysine methyl ester diisocyanate (LDI), diisocyanatopentane (PDI), diisocyanatohexane (HDI), methylenebis(cyclohexylisocyanate) (HMDI), 1,3-bis(isocyanatomethyl)cyclohexane (HXD1), 3-isocyanatomethyl-3,5,5-trimethylcyclohexyl isocyanate (IPDI), diisocyanatoxylene (XDI), diisocyanatodiphenylmethane (MDI), diisocyanatotoluene (TDI), and the like.

The isocyanate composition of the present embodiment preferably further contains one or more compounds selected from the group consisting of a carbamate compound and a carbonate, each of which is 2.0 ppm by mass or more and $1.0 \times 10^4$ ppm by mass or less.

The carbamate compound and the carbonate also exhibit the same effect as that of the above-described carbonyl compound (I). The content of these compounds in the isocyanate composition is also preferably increased to suppress the denaturation reaction of the isocyanate compound, but it is preferable that the content thereof not be excessively large in consideration of suppression of coloring or the appearance during use. Therefore, the lower limit value of the content of each of the carbamate compound and the carbonate is preferably 2.0 ppm by mass, more preferably 3.0 ppm by mass, further more preferably 5.0 ppm by mass, and particularly preferably 10 ppm by mass, with respect to the total mass of the isocyanate composition. On the other hand, the upper limit value of the content of each of the carbamate compound and the carbonate is preferably $1.0 \times 10^4$ ppm by mass, more preferably $3.0 \times 10^3$ ppm by mass, and further more preferably $1.0 \times 10^3$ ppm by mass, with respect to the total mass of the isocyanate composition.

That is, the content of each of the carbamate compound and the carbonate is preferably 2.0 ppm by mass or more and $1.0 \times 10^4$ ppm by mass or less, more preferably 3.0 ppm by mass or more and $3.0 \times 10^3$ mass ppm or less, further more preferably 5.0 ppm by mass or more and $1.0 \times 10^3$ ppm by mass or less, and particularly preferably 10 ppm by mass or more and $1.0 \times 10^3$ ppm by mass or less, with respect to the total mass of the isocyanate composition.

In a case where the content of each of the carbamate compound and the carbonate is equal to or more than the lower limit value, the denaturation reaction of the isocyanate compound can be suppressed. On the other hand, in a case where the content of the carbamate compound and the carbonate is equal to or less than the upper limit value, coloring caused by the unsaturated bond can be suppressed, and the appearance can be maintained favorably.

In a case where the isocyanate composition of the present embodiment contains all of the carbonyl compound (I), the carbamate compound, and the carbonate, the lower limit value of the total content of these compounds is preferably 2.0 ppm by mass, more preferably 3.0 ppm by mass, further more preferably 5.0 ppm by mass, and particularly preferably 10 ppm by mass, with respect to the total mass of the isocyanate composition. On the other hand, the upper limit value of the total content of these compounds is preferably $1.0 \times 10^5$ ppm by mass, more preferably $1.0 \times 10^4$ ppm by mass, further more preferably $3.0 \times 10^3$ ppm by mass, and particularly preferably $1.0 \times 10^3$ ppm by mass, with respect to the total mass of the isocyanate composition.

That is, the total content of the carbonyl compound (I), the carbamate compound, and the carbonate is preferably 2.0 ppm by mass or more and $1.0 \times 10^5$ ppm by mass or less, more preferably 3.0 ppm by mass or more and $1.0 \times 10^4$ ppm by mass or less, further more preferably 5.0 ppm by mass or more and $3.0 \times 10^3$ ppm by mass or less, and particularly preferably 10 ppm by mass or more and $1.0 \times 10^3$ ppm by mass or less, with respect to the total mass of the isocyanate composition.

In a case where the total content of these compounds is equal to or more than the lower limit value, the denaturation reaction of the isocyanate compound can be further suppressed. On the other hand, in a case where the total content of these compounds is equal to or less than the upper limit value, coloring caused by the unsaturated bond can be further suppressed, and the appearance can be favorably maintained.

<Carbamate compound>

As the carbamate compound, a compound represented by General Formula (III) (hereinafter, referred to as

"carbamate compound (III)" in some cases) is preferably used.

[Chem. 57]

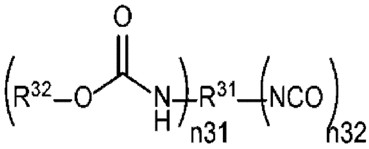

(III)

(In General Formula (III), $R^{31}$ is an (n31 + n32)-valent organic group, and satisfies a relational formula: $R^{31}$ = $R^{11}$. $R^{32}$ is a monovalent organic group, and satisfies a relational formula: $R^{32}$ = $R^{12}$. n31 is an integer of 1 or more and 8 or less, n32 is an integer of 0 or more and 7 or less, and a sum of n31 and n32 is an integer of 2 or more and 8 or less, and satisfies a relational formula: n31 + n32 = n11 + n12.)

[$R^{31}$, $R^{31}$, n31, n32]

$R^{31}$, $R^{31}$, n31, and n32 are as described in [Carbamate compound (III)] in <<Method for producing carbonyl compound>> described above.

Examples of the preferable carbamate compound (III) include compounds represented by Formulae (III-1a) to (III-24b). In addition, carbamate compounds (III-1a) to (III- 1c), carbamate compounds (III-2a) to (III-2c), carbamate compounds (III-3a) to (III-3c), carbamate compounds (III-4a) to (III-4c), carbamate compounds (III-5a) to (III-5c), carbamate compounds (III-6a) to (III-6c), carbamate compounds (III-7a) to (III-7b), carbamate compounds (III-8a) to (III-8b), carbamate compounds (III-9a) to (III-9b), carbamate compounds (111-lOa) to (111-lOb), carbamate compounds (111-11a) to (III-11b), carbamate compounds (III-12a) to (III-12b), carbamate compounds (III-22a) to (III-22b), or carbamate compounds (III-24a) to (III-24b) can be a mixture, or single compounds.

[Chem. 58]

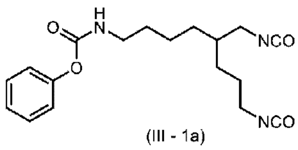

(III - 1a)

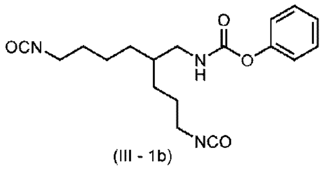

(III - 1b)

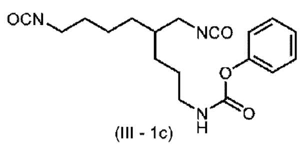

(III - 1c)

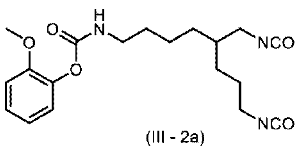

(III - 2a)

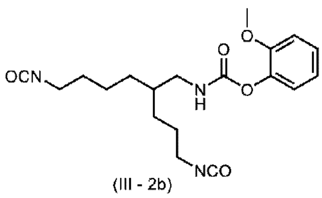

(III - 2b)

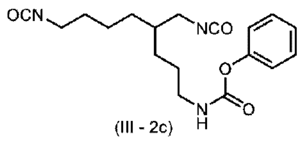

(III - 2c)

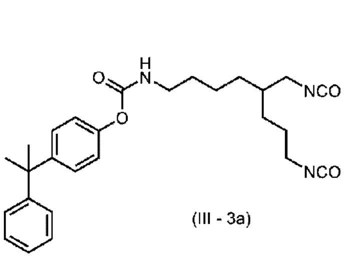

(III - 3a)

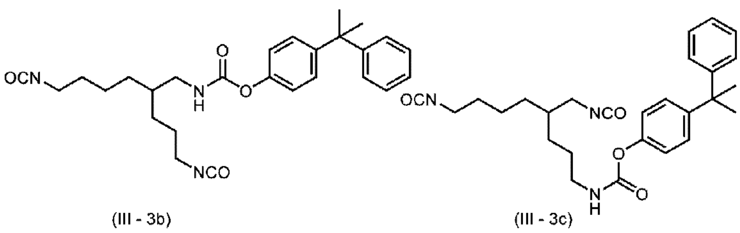

(III - 3b)

(III - 3c)

[Chem. 59]

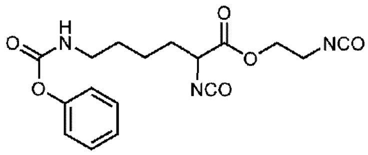
(III - 4a)

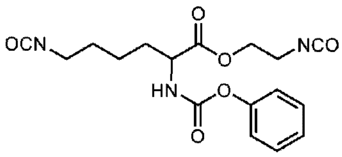
(III - 4b)

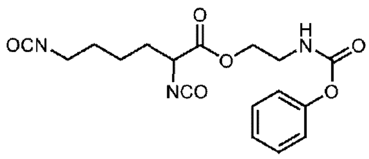
(III - 4c)

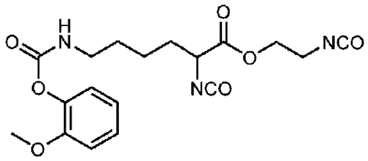
(III - 5a)

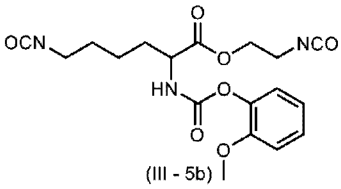
(III - 5b)

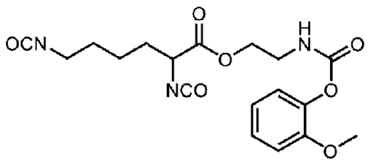
(III - 5c)

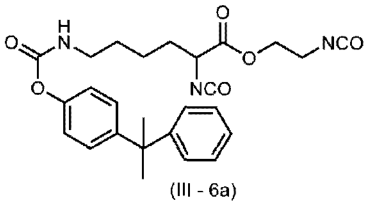
(III - 6a)

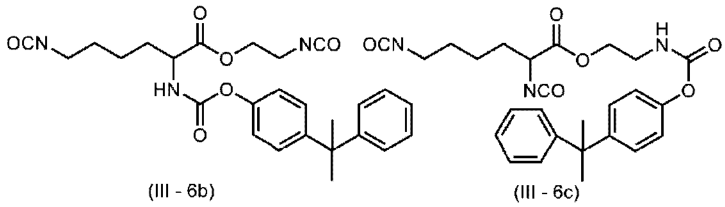
(III - 6b)

(III - 6c)

[Chem. 60]

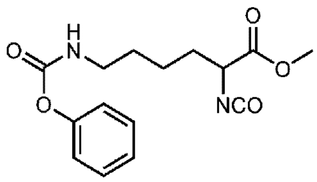
(III - 7a)

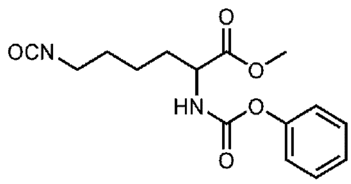
(III- 7b)

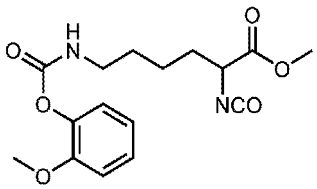
(III - 8a)

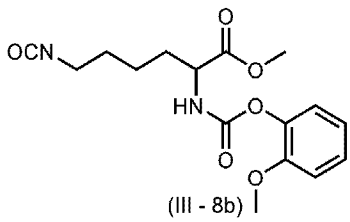
(III - 8b)

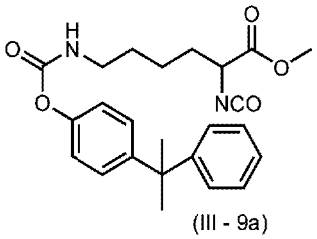
(III - 9a)

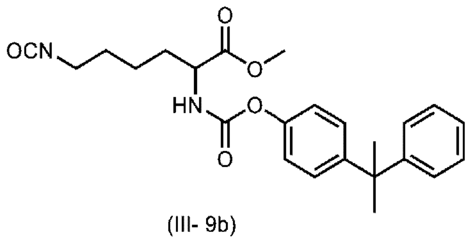
(III- 9b)

[Chem. 61]

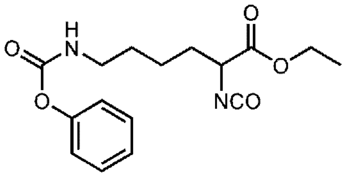

(III - 10a)

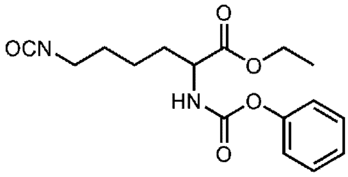

(III - 10b)

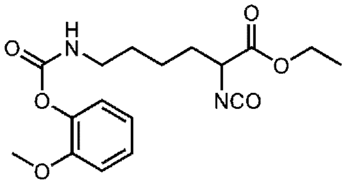

(III - 11a)

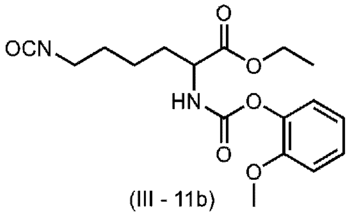

(III - 11b)

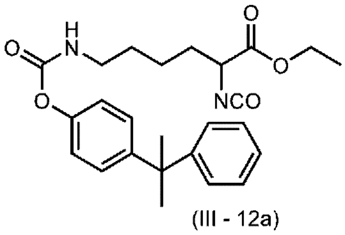

(III - 12a)

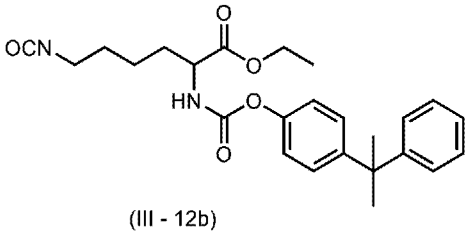

(III - 12b)

[Chem. 62]

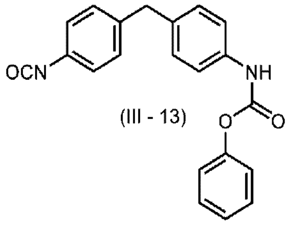

(III - 13)

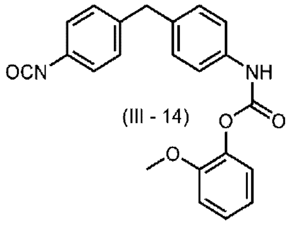

(III - 14)

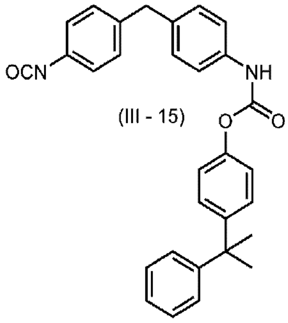

(III - 15)

[Chem. 63]

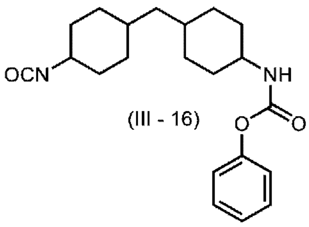

(III - 16)

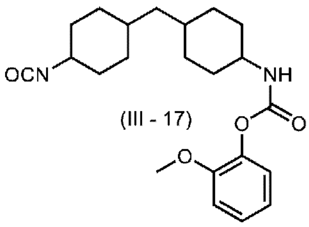

(III - 17)

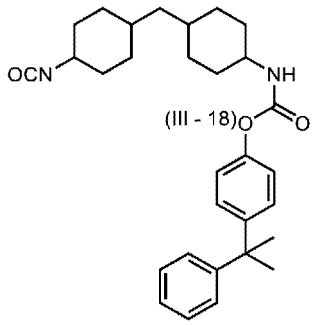

(III - 18)

[Chem. 64]

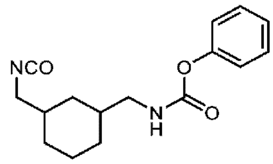
(III - 19)

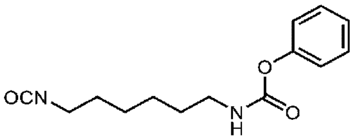
(III - 20)

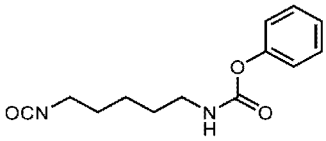
(III - 21)

[Chem. 65]

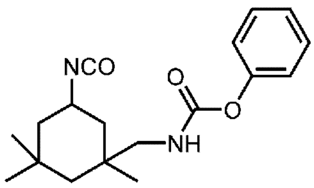
(III - 22a)

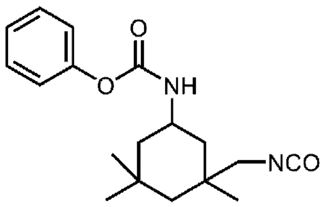
(III - 22b)

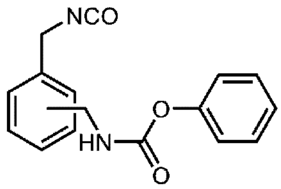
(III - 23)

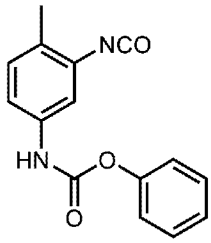
(III - 24a)

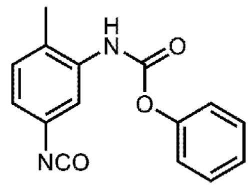
(III - 24b)

In addition, the carbamate compound (III) may be used alone or in combination of 2 or more thereof. In a case where 2 or more types of the carbamate compounds (III) are used in combination, since the effects of improving the stability of the isocyanate compound for each carbamate compound (III) are the same, the carbamate compounds (III) can be mixed and used at any ratio.

<Carbonate>

As the carbonate, a compound represented by General Formula (IV) (hereinafter, referred to as "carbonate (IV)" in some cases) is preferably used.

[Chem. 66]

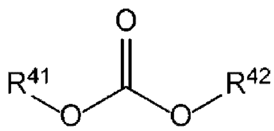
(IV)

(In General Formula (IV), $R^{41}$ and $R^{42}$ are each independently a monovalent organic group, and satisfy a relational formula: $R^{41} = R^{42} = R^{12}$.)

($R^{41}$ and $R^{42}$)

$R^{41}$ and $R^{42}$ are as described in <Carbonate> in <<Method for producing carbonyl compound>> described above.

Examples of the preferred carbonate (IV) include diphenyl carbonate, bis(2-methoxyphenyl)carbonate, bis(2-ethoxyphenyl)carbonate, and the like.

<Method for producing isocyanate composition>

The isocyanate composition of the present embodiment can be produced by mixing an isocyanate composition, a carbonyl compound (I), and one or more compounds selected from the group consisting of a carbamate compound and carbonate, depending on the necessity, to a set content.

Alternatively, a composition including an isocyanate compound (II) obtained by pyrolysis of a carbamate compound represented by General Formula (VI) (hereinafter, referred to as "carbamate compound (VI)" in some cases) can be used as the isocyanate composition of the present embodiment.

[Chem. 67]

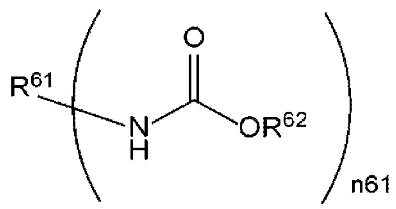

(VI)

In General Formula (VI), $R^{61}$ is an n61-valent (di- or higher valent and octa- or lower valent) organic group, and satisfies a relational formula: $R^{61} = R^{21}$. That is, $R^{61}$ is the same as $R^{21}$.

In General Formula (VI), $R^{62}$ is a monovalent organic group, and satisfies a relational formula: $R^{62} = R^{12}$. That is, $R^{62}$ is the same as $R^{12}$.

n61 represents the number of carbamate groups, is an integer of 2 or more and 8 or less, and satisfies a relational formula: n61 = n21. That is, n61 is the same as n21.

Specifically, a composition containing the isocyanate compound (II) is obtained by pyrolysis of a carbamate compound (VI) in the presence of the carbonate (IV) as a solvent.

At this time, since a hydroxy compound is generated as a by-product, it is preferable to proceed with the pyrolysis reaction while extracting and separating the hydroxy compound.

In addition, the pyrolysis reaction is preferably carried out by a continuous method. The continuous method is a method of continuously supplying a reaction solution containing the carbamate compound (VI) to a reactor to carry out a pyrolysis reaction of the carbamate compound (VI), and continuously extracting a hydroxy compound produced as a by-product from the reactor.

A pyrolysis temperature varies depending on the type of carbamate compound (VI) to be used, but can be, for example, 140°C or higher and 380°C or lower.

In addition, a reaction pressure varies depending on the type of compound to be used or the reaction temperature, but may be any of reduced pressure, normal pressure, and pressurized pressure. The pressure can be set to a pressure at which a saturated vapor pressure of an aprotic solvent to be used is obtained, and the pressure is preferably in a range of 20 Pa or more and $10 \times 10^6$ Pa or less.

A reaction time (retention time in the case of a continuous method) is not particularly limited, and can be set to 0.001 hours or more and 100 hours or less.

The carbonyl compound (I) is presumed to be a reaction product of the isocyanate compound (II) and the carbonate (IV), and is considered to be generated under pyrolysis reaction conditions. As a result, the isocyanate composition obtained in the pyrolysis reaction of the carbamate compound (VI) contains a specific amount of the carbonyl compound (1).

In the pyrolysis reaction, the carbamate compound (III) can also be said to be a reaction intermediate before becoming the isocyanate compound (II) which is a final product, generated from the carbamate compound (VI). As the reaction is stopped at a time point at which the content of the isocyanate compound (11) reaches a specific amount or more, the isocyanate composition including the carbamate compound (III) which is the reaction intermediate is obtained.

The isocyanate composition obtained in the pyrolysis reaction of the carbamate compound (VI) contains the carbonate (IV) as a solvent, but, in a case where the amount thereof is large, the carbonate (IV) can be separated from the isocyanate composition such that the content of the carbonate (IV) in the isocyanate composition is within a specific range, using a known separation method such as distillation separation.

In addition, in a case where the isocyanate composition of the present embodiment is produced by mixing the isocyanate compound, the carbonyl compound (1), and one or more compounds selected from the group consisting of

a carbamate compound and carbonate, depending on the necessity, so as to reach the above-described content, each of the carbonyl compound, the carbamate compound, and the carbonate can be produced by the method shown below. In addition, for the isocyanate compound, a compound obtained by pyrolysis of the carbamate compound (VI) can be purified and used depending on the necessity.

[Method for producing carbonyl compound (I)]

The carbonyl compound (I) is obtained by heating a mixture of the isocyanate compound (II) and the carbonate (IV), a mixture of the carbamate compound (III) or the carbamate compound (VI) and the carbonate (IV), or a mixture of the isocyanate compound (II), the carbamate compound (111) or the carbamate compound (VI), the carbonate (IV), and the hydroxy compound (hereinafter, this mixture is referred to as "raw material mixture of carbonyl compound (I)" in some cases). Examples of the hydroxy compound referred to herein include the same one as the hydroxy compound produced as a by-product in the pyrolysis reaction of the carbamate compound (VI), and the details will be described later.

Regarding the use amount (molar amount) of the carbonate (IV), a use amount of the carbonate solvent is preferably large from the viewpoint of suppressing side reactions, but considering the size of the reactor and the like, the use amount is preferably 0.001 times or more and 100 times or less, more preferably 0.01 times or more and 80 times or less, and further more preferably 0.1 times or more and 50 times or less, in terms of the stoichiometric ratio with respect to a total molar amount of the carbamate compound (III), the carbamate compound (VI), and the isocyanate compound (II).

A reaction temperature is usually 100°C or higher and 400°C or lower, and a high temperature is preferable to increase the reaction rate. On the other hand, at a high temperature, there is a case where a side reaction is caused by at least any compound of the carbamate compound and the isocyanate compound, and thus the reaction temperature is preferably 130°C or higher and 300°C or lower, and more preferably 150°C or higher and 280°C or lower. To keep the reaction temperature constant, known cooling devices and heating devices may be installed in the reactor.

In addition, a reaction pressure varies depending on the type of compound to be used and the reaction temperature, but the reaction pressure may be any one of reduced pressure, normal pressure, or pressurized pressure, and is usually in a range of 20 Pa or more and $1 \times 10^6$ Pa or less.

A reaction time (retention time in the case of a continuous method) is not particularly limited, but is usually 0.001 hours or longer and 100 hours or shorter, preferably 0.01 hours or longer and 50 hours or shorter, and more preferably 0.1 hours or longer and 10 hours or shorter.

In the production of the carbonyl compound by mixing and heating, it is possible to increase the generation rate and generation amount of the carbonyl compound by bringing the raw material liquid into contact with stainless steel and heating the raw material liquid. As the stainless steel, any shape can be used as long as the stainless steel is made of SUS316 or SUS304, and for example, a filling material or a metal piece is preferably used. The filling material is not particularly limited, but DIXON Packing, Me MAHON Packing, Coil Pack, MESH RING, CANNON Packing, HELI PACK, RASCHIG RING, PRICKLE RING, or the like is used.

In a case where a volume of the raw material liquid of the carbonyl compound is denoted as V and a surface area of the stainless steel is denoted as A, the larger the contact area with stainless steel per unit volume of the raw material liquid, a value of the generation rate A/V of the carbonyl compound is preferably 0.001 $m^2/m^3$ or more and 100,000 $m^2/m^3$ or less, more preferably 0.01 $m^2/m^3$ or more and 50,000 $m^2/m^3$ or less, and further more preferably 0.1 $m^2/m^3$ or more and 10,000 $m^2/m^3$ or less.

A reaction form is not particularly limited, but a reactor in which the raw material mixture of the carbonyl compound (I), or the raw material mixture of the carbonyl compound (I) and stainless steel can be efficiently mixed and heated is preferable, and for example, a method of heating a raw material liquid in a stirring tank or a distillation column made of stainless steel is preferable.

The distillation method is not particularly limited as long as the light boiling component of the liquid obtained after heating the raw material mixture of the carbonyl compound (I) may be distilled off, and the light boiling component can be separated as a gas-phase component. The light boiling point component (light boiling component) refers to a component having a boiling point lower than that of the carbonyl compound (I), varies depending on the material to be used, and is mainly one or more compounds selected from the group consisting of the carbonate (IV), the isocyanate compound (II), and the hydroxy compound.

[Method for producing carbamate compound (III)]

There is a method of obtaining the carbamate compound (III) by mixing the isocyanate compound (II) and the hydroxy compound represented by Formula (V) (hereinafter, referred to "hydroxy compound (V)" in some times), and heating the mixture, and a method of obtaining the isocyanate compound (II) by pyrolysis of the carbamate compound represented by Formula (VI).

[Chem. 68]

$$R^{51}\text{-OH} \qquad (V)$$

In General Formula (V), $R^{51}$ is a monovalent organic group, and satisfies a relational formula: $R^{51} = R^{12}$. That is, $R^{51}$ is the same as $R^{12}$.

It is preferable that a mixing ratio in a case where the isocyanate compound (II) and the hydroxy compound (V) are mixed and heated to produce a mixture be such that a ratio NCO: OH of the isocyanate group of the isocyanate compound (II) to the hydroxyl group of the hydroxy compound (V) is (n31 + n32): n31.

A reaction temperature is usually 40°C or higher and 400°C or lower, and a high temperature is preferable to increase the reaction rate. On the other hand, at a high temperature, there is a case where a side reaction is caused by at least any compound of the carbamate compound and the isocyanate compound, and thus the reaction temperature is preferably 80°C or higher and 300°C or lower, and more preferably 100°C or higher and 250°C or lower. To keep the reaction temperature constant, known cooling devices and heating devices may be installed in the reactor.

In addition, a reaction pressure varies depending on the type of compound to be used and the reaction temperature, but the reaction pressure may be any one of reduced pressure, normal pressure, or pressurized pressure, and is usually in a range of 20 Pa or more and $1 \times 10^6$ Pa or less.

A reaction time (retention time in the case of a continuous method) is not particularly limited, but is usually 0.001 hours or longer and 100 hours or shorter, preferably 0.01 hours or longer and 50 hours or shorter, and more preferably 0.1 hours or longer and 10 hours or shorter.

In the case of producing by a pyrolysis reaction, the above-described method for producing the isocyanate compound (II) can be used.

[Method for producing carbonate (IV)]

The carbonate (IV) can be synthesized using the methods described in Japanese Patent No. 3071008 (Reference 1) and Japanese Patent No. 4137941 (Reference 2). Specifically, the carbonate (IV) can be produced by a method of reacting an aromatic monohydroxy compound with a chlorocarbonic ester of phosgene or an aromatic monohydroxy compound in the presence of activated carbon while detaching hydrogen chloride, or a method including step (1) of reacting an organometallic compound with carbon dioxide to obtain a reaction mixture containing a dialkyl carbonate formed in the reaction; step (2) of separating the dialkyl carbonate from the reaction mixture to obtain a residual solution; step (3) of reacting the residual solution with alcohol to form at least one organometallic compound and water, and removing the water from the organometallic compound; and step (4) of reacting the dialkyl carbonate separated in step (2) with an aromatic hydroxy compound to obtain an aromatic carbonate. The steps (3) and (4) can be carried out in the same order or in reverse order, or partially or wholly simultaneously.

<Usage>

The isocyanate composition of the present embodiment sufficiently suppresses coloring and has excellent storage stability, and thus is suitably used as a curing agent raw material in the fields where appearance quality is required, such as a baking coating material, automobile clear coating material, and coil coating material.

[Examples]

Hereinafter, the present invention will be described in detail based on examples, but the scope of the present invention is not limited to the examples. Hereinafter, "%" means "% by mass" and "ppm" means "ppm by mass".

<Analysis method>

(1) $^1$H-NMR analysis method

$^1$H-NMR analysis was implemented using JNM-A400 FT-NMR system manufactured by JEOL Ltd. as a device.

(1-1) Preparation of ’H-NMR analysis sample

0.3 g of a sample solution was weighed, 0.7 g of deuterochloroform and 0.05 g of dimethyldiphenylsilane as

an internal standard substance were added thereto, and uniformly mixed to obtain a solution as an NMR analysis sample.

(1-2) Quantitative analysis method

Analysis was implemented on each standard substance, and quantitative analysis of the analysis sample solution was implemented based on the created calibration curve.

(2) Gas chromatography analysis method

Analysis was carried out under the following conditions.

(Measurement conditions)

Device: GC-2010, manufactured by Shimadzu Corporation
Column: DB-1
Diameter 0.25 mm, length 30 m, film thickness 1.0 μm
Column temperature: 60°C to 300°C
Inlet temperature: 300°C
Carrier gas: helium
Carrier gas flow rate: 40 mL/min
Detector: FID (hydrogen flame ionization detector)

(2-1) Preparation of gas chromatography analysis sample

1.0 g of the sample solution was weighed, and 10 g of acetonitrile and 0.1 g of anisole as an internal standard substance were added thereto and uniformly mixed to obtain a solution as a gas chromatography analysis sample.

(3) Liquid chromatography analysis method

Analysis was carried out under the following conditions.

(Measurement conditions)

Device: LC-10AT manufactured by Shimadzu Corporation
Column: Inertsil ODS
Particle diameter 5 μm, inner diameter 2.1 mm, length 250 mm
Column temperature: 40°C
Developing solvent: water/acetonitrile = 90/10
Developing solvent flow rate: 1 mL/min
Detector: photodiode array detector

(3-1) Preparation of liquid chromatography analysis sample

1.0 g of the sample solution was weighed, 10 g of acetic acid was added thereto, and uniformly mixed to obtain a solution as a liquid chromatography analysis sample.

(3-2) Quantitative analysis method

Analysis was implemented on each standard substance, and quantitative analysis of the analysis sample solution was implemented based on the created calibration curve.

(4) Purification method using column fractionation device

The generated carbonyl compound was isolated under the following conditions.

(Conditions)

Device: EPCLC-AI-580S, manufactured by Yamazen Corporation
Elution position-controlled purification chromatograph
Injection column: M or L
Main column: High-flash S, M, or L
Developing solvent: ethyl acetate/hexane
Developing solvent flow rate: 0 mL/min to 80 mL/min
Detector: UV detector

From measured values of $^1$H-NMR, GC, and LC, a yield of the generated isocyanate compound and a value of { 3 × (molar amount of isocyanurate group) + 2 × (molar amount of carbodiimide group) + 3 × (molar amount of uretonimine group) + 2 × (molar amount of allophanate group)}/(molar amount of carbonyl compound) was calculated. In addition, the carbonyl compound was quantified from the measured value of LC.

<Evaluation method>

[Storage test]

100 g of each isocyanate composition was placed in a screwtop bottle having a capacity of 200 mL, and kept and stored at 25°C for 300 days in a nitrogen atmosphere.

[Evaluation 1]

(Hazen color number)

The Hazen color number before and after the storage was measured with a Hazen meter.

[Evaluation 2]

100 g of each isocyanate composition after storage was pressure-filtered using a membrane filter having a pore size of 1 μm, a filtration residual mass was obtained from a filter mass before and after filtration, and a denaturation amount was calculated by the equation shown below.

$$(\text{Denaturation amount (\% by mass)}) = \{(\text{filter mass after filtration}) - (\text{filter mass before filtration})\} \times 100/100\ \text{g}$$

[Example 1-1]

Step (1-1): Production step of carbamate compound

A reaction was carried out using the device shown in FIG. 1. The device shown in FIG. 1 was also used in the production of carbamate compounds in Example 1-1 and the subsequent examples.

In a state in which a line 14 was closed, 3.33 kg (19.2 mol) of 4-aminomethyl-1,8-octanediamine was supplied from a storage tank 101 through a line 11 to a reaction container 104 made of SUS with a baffle, 5.50 kg (58.5 mol) of phenol was supplied from a storage tank 102 through a line 12 to the reaction container 104, and stirring was carried out for homogenization. Subsequently, in a state in which a line 16 was closed, 5.50 kg (58.5 mol) of phenol was supplied from the storage tank 102 through a line 15 to a reactor 105 made of SUS with a baffle, and 20.52 kg (95.9 mol) of diphenyl carbonate was supplied from a storage tank 103 through a line 13 to a reaction container 105. A liquid temperature of the reaction container 105 was adjusted to 65°C, stirring was carried out for homogenization, and then the mixture solution of 4-aminomethyl-1,8-octanediamine and phenol was supplied from the reaction container 104 through the line 14 so that the internal temperature thereof did not exceed 70°C. After continuing stirring for 2 hours, as a temperature of the reaction solution was raised to 120°C, and the internal pressure was set to about 1 kPa, 15.53 kg of phenol in the solution was extracted to a storage tank 107 through a line 17 and a capacitor (condensing device) A11.

As a result of analyzing the solution after the reaction (hereinafter, referred to as "reaction solution (1-1)") by liquid chromatography, a carbamate compound corresponding to 4-aminomethyl-1,8-octanediamine was generated at a yield of 99% by mass. The line 16 was opened, and the reaction solution (1-1) was transferred to a storage tank 106 through the line 16. A mass of the reaction solution (1 -1) was 19.40 kg.

Step (1-2): Pyrolysis step of carbamate compound

The reaction was carried out using the device shown in FIG. 2. The device shown in FIG. 2 was also used for the pyrolysis of the carbamate compounds in Example 1-1 and the subsequent examples.

In a state in which a line 24 was closed, 19.40 kg of diphenyl carbonate was supplied from a storage tank 202 through a line 22 to a reaction container 201 made of SUS with a baffle. A temperature of a multi-stage distillation column 203 was raised to 170°C, a jacket temperature of the reaction container 201 was heated to 228°C, and a pressure was reduced to 14 kPa. 19.40 kg of the reaction solution (1-1) recovered in the storage tank 106 in step (1-1) was heated to 120°C, and supplied to the reaction container 201 through a line 21 for about 15 minutes to carry out pyrolysis of the carbamate compound. The pressure was adjusted in a range of 8 to 14 kPa, and the phenol generated by pyrolysis was separated from diphenyl carbonate or 1,8-diisocyanato-4-isocyanatomethyloctane (TTI) as the product in the distillation column 203, and was recovered in a storage tank 204 through a line 25, a capacitor A21 and a line 27. A reflux ratio at this time was 1.2. After the entire reaction solution (1-1) was transferred, phenol was continuously extracted at an internal temperature of 220°C. The reaction was ended 4 hours after the transfer of the entire reaction solution (1-1), and the reaction solution was extracted from a line 28 and transferred to a storage tank 205. A mass of the reaction solution transferred to the storage tank 205 was 18.63 kg. A part of the reaction solution (hereinafter, referred to as "reaction solution (1-2)") was purified with a column fractionation device to isolate the carbonyl compound. The isolated carbonyl compound was a mixture of compounds represented by Formulae (I-1a) to (1-1c). In addition, FIG. 5 shows a $^1$H-NMR spectrum of the carbonyl compound.

[Chem. 69]

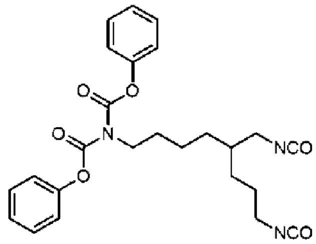

(I - 1a)

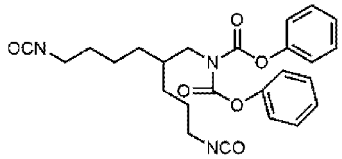

(I - 1b)

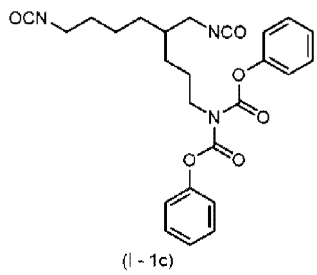

(I - 1c)

A calibration curve of LC was created from the obtained carbonyl compound, and the carbonyl compound was quantified by an absolute calibration curve method. In the subsequent examples, the operation of isolating the carbonyl compound was not carried out, and the carbonyl compound was quantified from the calibration curve.

In addition, as a result of carrying out $^1$H-NMR and gas chromatography analysis, TTI was generated with a yield of 70% by mass, and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 3.7.

Step (1-3): Light boiling separation step

The reaction was carried out using the device shown in FIG. 3. The device shown in FIG. 3 was also used for the light boiling separation after Example 1-1.

The reaction solution (1-2) was continuously fed to a middle stage of a continuous multi-stage distillation column 301 from the storage tank 205 through a line 31 at 3.73 kg/hour, and distillation separation of the liquid-phase component was carried out. A heat amount required for distillation separation was supplied by circulating a column lower liquid through a reboiler A32 and a line 33. A liquid temperature at a column bottom portion of the continuous multi-stage distillation column was 220°C, and a pressure at the top of the column was 1.5 kPa. The gas distilled from the top of the continuous multi-stage distillation column 301 was condensed in a capacitor (condensing device) A31 through a line 32, and continuously extracted to a storage tank 302 through a line 36. In addition, an extraction rate of a line 34 in a steady

state was 1.49 kg/hour, and continuously extracted to a storage tank 303. The amount of the liquid recovered in the storage tank 303 (hereinafter, referred to as "reaction solution (1-3)") was 7.45 kg, as a result of analysis by NMR, LC, and gas chromatography, TTI was recovered with respect to the supplied reaction solution (1-2) at a yield of 82% by mass, and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 4.6.

Step (1-4): high boiling separation step

The reaction was carried out using the device shown in FIG. 4. The device shown in FIG. 4 was also used for the high boiling separation in Example 1-1 and the subsequent examples.

A thin-film distillation device 401 (manufactured by Kobelco Eco-Solutions Co., Ltd., Japan) was heated to 190°C, and the internal pressure was set to 0.3 kPa. The reaction solution (1-3) recovered in the storage tank 303 in step (1-3) was supplied to an upper portion of the thin-film distillation device 401 at about 1.0 kg/hour through a line 41 to carry out separation of the isocyanate and a high boiling component. The generated gas-phase component was transferred to a storage tank 402 through a line 42 and a capacitor (condensing device) A41. An amount of the liquid recovered from the storage tank 402 was 3.35 kg, and a TTI recovery rate was 121% by mass. The reason why the TTI recovery rate exceeds 100% by mass is that a part of the isocyanate denaturation product generated in the pyrolysis step or the light boiling separation step is regenerated to TTI.

[Example 1-2]

Step (2-2): Pyrolysis step of carbamate compound

A pyrolysis reaction was carried out by the same operation as that of Example 1-1, except that 19.4 kg of the reaction solution (1-1) of Example 1-1 and 19.4 kg of diphenyl carbonate were used, the reaction solution (1-1) was supplied to the reactor over about 13 minutes to start a reaction, the reaction was carried out at a jacket temperature of 248°C, an internal temperature of 240°C, a reflux ratio of 0.4, and a pressure in a range of 14 to 26 kPa, and extraction of phenol was continued for 3 hours after the transfer of the entire reaction solution (1-1). A mass of the reaction solution transferred to the storage tank 205 was 20.96 kg. As a result of analysis of the reaction solution (hereinafter, referred to as "reaction solution (2-2)") by NMR, LC, and gas chromatography, TTI was generated at a yield of 78% by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 4.3.

Step (2-3): Light boiling separation step

Light boiling separation was carried out by the same operation as that of Example 1-1, except that the reaction solution (2-2) was continuously fed at 4.19 kg/hour, and an extraction rate of the line 34 in a steady state was 1.26 kg/hour. The amount of the liquid recovered in the storage tank 303 was 6.29 kg, and as a result of analysis by NMR, LC, and gas chromatography, TTI was recovered at a yield of 77% by mass with respect to the supplied reaction solution (2-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 6.3.

Step (2-4): High boiling separation step

High boiling separation was carried out by the same operation as that of Example 1-1, except that the liquid recovered in the storage tank 303 in step (2-3) was used. The amount of the liquid recovered in a storage tank 402 was 3.36 kg, and a TTI recovery rate was 116% by mass.

[Example 1-3]

Step (3-2): Pyrolysis step of carbamate compound

A pyrolysis reaction was carried out by the same operation as that of Example 1-1, except that 19.4 kg of the reaction solution (1-1) of Example 1-1 and 19.4 kg of diphenyl carbonate were used, the reaction solution (1-1) was supplied to the reactor over about 9 minutes to start the reaction, the reaction was carried out at a jacket temperature

of 258°C, an internal temperature of 250°C, a reflux ratio of 2.0, and a pressure in a range of 21 to 32 kPa, and extraction of phenol was continued for 2.5 hours after the transfer of the entire reaction solution (1-1). A mass of the reaction solution transferred to the storage tank 205 was 22.12 kg. As a result of analysis of the reaction solution (hereinafter, referred to as "reaction solution (3-2)") by NMR, LC, and gas chromatography, TTI was generated at a yield of 73% by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 3.9.

Step (3-3): Light boiling separation step

Light boiling separation was carried out by the same operation as that of Example 1-1, except that the reaction solution (3-2) was continuously fed at 4.42 kg/hour, and an extraction rate of the line 34 in a steady state was 1.42 kg/hour. The amount of the liquid recovered in the storage tank 303 was 7.08 kg, and as a result of analysis by NMR, LC, and gas chromatography, TTI was recovered at a yield of 80% by mass with respect to the supplied reaction solution (3-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 5.5.

Step (3-4): High boiling separation step

High boiling separation was carried out by the same operation as that of Example 1-1, except that the liquid recovered in the storage tank 303 in step (3-3) was used. The amount of the liquid recovered in the storage tank 402 was 3.35 kg, and the TTI recovery rate was 119% by mass.

[Example 1-4]

Step (4-2): Pyrolysis step of carbamate compound

A pyrolysis reaction was carried out by the same operation as that of Example 1-1, except that 19.4 kg of the reaction solution (1-1) of Example 1-1 and 19.4 kg of diphenyl carbonate were used, the reaction solution (1-1) was supplied to the reactor over about 14 minutes to start the reaction, the reaction was carried out at a jacket temperature of 238°C, an internal temperature of 230°C, a reflux ratio of 1.5, and a pressure in a range of 8 to 14 kPa, and extraction of phenol was continued for 3 hours after the transfer of the entire reaction solution (1-1). A weight of the reaction solution transferred to a storage tank 205 was 22.12 kg. As a result of analysis of the reaction solution (hereinafter, referred to as "reaction solution (4-2)") by NMR, LC, and gas chromatography, TTI was generated at a yield of 79% by mass. A value of {3 × (molar amount of isocyanurate group) + 2 × (molar amount of carbodiimide group) + 3 × (molar amount of uretonimine group) + 2 × (molar amount of allophanate group) } /(molar amount of carbonyl compound) of the reaction solution at this time was 1.6.

Step (4-3): Light boiling separation step

Light boiling separation was carried out by the same operation as that of Example 1-1, except that the reaction solution (4-2) was continuously fed at 4.42 kg/hour and the extraction rate of the line 34 in a steady state was 1.68 kg/hour. The amount of the liquid recovered in the storage tank 303 was 8.41 kg, and, as a result of analysis by NMR, LC, and gas chromatography, TTI was recovered at a yield of 85% by mass with respect to the supplied reaction solution (4-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 2.6.

Step (4-4): High boiling separation step

High boiling separation was carried out by the same operation as that of Example 1-1, except that the liquid recovered in the storage tank 303 in step (4-3) was used. The amount of the liquid recovered in the storage tank 402 was 4.05 kg, and the TTI recovery rate was 125% by mass.

[Example 1-5]

Step (5-1): Production step of carbamate compound

Carbamate synthesis was carried out by the same operation as that of Example 1-1, except that 0.23 kg (69.2 mol) of urea was used instead of diphenyl carbonate, phenol supplied to the storage tank 102 was 25.87 kg (275.2 mol), and stirring was performed at a reaction temperature of a reactor 104 of 240°C for 30 minutes. The amount of phenol and ammonia extracted into a storage tank 107 was 24.14 kg.
As a result of analysis of the solution after the reaction (hereinafter, referred to as "reaction solution (5-1)") by liquid chromatography, the corresponding carbamate compound was generated at a yield of 99% by mass. The line 16 was opened, and the reaction solution (5-1) was transferred to the storage tank 106 through the line 16. A mass of the reaction solution (5-1) was 10.79 kg.

Step (5-2): Pyrolysis step of carbamate compound

A pyrolysis reaction was carried out by the same operation as that of Example 1 - 1, except that 10.8 kg of the reaction solution (5-1) of Example 1-1 and 28.0 kg of diphenyl carbonate were used, the reaction solution (5-1) was supplied to the reactor over about 13 minutes to start the reaction, the reaction was carried out at a jacket temperature of 238°C, an internal temperature of 230°C, a reflux ratio of 1.7, and a pressure in a range of 8 to 14 kPa, and extraction of phenol was continued for 3 hours after the transfer of the entire reaction solution (5-1). A mass of the reaction solution transferred to the storage tank 205 was 21.34 kg. As a result of analysis of the reaction solution (hereinafter, referred to as "reaction solution (5-2)") by NMR, LC, and gas chromatography, TTI was generated at a yield of 78% by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.5.

Step (5-3): light boiling separation step

Light boiling separation was carried out by the same operation as that of Example 1-1, except that the reaction solution (5-2) was continuously fed at 4.27 kg/hour and the extraction rate of the line 34 in a steady state was 1.75 kg/hour. The amount of the liquid recovered in the storage tank 303 was 8.75 kg, and as a result of analysis by NMR, LC, and gas chromatography, TTI was recovered at a yield of 84% by mass with respect to the supplied reaction solution (5-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 2.5.

Step (5-4): High boiling separation step

High boiling separation was carried out by the same operation as that of Example 1-1, except that the liquid recovered in the storage tank 303 in step (5-3) was used. The amount of the liquid recovered in the storage tank 402 was 4.05 kg, and the TTI recovery rate was 128% by mass.

[Example 1-6]

Step (6-1): Production step of carbamate compound

Carbamate synthesis was carried out by the same operation as that of Example 1-5, except that 12.40 kg (58.5 mol) of 4-cumylphenol instead of phenol was supplied to the reactor 104 made of SUS with a baffle, and 18.97 kg (89.47 mol) of 4-cumylphenol instead of phenol was supplied to the reactor 105 made of SUS with a baffle. The amount of 4-cumylphenol and ammonia extracted into the storage tank 107 was 16.97 kg.
As a result of analysis of the solution after the reaction (hereinafter, referred to as "reaction solution (6-1)" by liquid chromatography, the corresponding carbamate compound was generated at a yield of 99% by mass. The line 16 was opened, and the reaction solution (6-1) was transferred to the storage tank 106 through the line 16. A mass of the reaction solution (6-1) was 17.96 kg.

Step (6-2): Pyrolysis step of carbamate compound

A pyrolysis reaction was carried out by the same operation as that of Example 1-5, except that 18.0 kg of the

reaction solution (6-1) and 28.0 kg of bis(4-cumylphenyl)carbonate were used, the reaction solution (6-1) was supplied to the reactor over about 12 minutes to start the reaction, the reaction was carried out at a jacket temperature of 238°C, an internal temperature of 230°C, a reflux ratio of 1.5, and a pressure in a range of 3 to 5 kPa, and extraction of 4-cumylphenol was continued for 3 hours after the transfer of the entire reaction solution (6-1). A mass of the reaction solution transferred to the storage tank 205 was 32.19 kg. As a result of analysis of the reaction solution (hereinafter, referred to as "reaction solution (6-2)") by NMR, LC, and gas chromatography, TTI was generated at a yield of 75% by mass. A value of {3 × (molar amount of isocyanurate group) + 2 × (molar amount of carbodiimide group) + 3 × (molar amount of uretonimine group) + 2 × (molar amount of allophanate group)}/(molar amount of carbonyl compound) of the reaction solution at this time was 1.4. A carbonyl compound was isolated by purifying a part of the reaction solution (6-2) with a column fractionation device. The isolated carbonyl compound was a mixture of compounds represented by Formulae (I-3a) to (I-3c).

[Chem. 70]

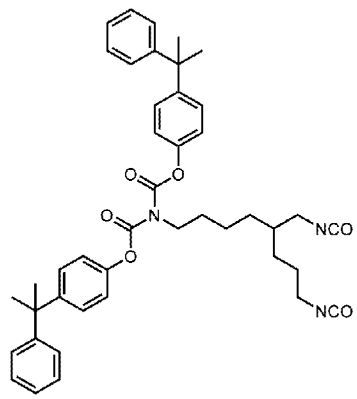

(I - 3a)

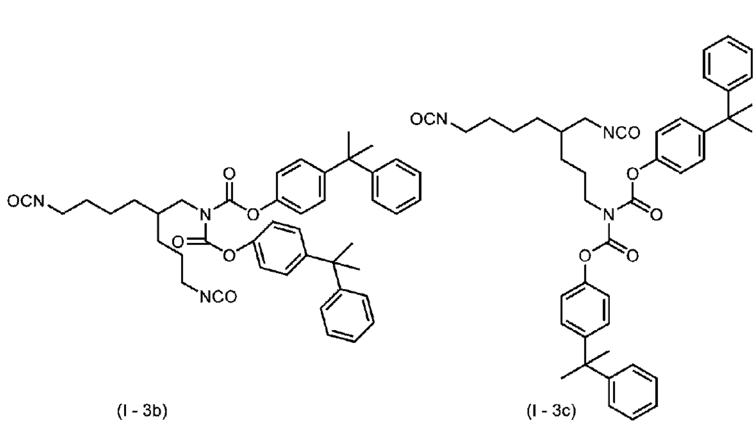

(I - 3b) (I - 3c)

Step (6-3): Light boiling separation step

Light boiling separation was carried out by the same operation as that of Example 1-1, except that the reaction solution (6-2) was continuously fed at 16.10 kg/hour and the extraction rate of the line 34 in a steady state was 15.77 kg/hour. The amount of the liquid recovered in the storage tank 303 was 31.55 kg, and as a result of analysis by NMR, LC, and gas chromatography, TTI was recovered at a yield of 95% by mass with respect to the supplied reaction solution (6-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.6.

Step (6-4): High boiling separation step

High boiling separation was carried out by the same operation as that of Example 1-1, except that the liquid recovered in the storage tank 303 in step (6-3) was used. The amount of the liquid recovered in the storage tank 402 was 4.19 kg, and the TTI recovery rate was 122% by mass.

(Comparative Example 1-1)

Step (1'-2): Pyrolysis step of carbamate compound

A pyrolysis reaction was carried out by the same operation as that of Example 1-1, except that 19.4 kg of the reaction solution (1-1) of Example 1-1 and 5.45 kg of Barrel process oil B-03 (benzyl toluene, Matsumura Oil Co., Ltd.) were used, the reaction solution (1-1) was supplied to the reactor over about 14 minutes to start the reaction, the reaction was carried out at a jacket temperature of 238°C, an internal temperature of 230°C, a reflux ratio of 1.5, and a pressure in a range of 25 to 35 kPa, and extraction of phenol was continued for 1.5 hours after the transfer of the entire reaction solution (1-1). A mass of the reaction solution transferred to the storage tank 205 was 5.71 kg. As a result of analysis of the reaction solution (hereinafter, referred to as "reaction solution (1'-2)") by NMR, LC, and gas chromatography, TTI was generated at a yield of 69% by mass. Generation of a carbonyl compound was not observed in the reaction solution

at this time, and by-products having an isocyanurate group, a carbodiimide group, a uretonimine group, or an allophanate group were observed.

Step (1'-3): Light boiling separation step

Light boiling separation was carried out by the same operation as that of Example 1-1, except that the reaction solution (1'-2) was continuously fed at 4.35 kg/hour and the extraction rate of the line 34 in a steady state was 1.74 kg/hour, and as a result, the viscosity of the liquid increased during the operation, and continuous operation became difficult.

[Example 1-7]

Step (7-1): Production step of carbamate compound

A reaction was carried out using the device shown in FIG. 1.

In a state in which the line 14 was closed, 3.33 kg (16.8 mol) of 4,4'-diaminodiphenylmethane was supplied from the storage tank 101 through the line 11 to the reaction container 104 made of SUS with a baffle, 2.53 kg (27.0 mol) of phenol was supplied from the storage tank 102 through the line 12 to the reaction container 104, and stirring was carried out for homogenization. Subsequently, in a state in which the line 16 was closed, 2.53 kg (27.0 mol) of phenol was supplied from the storage tank 102 through the line 15 to the reaction container 105 made of SUS with a baffle, and 11.96 kg (55.6 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of 4,4'-diaminodiphenylmethane and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 7.58 kg of phenol in the solution was extracted to the storage tank 107 through the line 17 and the capacitor (condensing device) A11.

As a result of analysis of the solution after the reaction (hereinafter, referred to as "reaction solution (7-1)") by liquid chromatography, a carbamate compound corresponding to 4,4'-diaminodiphenylmethane was generated at a yield of 95% by mass. The line 16 was opened, and the reaction solution (7-1) was transferred to the storage tank 106 through the line 16. A mass of the reaction solution (7-1) was 12.77 kg.

Step (7-2): Pyrolysis step of carbamate compound

A pyrolysis reaction was carried out by the same operation as that of Example 1-1, except that 12.77 kg of the reaction solution (7-1) and 12.77 kg of diphenyl carbonate were used, the reaction solution (7-1) was supplied to the reactor over about 10 minutes to start the reaction, the reaction was carried out at a jacket temperature of 238°C, an internal temperature of 230°C, a reflux ratio of 0.8, and a pressure in a range of 11 to 16 kPa, and extraction of phenol was continued for 3 hours after the transfer of the entire reaction solution (7-1). A mass of the reaction solution transferred to the storage tank 205 was 1 1.75 kg. A carbonyl compound was isolated by purifying a part of the reaction solution (hereinafter, referred to as "reaction solution (7-2)") with a column fractionation device. The isolated carbonyl compound was a compound represented by Formula (1-2).

[Chem. 71]

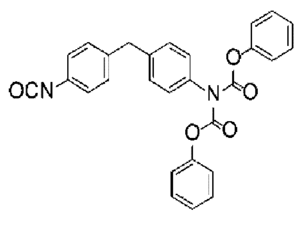

( I -2 )

A calibration curve of LC was created from the obtained carbonyl compound, and the carbonyl compound was quantified by an absolute calibration curve method. In addition, as a result of analysis by NMR and gas chromatography, diphenylmethane diisocyanate (MDI) was generated at a yield of 70% by mass. A value of { 3 × (molar amount of

isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.3.

Step (7-3): Light boiling separation step

Light boiling separation was carried out by the same operation as that of Example 1-1, except that the reaction solution (7-2) was continuously fed at 2.35 kg/hour and the extraction rate of the line 34 in a steady state was 1.97 kg/hour. The amount of the liquid recovered in the storage tank 303 was 9.87 kg, as a result of analysis by NMR, LC, and gas chromatography, MDI was recovered at a yield of 79% by mass with respect to the supplied reaction solution (7-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 2.3.

Step (7-4): High boiling separation step

High boiling separation was carried out by the same operation as that of Example 1-1, except that the liquid recovered in the storage tank 303 in step (7-3) was used, the operation temperature was set to 170°C, and the internal pressure was set to 0.3 kPa. The amount of the liquid recovered in the storage tank 402 was 4.54 kg, and the MDI recovery rate was 130% by mass.

[Example 1-8]

Step (8-1): Production step of carbamate compound

In a state in which the line 14 was closed, 3.33 kg (16.8 mol) of lysine β-aminoethyl ester trihydrochloride was supplied from the storage tank 101 through the line 11 to the reaction container 104 made of SUS with a baffle, 2.52 kg (26.7 mol) of phenol was supplied from the storage tank 102 through the line 12 to the reaction container 104, and stirring was carried out for homogenization. Subsequently, in a state in which the line 16 was closed, 2.52 kg (26.7 mol) of phenol was supplied from the storage tank 102 through the line 15 to the reaction container 105 made of SUS with a baffle, and 11.91 kg (55.6 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of lysine β-aminoethyl ester trihydrochloride and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 8.22 kg of phenol in the solution was extracted to the storage tank 107 through the line 17 and the capacitor (condensing device) A11.

As a result of analysis of the solution after the reaction (hereinafter, referred to as "reaction solution (8-1)") by liquid chromatography, a carbamate compound corresponding to lysine β-aminoethyl ester was generated at a yield of 96% by mass. The line 16 was opened, and the reaction solution (8-1) was transferred to the storage tank 106 through the line 16. A mass of the reaction solution (8-1) was 11.45 kg.

Step (8-2): Pyrolysis step of carbamate compound

A pyrolysis reaction was carried out by the same operation as that of Example 1-1, except that 11.45 kg of the reaction solution (8-1) and 10.00 kg of diphenyl carbonate were used, the reaction solution (8-1) was supplied to the reactor over about 15 minutes to start the reaction, the reaction was carried out at a jacket temperature of 238°C, an internal temperature of 230°C, a reflux ratio of 3.2, and a pressure in a range of 11 to 16 kPa, and extraction of phenol was continued for 3 hours after the transfer of the entire reaction solution (8-1). A mass of the reaction solution (hereinafter, referred to as "reaction solution (8-2)") transferred to the storage tank 205 was 9.01 kg. The results of each of $^1$H-NMR and gas chromatography-mass spectrometry of the reaction solution (8-2) are shown in FIGS. 6A and 6B. In addition, a carbonyl compound was isolated by purifying a part of the reaction solution (8-2) with a column fractionation device. The isolated carbonyl compound was a mixture of compounds represented by Formulae (I-4a) to (I-4c).

[Chem. 72]

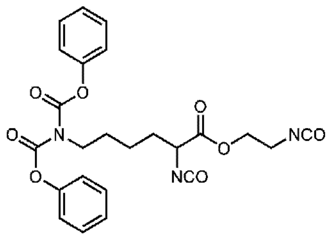
(I - 4a)

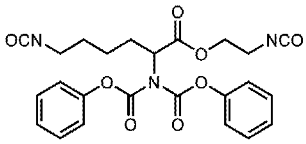
(I - 4b)

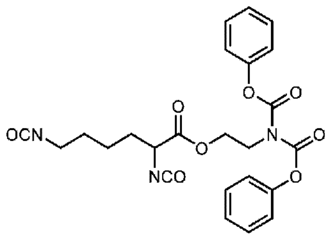
(I - 4c)

A calibration curve of LC was created from the obtained carbonyl compound, and the carbonyl compound was quantified by an absolute calibration curve method. In addition, as a result of analysis by NMR and gas chromatography, lysine triisocyanate (LTI) was generated at a yield of 77% by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.4.

Step (8-3): Light boiling separation step

Light boiling separation was carried out by the same operation as that of Example 1-1, except that the reaction solution (8-2) was continuously fed at 1.80 kg/hour and the extraction rate of the line 34 in a steady state was 1.10 kg/hour. The amount of the liquid recovered in the storage tank 303 was 5.50 kg, and as a result of analysis by NMR, LC, and gas chromatography, LTI was recovered at a yield of 83% by mass with respect to the supplied reaction solution (8-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 2.4.

Step (8-4): High boiling separation step

High boiling separation was carried out by the same operation as that of Example 1-1, except that the liquid recovered in the storage tank 303 in step (8-3) was used, the operation temperature was set to 190°C, and the internal pressure was set to 0.1 kPa. The amount of the liquid recovered in the storage tank 402 was 2.38 kg, and the LTI recovery rate was 125% by mass.

[Example 1-9]

Step (9-1): Production step of carbamate compound

Carbamate synthesis was carried out by the same operation as that of Example 1-8, except that 0.84 kg (6.75 mol) of 2-methoxyphenol instead of phenol was supplied to the reactor 104 made of SUS with a baffle, 0.84 kg (6.75 mol) of 2-methoxyphenol instead of phenol, and 15.26 kg (55.6 mol) of bis(2-methoxyphenyl)carbonate instead of diphenyl carbonate were supplied to the reactor 105 made of SUS with a baffle. The amount of 2-methoxyphenol extracted into the storage tank 107 was 6.35 kg.

As a result of analysis of the solution after the reaction (hereinafter, referred to as "reaction solution (9-1)") by liquid chromatography, the corresponding carbamate compound was generated at a yield of 98% by mass. The line 16 was opened, and the reaction solution (9-1) was transferred to the storage tank 106 through the line 16. A mass of the reaction solution (9-1) was 13.95 kg.

Step (9-2): Pyrolysis step of carbamate compound

A pyrolysis reaction was carried out by the same operation as that of Example 1-8, except that 14.0 kg of the reaction solution (9-1) and 8.0 kg of bis(2-methoxyphenyl)carbonate were used, the reaction solution (9-1) was supplied to the reactor over about 14 minutes to start the reaction, the reaction was carried out at a jacket temperature of 238°C, an internal temperature of 230°C, a reflux ratio of 3.0, and a pressure in a range of 8 to 13 kPa, and extraction of 2-methoxyphenol was continued for 3 hours after the transfer of the entire reaction solution (9-1). A mass of the reaction solution transferred to the storage tank 205 was 12.05 kg. As a result of analysis of the reaction solution (hereinafter, referred to as "reaction solution (9-2)") by NMR, LC, and gas chromatography, LTI was generated at a yield of 78% by

mass. A value of {3 × (molar amount of isocyanurate group) + 2 × (molar amount of carbodiimide group) + 3 × (molar amount of uretonimine group) + 2 × (molar amount of allophanate group)}/(molar amount of carbonyl compound) of the reaction solution at this time was 1.5. A carbonyl compound was isolated by purifying a part of the reaction solution (9-2) with a column fractionation device. The isolated carbonyl compound was a mixture of compounds represented by Formulae (I-5a) to (I-5c).

[Chem. 73]

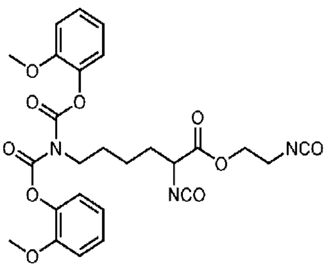
(I - 5a)

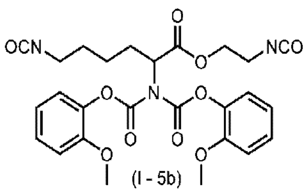
(I - 5b)

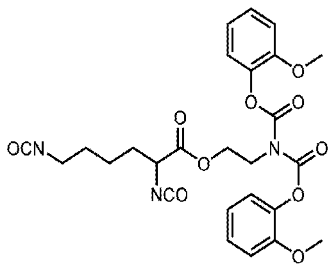
(I - 5c)

Step (9-3): Light boiling separation step

Light boiling separation was carried out by the same operation as that of Example 1-1, except that the reaction solution (9-2) was continuously fed at 2.41 kg/hour under the condition of a pressure of 1.0 kPa, and the extraction rate of the line 34 in a steady state was 1 .16 kg/hour. The amount of the liquid recovered in the storage tank 303 was 5.79 kg, and as a result of analysis by NMR, LC, and gas chromatography, LTI was recovered at a yield of 85% by mass with respect to the supplied reaction solution (9-2), and a value of {3 × (molar amount of isocyanurate group) + 2 × (molar amount of carbodiimide group) + 3 × (molar amount of uretonimine group) + 2 × (molar amount of allophanate group)} /(molar amount of carbonyl compound) of the reaction solution at this time was 2.2.

Step (9-4): High boiling separation step

High boiling separation was carried out by the same operation as that of Example 1-1, except that the liquid recovered in the storage tank 303 in step (9-3) was used. The amount of the liquid recovered in the storage tank 402 was 2.43 kg, and the LTI recovery rate was 123% by mass.

[Example 1-10]

Step (10-1): Production step of carbamate compound

In a state in which the line 14 was closed, 3.33 kg (14.3 mol) of lysine methyl ester dihydrochloride was supplied from the storage tank 101 through the line 11 to the reaction container 104 made of SUS with a baffle, 1.91 kg (20.3 mol) of phenol was supplied from the storage tank 102 through the line 12 to the reaction container 104, and stirring was carried out for homogenization. Subsequently, in a state in which the line 16 was closed, 1.91 kg (20.3 mol) of phenol was supplied from the storage tank 102 through the line 15 to the reaction container 105 made of SUS with a baffle, and 10.17 kg (47.5 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of lysine methyl ester dihydrochloride and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 5.93 kg of phenol in the solution was extracted to the storage tank 107 through the line 17 and the capacitor (condensing device) A11.

As a result of analysis of the solution after the reaction (hereinafter, referred to as "reaction solution (10-1)") by liquid chromatography, a carbamate compound corresponding to lysine methyl ester was generated at a yield of 97% by mass. The line 16 was opened, and the reaction solution (10-1) was transferred to the storage tank 106 through the line 16. A mass of the reaction solution (10-1) was 11.38 kg.

Step (10-2): Pyrolysis step of carbamate compound

A pyrolysis reaction was carried out by the same operation as that of Example 1-1, except that 11.38 kg of the reaction solution (10-1) and 11.38 kg of diphenyl carbonate were used, the reaction solution (10-1) was supplied to the reactor over about 12 minutes to start the reaction, the reaction was carried out at a jacket temperature of 238°C, an internal temperature of 230°C, a reflux ratio of 0.9, and a pressure in a range of 20 to 29 kPa, and extraction of phenol was continued for 3 hours after the transfer of the entire reaction solution (10-1). A mass of the reaction solution transferred to the storage tank 205 was 19.35 kg. A carbonyl compound was isolated by purifying a part of the reaction solution (hereinafter, referred to as "reaction solution (10-2)") with a column fractionation device. The isolated carbonyl compound was a mixture of compounds represented by Formulae (I-7a) to (I-7b).

[Chem. 74]

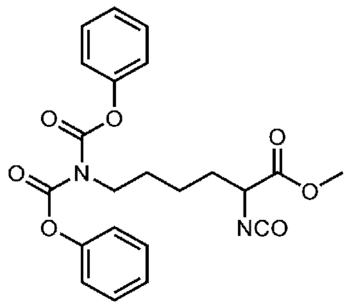

(I - 7a)

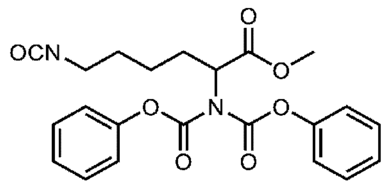

(I - 7b)

A calibration curve of LC was created from the obtained carbonyl compound, and the carbonyl compound was quantified by an absolute calibration curve method. In addition, as a result of analysis by NMR and gas chromatography, lysine diisocyanate (LDI) was generated at a yield of 81 % by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.4.

Step (10-3): Light boiling separation step

Light boiling separation was carried out by the same operation as that of Example 1-1, except that the reaction solution (10-2) was continuously fed at 3.87 kg/hour and the extraction rate of the line 34 in a steady state was 1.47 kg/hour. The amount of the liquid recovered in the storage tank 303 was 7.35 kg, and as a result of analysis by NMR, LC, and gas chromatography, LDI was recovered at a yield of 86% by mass with respect to the supplied reaction solution (10-2), and a value of {3 × (molar amount of isocyanurate group) + 2 × (molar amount of carbodiimide group) + 3 × (molar amount of uretonimine group) + 2 × (molar amount of allophanate group) I /(molar amount of carbonyl compound) of the reaction solution at this time was 2.5.

Step (10-4): High boiling separation step

High boiling separation was carried out by the same operation as that of Example 1-1, except that the liquid recovered in the storage tank 303 in step (10-3) was used, the operation temperature was set to 180°C, and the internal pressure was set to 0.1 kPa. The amount of the liquid recovered in the storage tank 402 was 4.57 kg, and the LDI recovery rate was 122% by mass.

[Example 1-11]

Step (11-1): Production step of carbamate compound

Carbamate synthesis was carried out by the same operation as that of Example 1-10, except that 3.33 kg (13.5 mol) of lysine ethyl ester dihydrochloride instead of lysine methyl ester dihydrochloride and 1.70 kg (18.04 mol) of phenol were supplied to the reactor 104 made of SUS with a baffle, and 1.70 kg (18.04 mol) of phenol and 9.59 kg (44.81 mol) of diphenyl carbonate were supplied to the reactor 105 made of SUS with a baffle. The amount of phenol extracted into the storage tank 107 was 5.59 kg.

As a result of analysis of the solution after the reaction (hereinafter, referred to as "reaction solution (11-1)") by liquid chromatography, the corresponding carbamate compound was generated at a yield of 97% by mass. The line 16 was opened, and the reaction solution (11-1) was transferred to the storage tank 106 through the line 16. A mass of the reaction solution (11-1) was 10.74 kg.

Step (11-2): Pyrolysis step of carbamate compound

A pyrolysis reaction was carried out by the same operation as that of Example 1-10, except that 10.7 kg of the reaction solution (11-1) and 10.74 kg of diphenyl carbonate were used, the reaction solution (11-1) was supplied to the reactor over about 12 minutes to start the reaction, the reaction was carried out at a jacket temperature of 238°C, an internal temperature of 230°C, a reflux ratio of 0.9, and a pressure in a range of 20 to 29 kPa, and extraction of phenol was continued for 3 hours after the transfer of the entire reaction solution (11-1). A mass of the reaction solution transferred to the storage tank 205 was 18.25 kg. As a result of analysis of the reaction solution (hereinafter, referred to as "reaction solution (11-2)") by NMR, LC, and gas chromatography, LDI-Et was generated at a yield of 82% by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.3. A carbonyl compound was isolated by purifying a part of the reaction solution (11-2) with a column fractionation device. The isolated carbonyl compound was a mixture of compounds represented by Formulae (I-10a) to (I-10b).

[Chem. 75]

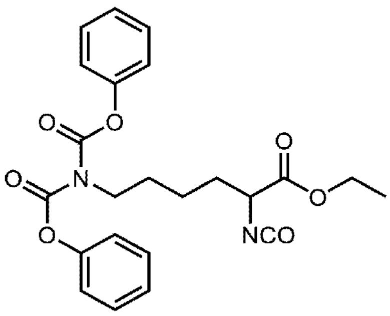

(I - 10a)

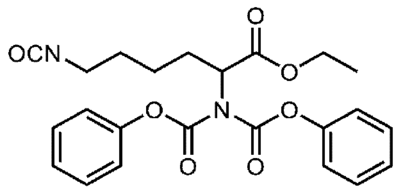

(I - 10b)

Step (11-3): Light boiling separation step

Light boiling separation was carried out by the same operation as that of Example 1-1, except that the reaction solution (11-2) was continuously fed at 3.65 kg/hour and the extraction rate of the line 34 in a steady state was 1.31 kg/hour. The amount of the liquid recovered in the storage tank 303 was 6.57 kg, and as a result of analysis by NMR, LC, and gas chromatography, LDI-Et was recovered at a yield of 85% by mass with respect to the supplied reaction solution (11-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 2.2.

Step (11-4): High boiling separation step

High boiling separation was carried out by the same operation as that of Example 1-1, except that the liquid recovered in the storage tank 303 in step (11-3) was used. The amount of the liquid recovered in the storage tank 402 was 4.10 kg, and the recovery rate of LDI-Et was 120% by mass.

[Example 1-12]

Step (12-1): Production step of carbamate compound

In a state in which the line 14 was closed, 3.33 kg (15.8 mol) of 4,4'-methylenebis(cyclohexylamine) was supplied from the storage tank 101 through the line 11 to the reaction container 104 made of SUS with a baffle, and 2.29 kg (24.4 mol) of phenol was supplied from the storage tank 102 through the line 12 to the reaction container 104, and stirring

was carried out for homogenization. Subsequently, in a state in which the line 16 was closed, 2.29 kg (24.4 mol) of phenol was supplied from the storage tank 102 through the line 15 to the reaction container 105 made of SUS with a baffle, and 11.27 kg (52.7 mol) of diphenyl carbonate was supplied from the storage tank 103 through the line 13 to the reaction container 105. The liquid temperature of the reaction container 105 was adjusted to 65°C, stirring was carried out for homogenization, and then the mixture solution of 4,4'-methylenebis(cyclohexylamine) and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 6.94 kg of phenol in the solution was extracted to the storage tank 107 through the line 17 and the capacitor (condensing device) A11.

As a result of analysis of the solution after the reaction (hereinafter, referred to as "reaction solution (12-1)") by liquid chromatography, a carbamate compound corresponding to 4,4'-methylenebis(cyclohexylamine) was generated at a yield of 99% by mass. The line 16 was opened, and the reaction solution (12-1) was transferred to the storage tank 106 through the line 16. A mass of the reaction solution (12-1) was 12.24 kg.

Step (12-2): Pyrolysis step of carbamate compound

A pyrolysis reaction was carried out by the same operation as that of Example 1-1, except that 12.24 kg of the reaction solution (12-1) and 12.24 kg of diphenyl carbonate were used, the reaction solution (12-1) was supplied to the reactor over about 10 minutes to start the reaction, the reaction was carried out at a jacket temperature of 238°C, an internal temperature of 230°C, a reflux ratio of 4.5, and a pressure in a range of 11 to 16 kPa, and extraction of phenol was continued for 3 hours after the transfer of the entire reaction solution (12-1). A mass of the reaction solution transferred to the storage tank 205 was 9.79 kg. A carbonyl compound was isolated by purifying a part of the reaction solution (hereinafter, referred to as "reaction solution (12-2)") with a column fractionation device. The isolated carbonyl compound was a compound represented by Formula (1-16).

[Chem. 76]

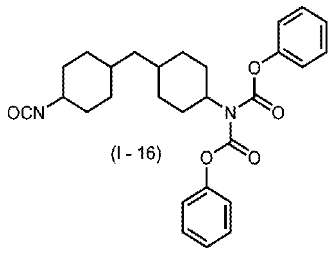

(I - 16)

A calibration curve of LC was created from the obtained carbonyl compound, and the carbonyl compound was quantified by an absolute calibration curve method. In addition, as a result of analysis by NMR, LC, and gas chromatography, methylenebis(cyclohexylisocyanate) (HMDI) was generated at a yield of 76% by mass. A value of {3 × (molar amount of isocyanurate group) + 2 × (molar amount of carbodiimide group) + 3 × (molar amount of uretonimine group) + 2 × (molar amount of allophanate group)}/(molar amount of carbonyl compound) the value of the reaction solution at this time was 2.0.

Step (12-3): Light boiling separation step

Light boiling separation was carried out by the same operation as that of Example 1-1, except that the reaction solution (12-2) was continuously fed at 1.96 kg/hour and the extraction rate of the line 34 in a steady state was 1.65 kg/hour. The amount of the liquid recovered in the storage tank 303 was 8.22 kg, and, as a result of analysis by NMR, LC, and gas chromatography, HMDI was recovered at a yield of 82% by mass with respect to the supplied reaction solution (12-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 3.4.

Step (12-4): High boiling separation step

High boiling separation was carried out by the same operation as that of Example 1-1, except that the liquid recovered in the storage tank 303 in step (12-3) was used, the operation temperature was set to 190°C, and the internal

pressure was set to 0.3 kPa. The amount of the liquid recovered in the storage tank 402 was 4.85 kg, and the HMDI recovery rate was 125% by mass.

[Example 1-13]

Step (13-1): Production step of carbamate compound

In a state in which the line 14 was closed, 3.33 kg (23.1 mol) of 1,3-di(aminomethyl)cyclohexane was supplied from the storage tank 101 through the line 11 to the reaction container 104 made of SUS with a baffle, 4.11 kg (43.7 mol) of phenol was supplied from the storage tank 102 through the line 12 to the reaction container 104, and stirring was carried out for homogenization. Subsequently, in a state in which the line 16 was closed, 4.11 kg (43.7 mol) of phenol was supplied from the storage tank 102 through the line 15 to the reaction container 105 made of SUS with a baffle, and 16.44 kg (76.8 mol) of diphenyl carbonate was supplied from the storage tank 103 through the line 13 to the reaction container 105. The liquid temperature of the reaction container 105 was adjusted to 65°C, stirring was carried out for homogenization, and then the mixture solution of 1,3-di(aminomethyl)cyclohexane and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 11.74 kg of phenol in the solution was extracted to the storage tank 107 through the line 17 and the capacitor (condensing device) A11.

As a result of analysis of the solution after the reaction (hereinafter, also referred to as "reaction solution (13-1)") by liquid chromatography, a carbamate compound corresponding to 1,3-di(aminomethyl)cyclohexane was generated at a yield of 99% by mass. The line 16 was opened, and the reaction solution (13-1) was transferred to the storage tank 106 through the line 16. A mass of the reaction solution (13-1) was 16.24 kg.

Step (13-2): Pyrolysis step of carbamate compound

A pyrolysis reaction was carried out by the same operation as that of Example 1-1, except that 16.24 kg of the reaction solution (13-1) and 16.24 kg of diphenyl carbonate were used, the reaction solution (13-1) was supplied to the reactor over about 15 minutes to start the reaction, the reaction was carried out at a jacket temperature of 238°C, an internal temperature of 230°C, a reflux ratio of 4.0, and a pressure in a range of 11 to 16 kPa, and extraction of phenol was continued for 3 hours after the transfer of the entire reaction solution (13-1). A mass of the reaction solution transferred to the storage tank 205 was 13.97 kg. A carbonyl compound was isolated by purifying a part of the reaction solution (hereinafter, referred to as "reaction solution (13-2)") with a column fractionation device. The isolated carbonyl compound was a compound represented by Formula (I-19).

[Chem. 77]

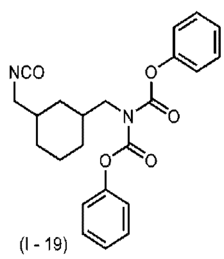

(I - 19)

A calibration curve of LC was created from the obtained carbonyl compound, and the carbonyl compound was quantified by an absolute calibration curve method. In addition, as a result of analysis by NMR, LC, and gas chromatography, 1,3-bis(isocyanatomethyl)cyclohexane (HXDI) was generated at a yield of 78% by mass. A value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 1.7.

Step (13-3): Light boiling separation step

Light boiling separation was carried out by the same operation as that of Example 1-1, except that the reaction solution (13-2) was continuously fed at 2.79 kg/hour and the extraction rate of the line 34 in a steady state was 1.90

kg/hour. The amount of the liquid recovered in the storage tank 303 was 9.50 kg, and as a result of analysis by NMR, LC, and gas chromatography, HXDI was recovered at a yield of 77% by mass with respect to the supplied reaction solution (13-2), and a value of {3 × (molar amount of isocyanurate group) + 2 × (molar amount of carbodiimide group) + 3 × (molar amount of uretonimine group) + 2 × (molar amount of allophanate group) }/(molar amount of carbonyl compound) of the reaction solution at this time was 3.6.

Step (13-4): High boiling separation step

High boiling separation was carried out by the same operation as that of Example 1-1, except that the liquid recovered in the storage tank 303 in step (13-3) was used, the operation temperature was set to 190°C, and the internal pressure was set to 0.3 kPa. The amount of the liquid recovered in the storage tank 402 was 4.93 kg, and the HXDI recovery rate was 122% by mass.

<Production of isocyanate compound and carbonyl compound (I) corresponding to isocyanate compound>

[Synthesis Example 1-1]

(Production of TTI and carbonyl compounds (I-1a) to (I-1c) corresponding to TTI)

1. Step (1-1): Production step of carbamate compound

A reaction was carried out using the device shown in FIG. 1. The device shown in FIG. 1 was also used in the production of carbamate compounds after Synthesis Example 1-1.

In a state in which the line 14 was closed, 3.33 kg (19.2 mol) of 4-aminomethyl-1,8-octanediamine was supplied from the storage tank 101 through the line 11 to the reaction container 104 made of SUS with a baffle, 5.50 kg (58.5 mol) of phenol was supplied from the storage tank 102 through the line 12 to the reaction container 104, and stirring was carried out for homogenization. Subsequently, in a state in which the line 16 was closed, 5.50 kg (58.5 mol) of phenol was supplied from the storage tank 102 through the line 15 to the reactor 105 made of SUS with a baffle, and 20.52 kg (95.9 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of 4-aminomethyl-1,8-octanediamine and phenol was supplied from the reaction container 104 through the line 14 so that the internal temperature thereof did not exceed 70°C. After continuing stirring for 2 hours, as a temperature of the reaction solution was raised to 120°C, and the internal pressure was set to about 1 kPa, 15.53 kg of phenol in the solution was extracted to the storage tank 107 through the line 17 and the capacitor (condensing device) A11.

As a result of analyzing the solution after the reaction (hereinafter, referred to as "reaction solution (1-1)") by liquid chromatography, a carbamate compound corresponding to 4-aminomethyl-1,8-octanediamine was generated at a yield of 99% by mass. The line 16 was opened, and the reaction solution (1-1) was transferred to the storage tank 106 through the line 16. A mass of the reaction solution (1-1) was 19.40 kg.

2. Step (1-2): Pyrolysis step of carbamate compound

The reaction was carried out using the device shown in FIG. 2. The device shown in FIG. 2 was also used for pyrolysis of the carbamate compounds in Synthesis Example 1-1 and the subsequent examples.

In a state in which the line 24 was closed, 19.40 kg of diphenyl carbonate was supplied from the storage tank 202 through the line 22 to the reaction container 201 made of SUS with a baffle. A temperature of the multi-stage distillation column 203 was raised to 170°C, a jacket temperature of the reaction container 201 was heated to 228°C, and a pressure was reduced to 14 kPa. 19.40 kg of the reaction solution (1-1) recovered in the storage tank 106 in step (1-1) was heated to 120°C, and supplied to the reaction container 201 through the line 21 for about 15 minutes to carry out pyrolysis of the carbamate compound. The pressure was adjusted in a range of 8 to 14 kPa, and the phenol generated by pyrolysis was separated from diphenyl carbonate or 1,8-diisocyanato-4-isocyanatomethyloctane (TTI) as the product in the distillation column 203, and was recovered in the storage tank 204 through the line 25, the capacitor A21 and the line 27. A reflux ratio at this time was 1.2. After the entire reaction solution (1-1) was transferred, phenol was continuously extracted at an internal temperature of 220°C. The reaction was ended 4 hours after the transfer of the entire reaction solution (1-1), and the reaction solution was extracted from a line 28 and transferred to a storage tank 205. A mass of the reaction solution transferred to the storage tank 205 was 18.63 kg. A part of the reaction solution (hereinafter, referred to as "reaction solution (1-2)") was purified with a column fractionation device to isolate the carbonyl compound. The isolated carbonyl compound was a mixture of compounds represented by Formulae (I-1a) to (I-1c) (hereinafter, the

mixture was referred to as "mixture (I-1)"in some cases). In addition, as a result of $^1$H-NMR and gas chromatography analysis, TTI was generated at a yield of 70% by mass.

[Chem. 78]

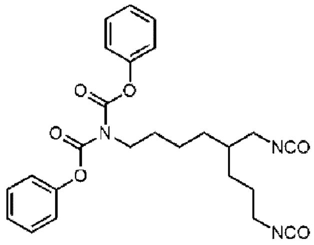
(I - 1a)

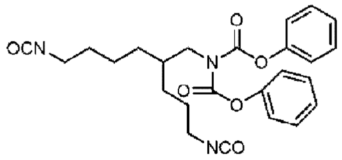
(I - 1b)

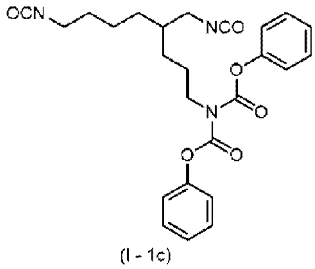
(I - 1c)

3. Step (1-3): Light boiling separation step

The reaction was carried out using the device shown in FIG. 3. The device shown in FIG. 3 was also used for the light boiling separation in Synthesis Example 1-1 and the subsequent examples.

The reaction solution (1-2) was continuously fed to a middle stage of the continuous multi-stage distillation column 301 from the storage tank 205 through the line 31 at 3.73 kg/hour, and distillation separation of the liquid phase component was carried out. A heat amount required for distillation separation was supplied by circulating a column lower liquid through the reboiler A32 and the line 33. A liquid temperature at a column bottom portion of the continuous multi-stage distillation column was 220°C, and a pressure at the top of the column was 1.5 kPa. The gas distilled from the top of the continuous multi-stage distillation column 301 was condensed in the capacitor (condensing device) A31 through the line 32, and continuously extracted to the storage tank 302 through the line 36. In addition, an extraction speed of the line 34 in a steady state was 1.49 kg/hour, and continuously extracted to the storage tank 303. The amount of the liquid recovered in the storage tank 303 (hereinafter, referred to as "reaction solution (1-3)") was 7.45 kg, and as a result of analysis by NMR, LC, and gas chromatography, TTI was recovered at a yield of 82% by mass with respect to the supplied reaction solution (1-2).

4. Step (1-4): High boiling separation step

The reaction was carried out using the device shown in FIG. 4. The device shown in FIG. 4 was also used for the high boiling separation in Synthesis Example 1-1 and the subsequent examples.

The thin-film distillation device 401 (manufactured by Kobelco Eco-Solutions Co., Ltd., Japan) was heated to 190°C, and the internal pressure was set to 0.3 kPa. The reaction solution (1-3) recovered in the storage tank 303 in step (1-3) was supplied to an upper portion of the thin-film distillation device 401 at about 1.0 kg/hour through the line 41 to carry out separation of the isocyanate and a high boiling component. The generated gas-phase component was transferred to the storage tank 402 through the line 42 and the capacitor (condensing device) A41. An amount of the liquid recovered from the storage tank 402 was 3.35 kg, and a TTI recovery rate was 121% by mass. The reason why the TTI recovery rate exceeds 100% by mass is that a part of the isocyanate denaturation product generated in the pyrolysis step or the light boiling separation step is regenerated to TTI.

[Synthesis Example 1-2]

(Production of TTI and carbonyl compounds (I-2a) to (I-2c) corresponding to TTI)

TTI was produced using the same method as that of Synthesis Example 1-1, except that 2-methoxyphenol was used instead of phenol, and bis(2-methoxyphenyl)carbonate was used instead of diphenyl carbonate in Synthesis Example 1-1. At this time, the isolated carbonyl compound was a mixture of compounds represented by Formulae (I-2a) to (I-2c) (hereinafter, the mixture is referred to as "mixture (1-2)" in some cases).

[Chem. 79]

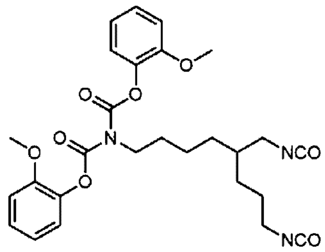

(I - 2a)

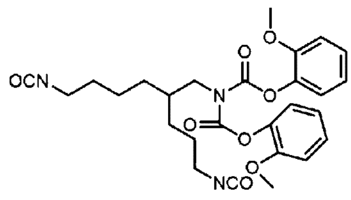

(I - 2b)

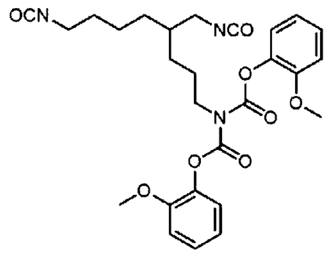

(I - 2c)

[Synthesis Example 1-3]

(Production of TTI and carbonyl compounds (I-3a) to (I-3c) corresponding to TTI)

TTI was produced using the same method as that of Synthesis Example 1-1, except that 4-cumylphenol was used instead of phenol, and bis(4-cumylphenol)carbonate was used instead of diphenyl carbonate in Synthesis Example 1-1. At this time, the isolated carbonyl compound was a mixture of compounds represented by Formulae (I-3a) to (I-3c) (hereinafter, the mixture is referred to as "mixture (I-3)" in some cases).

[Chem. 80]

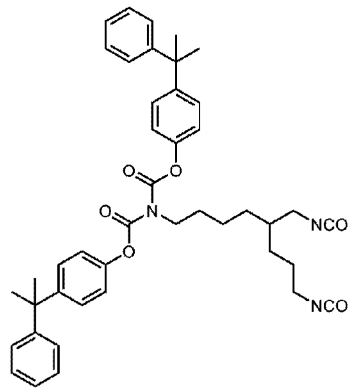

(I - 3a)

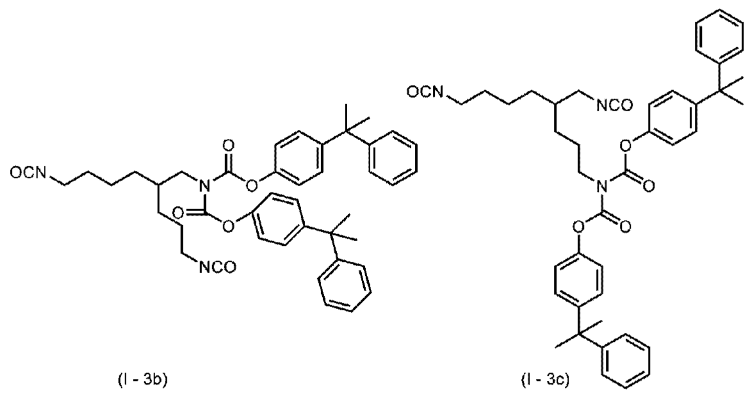

(I - 3b)

(I - 3c)

[Synthesis Example 1-4]

(Production of LTI and carbonyl compounds (I-4a) to (I-4b) corresponding to LTI)

1. Step (4-1): Production step of carbamate compound

In a state in which the line 14 was closed, 3.33 kg (16.8 mol) of lysine β-aminoethyl ester trihydrochloride was supplied from the storage tank 101 through the line 11 to the reaction container 104 made of SUS with a baffle, 2.52 kg (26.7 mol) of phenol was supplied from the storage tank 102 through the line 12 to the reaction container 104, and stirring was carried out for homogenization. Subsequently, in a state in which the line 16 was closed, 2.52 kg (26.7 mol) of phenol was supplied from the storage tank 102 through the line 15 to the reaction container 105 made of SUS with a baffle, and 11.91 kg (55.6 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of lysine β-aminoethyl ester trihydrochloride and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 8.22 kg of phenol in the solution was extracted to the storage tank 107 through the line 17 and

the capacitor (condensing device) A11.

As a result of analysis of the solution after the reaction (hereinafter, referred to as "reaction solution (4-1)") by liquid chromatography, a carbamate compound corresponding to lysine β-aminoethyl ester was generated at a yield of 96% by mass. The line 16 was opened, and the reaction solution (4-1) was transferred to the storage tank 106 through the line 16. A mass of the reaction solution (4-1) was 11.45 kg.

2. Step (4-2): Pyrolysis step of carbamate compound

A pyrolysis reaction was carried out by the same operation as that of Synthesis Example 1-1, except that 11.45 kg of the reaction solution (4-1) and 10.00 kg of diphenyl carbonate were used, the reaction solution (4-1) was supplied to the reactor over about 15 minutes to start the reaction, the reaction was carried out at a jacket temperature of 238°C, an internal temperature of 230°C, a reflux ratio of 3.2, and a pressure in a range of 11 to 16 kPa, and extraction of phenol was continued for 3 hours after the transfer of the entire reaction solution (4-1). A mass of the reaction solution transferred to the storage tank 205 was 9.01 kg. A carbonyl compound was isolated by purifying a part of the reaction solution (hereinafter, referred to as "reaction solution (4-2)") with a column fractionation device. The isolated carbonyl compound was a mixture of compounds represented by Formulae (I-4a) to (I-4c). (Hereinafter, the mixture may be referred to as "mixture (1-4)" in some cases). In addition, as a result of analysis by NMR and gas chromatography, lysine triisocyanate (LTI) was generated at a yield of 77% by mass.

[Chem. 81]

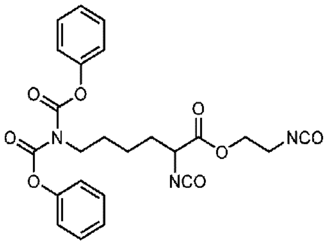

(I - 4a)

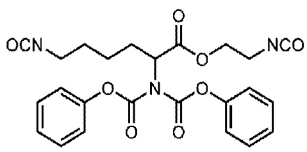

(I - 4b)

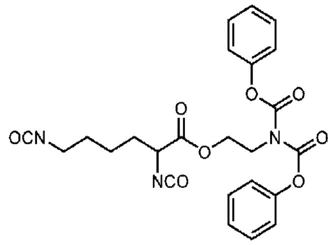

(I - 4c)

3. Step (4-3): Light boiling separation step

Light boiling separation was carried out by the same operation as that of Synthesis Example 1-1, except that the reaction solution (4-2) was continuously fed at 1.80 kg/hour and the extraction rate of the line 34 in a steady state was 1.10 kg/hour. The amount of the liquid recovered in the storage tank 303 was 5.50 kg, and as a result of analysis by NMR, LC, and gas chromatography, LTI was recovered at a yield of 83% by mass with respect to the supplied reaction solution (4-2).

4. Step (4-4): High boiling separation step

High boiling separation was carried out by the same operation as that of Synthesis Example 1-1, except that the liquid recovered in the storage tank 303 in step (4-3) was used, the operation temperature was set to 190°C, and the internal pressure was set to 0.1 kPa. The amount of the liquid recovered in the storage tank 402 was 2.38 kg, and the LTI recovery rate was 125% by mass.

[Synthesis Example 1-5]

(Production of LTI and carbonyl compounds (I-5a) to (I-5b) corresponding to LTI)

LTI was produced using the same method as that of Synthesis Example 1-4, except that 2-methoxyphenol was used instead of phenol, and bis(2-methoxyphenyl)carbonate was used instead of diphenyl carbonate in Synthesis Example 1-4. At this time, the isolated carbonyl compound was a mixture of compounds represented by Formulae (I-5a) to (I-5c) (hereinafter, the mixture is referred to as "mixture (I-5)" in some cases).

[Chem. 82]

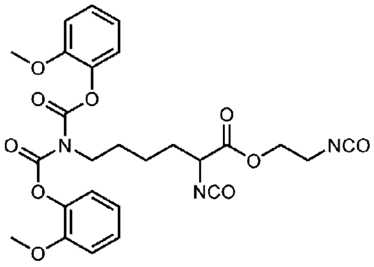
(I - 5a)

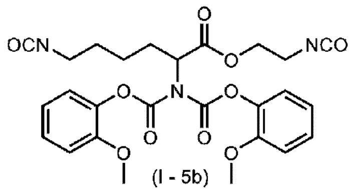
(I - 5b)

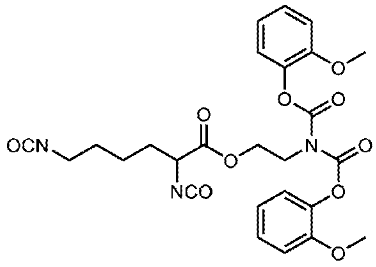
(I - 5c)

[Synthesis Example 1-6]

(Production of LDI and carbonyl compounds (I-7a) and (I-7b) corresponding to LDI)

1. Step (6-1): Production step of carbamate compound

In a state in which the line 14 was closed, 3.33 kg (14.3 mol) of lysine methyl ester dihydrochloride was supplied from the storage tank 101 through the line 11 to the reaction container 104 made of SUS with a baffle, 1.91 kg (20.3 mol) of phenol was supplied from the storage tank 102 through the line 12 to the reaction container 104, and stirring was carried out for homogenization. Subsequently, in a state in which the line 16 was closed, 1.91 kg (20.3 mol) of phenol was supplied from the storage tank 102 through the line 15 to the reaction container 105 made of SUS with a baffle, and 10.17 kg (47.5 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of lysine methyl ester dihydrochloride and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 5.93 kg of phenol in the solution was extracted to the storage tank 107 through the line 17 and the capacitor (condensing device) A11.

As a result of analysis of the solution after the reaction (hereinafter, referred to as "reaction solution (6-1)") by liquid chromatography, a carbamate compound corresponding to lysine methyl ester was generated at a yield of 97% by mass. The line 16 was opened, and the reaction solution (6-1) was transferred to the storage tank 106 through the line 16. A mass of the reaction solution (6-1) was 11.38 kg.

2. Step (6-2): Pyrolysis step of carbamate compound

A pyrolysis reaction was carried out by the same operation as that of Synthesis Example 1-1, except that 11.38 kg of the reaction solution (6-1) and 11.38 kg of diphenyl carbonate were used, the reaction solution (6-1) was supplied to the reactor over about 12 minutes to start the reaction, the reaction was carried out at a jacket temperature of 238°C, an internal temperature of 230°C, a reflux ratio of 0.9, and a pressure in a range of 20 to 29 kPa, and extraction of phenol was continued for 3 hours after the transfer of the entire reaction solution (6-1). A mass of the reaction solution transferred to the storage tank 205 was 19.35 kg. A carbonyl compound was isolated by purifying a part of the reaction solution (hereinafter, referred to as "reaction solution (6-2)") with a column fractionation device. The isolated carbonyl compound was a mixture of compounds represented by Formulae (I-7a) to (I-7b) (hereinafter, the mixture is referred to as "mixture (1-7)" in some cases). In addition, as a result of analysis by NMR and gas chromatography, lysine diisocyanate (LDI) was generated at a yield of 81% by mass.

[Chem. 83]

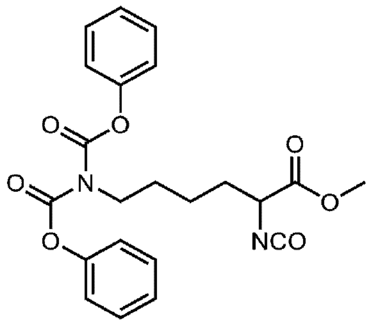

(I - 7a)

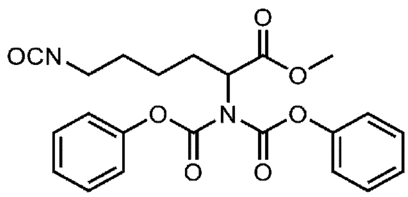

(I - 7b)

3. Step (6-3): Light boiling separation step

Light boiling separation was carried out by the same operation as that of Synthesis Example 1-1, except that the reaction solution (6-2) was continuously fed at 3.87 kg/hour and the extraction rate of the line 34 in a steady state was 1.47 kg/hour. The amount of the liquid recovered in the storage tank 303 was 7.35 kg, and as a result of analysis by NMR, LC, and gas chromatography, LDI was recovered at a yield of 86% by mass with respect to the supplied reaction solution (6-2).

4. Step (6-4): High boiling separation step

High boiling separation was carried out by the same operation as that of Synthesis Example 1-1, except that the liquid recovered in the storage tank 303 in step (6-3) was used, the operation temperature was set to 180°C, and the internal pressure was set to 0.1 kPa. The amount of the liquid recovered in the storage tank 402 was 4.57 kg, and the LDI recovery rate was 122% by mass.

[Synthesis Example 1-7]

(Production of LDI and carbonyl compounds (I-8a) and (I-8b) corresponding to

LDI)

LDI was produced using the same method as that of Synthesis Example 1-6, except that 2-methoxyphenol was used instead of phenol, and bis(2-methoxyphenyl)carbonate was used instead of diphenyl carbonate in Synthesis Example 1-6. At this time, the isolated carbonyl compound was a mixture of compounds represented by Formulae (I-8a) to (I-8b) (hereinafter, the mixture is referred to as "mixture (I-8)" in some cases).

[Chem. 84]

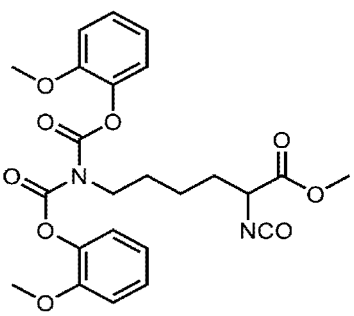

(I - 8a)

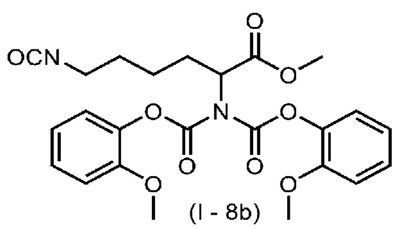

(I - 8b)

[Synthesis Example 1-8]

(Production of LDI-Et and carbonyl compounds (I-10a) to (I-10b) corresponding to LDI-Et)

LDI-Et was produced using the same method as that of Synthesis Example 1-6, except that lysine ethyl ester dihydrochloride was used instead of lysine methyl ester dihydrochloride in Synthesis Example 1-6. At this time, the isolated carbonyl compound was a mixture of compounds represented by Formulae (I-10a) to (I-10b) (hereinafter, the mixture is referred to as "mixture (1-10)" in some cases).

[Chem. 85]

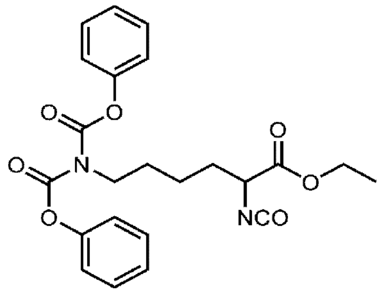

(I - 10a)

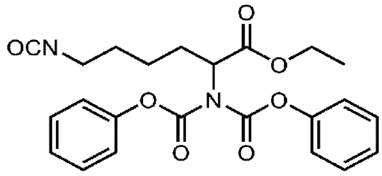

(I - 10b)

[Synthesis Example 1-9]

(Production of HDI and carbonyl compound (I-20) corresponding to HDI)

1. Step (9-1): Production step of carbamate compound

As a corresponding carbamate was synthesized from hexamethylenediamine, the temperature of the reaction solution was raised to 120°C, and the internal pressure was set to 1.0 kPa, 15.42 kg of phenol in the solution was extracted, by the same method as that in step (1-1), except that 3.33 kg of hexamethylenediamine was used instead of 4-aminomethyl-1,8-octanediamine and 20.40 kg of diphenyl carbonate was used, 5.50 kg of phenol was mixed with hexamethylenediamine, and 5.50 kg of phenol was supplied to the reactor.
As a result of analysis of the solution after the reaction (hereinafter, referred to as "reaction solution (9-1)") by liquid chromatography, a carbamate compound corresponding to hexamethylenediamine was generated at a yield of 99% by mass. A mass of the reaction solution (9-1) was 19.31 kg.

2. Step (9-2): Pyrolysis step of carbamate compound

A pyrolysis reaction was carried out by the same operation as that of Synthesis Example 1-1, except that 19.31 kg of the reaction solution (9-1) and 19.31 kg of diphenyl carbonate were used, the reaction solution (9-1) was supplied to the reactor over about 15 minutes to start the reaction, the reaction was carried out at a jacket temperature of 238°C, an internal temperature of 230°C, a reflux ratio of 2.2, and a pressure in a range of 20 to 29 kPa, and extraction of phenol was continued for 3 hours after the transfer of the entire reaction solution (9-1). A mass of the reaction solution after the reaction (hereinafter, referred to as "reaction solution (9-2)") was 33.22 kg. The carbonyl compound in the reaction solution (9-2) was isolated. The isolated carbonyl compound was a compound represented by Formula (1-20).

[Chem. 86]

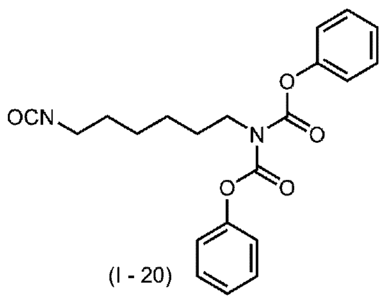

(I - 20)

In addition, as a result of analysis by NMR and gas chromatography, hexamethylene diisocyanate (HDI) was generated at a yield of 78% by mass.

3. Step (9-3): Light boiling separation step

Light boiling separation was carried out by the same operation as that of Synthesis Example 1-1, except that the reaction solution (9-2) was continuously fed at 6.64 kg/hour and the extraction rate of the line 34 in a steady state was 0.930 kg/hour. The amount of the liquid recovered in the storage tank 303 was 4.65 kg, and as a result of analysis by NMR, LC, and gas chromatography, HDI was recovered at a yield of 83% by mass with respect to the supplied reaction solution (9-2).

4. Step (9-4): High boiling separation step

High boiling separation was carried out by the same operation as that of Synthesis Example 1-1, except that the liquid recovered in the storage tank 303 in step (9-3) was used, the operation temperature was set to 170°C, and the internal pressure was set to 0.1 kPa. The amount of the liquid recovered in the storage tank 402 was 3.55 kg, and the HDI recovery rate was 122% by mass.

[Synthesis Example 1-10]

(Production of HMDI and carbonyl compound (1-8) corresponding to HMDI)

1. Step (10-1): Production step of carbamate compound

In a state in which the line 14 was closed, 3.33 kg (15.8 mol) of 4,4'-methylenebis(cyclohexylamine) was supplied from the storage tank 101 through the line 11 to the reaction container 104 made of SUS with a baffle, and 2.29 kg (24.4 mol) of phenol was supplied from the storage tank 102 through the line 12 to the reaction container 104, and stirring was carried out for homogenization. Subsequently, in a state in which the line 16 was closed, 2.29 kg (24.4 mol) of phenol was supplied from the storage tank 102 through the line 15 to the reaction container 105 made of SUS with a baffle, and 11.27 kg (52.7 mol) of diphenyl carbonate was supplied from the storage tank 103 through the line 13 to the reaction container 105. The liquid temperature of the reaction container 105 was adjusted to 65°C, stirring was carried out for homogenization, and then the mixture solution of 4,4'-methylenebis(cyclohexylamine) and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 6.94 kg of phenol in the solution was extracted to the storage tank 107 through the line 17 and the capacitor (condensing device) A11.

As a result of analysis of the solution after the reaction (hereinafter, referred to as "reaction solution (10-1)") by liquid chromatography, a carbamate compound corresponding to 4,4'-methylenebis(cyclohexylamine) was generated at a yield of 99% by mass. The line 16 was opened, and the reaction solution (10-1) was transferred to the storage tank 106 through the line 16. A mass of the reaction solution (8-1) was 12.24 kg.

2. Step (10-2): Pyrolysis step of carbamate compound

A pyrolysis reaction was carried out by the same operation as that of Synthesis Example 1-1, except that 12.24 kg of the reaction solution (10-1) and 12.24 kg of diphenyl carbonate were used, the reaction solution (10-1) was supplied to the reactor over about 10 minutes to start the reaction, the reaction was carried out at a jacket temperature of 238°C, an internal temperature of 230°C, a reflux ratio of 4.5, and a pressure in a range of 11 to 16 kPa, and extraction of phenol was continued for 3 hours after the transfer of the entire reaction solution (10-1). A mass of the reaction solution transferred to the storage tank 205 was 9.79 kg. A carbonyl compound was isolated by purifying a part of the reaction solution (hereinafter, referred to as "reaction solution (10-2)") with a column fractionation device. The isolated carbonyl compound was a compound represented by Formula (1-16).

[Chem. 87]

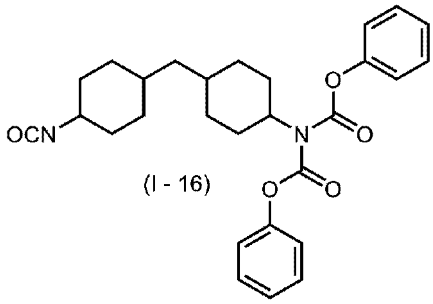

(I - 16)

In addition, as a result of analysis by NMR, LC, and gas chromatography, methylenebis(cyclohexylisocyanate) (HMDI) was generated at a yield of 76% by mass.

3. Step (10-3): Light boiling separation step

Light boiling separation was carried out by the same operation as that of Synthesis Example 1-1, except that the reaction solution (10-2) was continuously fed at 1.96 kg/hour and the extraction rate of the line 34 in a steady state was 1.65 kg/hour. The amount of the liquid recovered in the storage tank 303 was 8.22 kg, and as a result of analysis by NMR, LC, and gas chromatography, HMDI was recovered at a yield of 82% by mass with respect to the supplied reaction solution (10-2).

4. Step (10-4): High boiling separation step

High boiling separation was carried out by the same operation as that of Synthesis Example 1-1, except that the liquid recovered in the storage tank 303 in step (10-3) was used, the operation temperature was set to 190°C, and the internal pressure was set to 0.3 kPa. The amount of the liquid recovered in the storage tank 402 was 4.85 kg, and the HMDI recovery rate was 125% by mass.

[Synthesis Example 1-11]

(Production of HXDI and carbonyl compound (1-19) corresponding to HXDI)

1. Step (11-1): Production step of carbamate compound

In a state in which the line 14 was closed, 3.33 kg (23.1 mol) of 1,3-di(aminomethyl)cyclohexane was supplied from the storage tank 101 through the line 11 to the reaction container 104 made of SUS with a baffle, 4.11 kg (43.7 mol) of phenol was supplied from the storage tank 102 through the line 12 to the reaction container 104, and stirring was carried out for homogenization. Subsequently, in a state in which the line 16 was closed, 4.11 kg (43.7 mol) of phenol was supplied from the storage tank 102 through the line 15 to the reaction container 105 made of SUS with a baffle, and 16.44 kg (76.8 mol) of diphenyl carbonate was supplied from the storage tank 103 through the line 13 to the reaction container 105. The liquid temperature of the reaction container 105 was adjusted to 65°C, stirring was carried out for homogenization, and then the mixture solution of 1,3-di(aminomethyl)cyclohexane and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 11.74 kg of phenol in the solution was extracted to the storage tank 107 through the line 17 and the capacitor (condenser) A11.

As a result of analysis of the solution after the reaction (hereinafter, also referred to as "reaction solution (11-1)") by liquid chromatography, a carbamate compound corresponding to 1,3-di(aminomethyl)cyclohexane was generated at a yield of 99% by mass. The line 16 was opened, and the reaction solution (11-1) was transferred to the storage tank 106 through the line 16. A mass of the reaction solution (11-1) was 16.24 kg.

2. Step (11-2): Pyrolysis step of carbamate compound

A pyrolysis reaction was carried out by the same operation as that of Synthesis Example 1-1, except that 16.24

kg of the reaction solution (11-1) and 16.24 kg of diphenyl carbonate were used, the reaction solution (11-1) was supplied to the reactor over about 15 minutes to start the reaction, the reaction was carried out at a jacket temperature of 238°C, an internal temperature of 230°C, a reflux ratio of 4.0, and a pressure in a range of 11 to 16 kPa, and extraction of phenol was continued for 3 hours after the transfer of the entire reaction solution (11-1). A mass of the reaction solution transferred to the storage tank 205 was 13.97 kg. A carbonyl compound was isolated by purifying a part of the reaction solution (hereinafter, referred to as "reaction solution (11-2)") with a column fractionation device. The isolated carbonyl compound was a compound represented by Formula (1-19).

[Chem. 88]

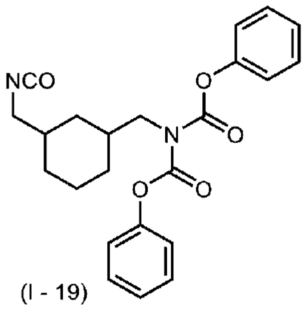

(I - 19)

In addition, as a result of analysis by NMR, LC, and gas chromatography, 1,3-bis(isocyanatomethyl)cyclohexane (HXDI) was generated at a yield of 78% by mass.

3. Step (11-3): Light boiling separation step

Light boiling separation was carried out by the same operation as that of Synthesis Example 1-1, except that the reaction solution (11-2) was continuously fed at 2.79 kg/hour and the extraction rate of the line 34 in a steady state was 1.90 kg/hour. The amount of the liquid recovered in the storage tank 303 was 9.50 kg, and as a result of analysis by NMR, LC, and gas chromatography, HXDI was recovered at a yield of 77% by mass with respect to the supplied reaction solution (11-2).

4. Step (11-4): High boiling separation step

High boiling separation was carried out by the same operation as that of Synthesis Example 1-1, except that the liquid recovered in the storage tank 303 in step (11-3) was used, the operation temperature was set to 190°C, and the internal pressure was set to 0.3 kPa. The amount of the liquid recovered in the storage tank 402 was 4.93 kg, and the HMDI recovery rate was 122% by mass.

[Synthesis Example 1-12]

(Production of PDI and carbonyl compound (1-21) corresponding to PDI)

1. Step (12-1): Production step of carbamate compound

As a corresponding carbamate was synthesized from 1,5-diaminopentane, the temperature of the reaction solution was raised to 120°C, and the internal pressure was set to 1.0 kPa, 18.01 kg of phenol in the solution was extracted, by the same method as that in step (1-1) of Synthesis Example 1-1, except that 3.33 kg of 1,5-diaminopentane was used instead of 4-aminomethyl-1,8-octanediamine and 23.20 kg of diphenyl carbonate was used, 6.48 kg of phenol was mixed with 1,5-diaminopentane, and 6.48 kg of phenol was supplied to the reactor.

As a result of analysis of the solution after the reaction (hereinafter, referred to as "reaction solution (12-1)") by liquid chromatography, a carbamate compound corresponding to 1,5-diaminopentane was generated at a yield of 99% by mass. The amount of 1,5-diaminopentane in the reaction solution (12-1) was 21.48 kg.

2. Step (12-2): Pyrolysis step of carbamate compound

A pyrolysis reaction was carried out by the same operation as that of Synthesis Example 1-1, except that 21.48

kg of the reaction solution (12-1) and 21.48 kg of diphenyl carbonate were used, the reaction solution (12-1) was supplied to the reactor over about 11 minutes to start the reaction, the reaction was carried out at a jacket temperature of 238°C, an internal temperature of 230°C, a reflux ratio of 4.5, and a pressure in a range of 20 to 29 kPa, and extraction of phenol was continued for 3 hours after the transfer of the entire reaction solution (12-1). A mass of the reaction solution after the reaction was 37.38 kg. A carbonyl compound was isolated by purifying a part of the reaction solution (hereinafter, referred to as "reaction solution (12-2)") with a column fractionation device. The isolated carbonyl compound was a compound represented by Formula (I-21).

[Chem. 89]

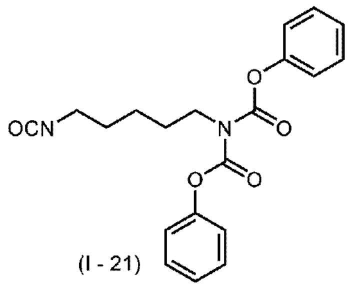

(I - 21)

In addition, as a result of analysis by NMR and gas chromatography, pentamethylene diisocyanate (PDI) was generated at a yield of 71% by mass.

3. Step (12-3): Light boiling separation step

Light boiling separation was carried out by the same operation as that of Synthesis Example 1-1, except that the reaction solution (12-2) was continuously fed at 7.48 kg/hour and the extraction rate of the line 34 in a steady state was 1.20 kg/hour. The amount of the liquid recovered in the storage tank 303 was 5.98 kg, and as a result of analysis by NMR, LC, and gas chromatography, PDI was recovered at a yield of 87% by mass with respect to the supplied reaction solution (12-2).

4. Step (12-4): High boiling separation step

High boiling separation was carried out by the same operation as that of Synthesis Example 1-1, except that the liquid recovered in the storage tank 303 in step (12-3) was used, the operation temperature was set to 160°C, and the internal pressure was set to 0.3 kPa. The amount of the liquid recovered in the storage tank 402 was 3.53 kg, and the PDI recovery rate was 115%.

[Synthesis Example 1-13]

(Production of IPDI and carbonyl compounds (I-22a) and (I-22b) corresponding to IPDI)

1. Step (13-1): Production step of carbamate compound

As a corresponding carbamate was synthesized from isophoronediamine, the temperature of the reaction solution was raised to 120°C, and the internal pressure was set to 1.0 kPa, 9.4 kg of phenol in the solution was extracted, using the same method as that of step (1-1) of Synthesis Example 1-1, except that 3.33 kg of isophoronediamine was used instead of 4-aminomethyl-1,8-octanediamine and 13.92 kg of diphenyl carbonate was used, 3.22 kg of phenol was mixed with isophoronediamine, and 3.22 kg of phenol was supplied to the reactor.

As a result of analysis of the solution after the reaction (hereinafter, referred to as "reaction solution (13-1)") by liquid chromatography, a carbamate compound corresponding to isophoronediamine was generated at a yield of 99% by mass. A mass of the reaction solution (13-1) was 14.29 kg.

2. Step (13-2): Pyrolysis step of carbamate compound

A pyrolysis reaction was carried out by the same operation as that of Synthesis Example 1-1, except that 14.29 kg of the reaction solution (13-2) and 14.29 kg of diphenyl carbonate were used, the reaction solution (13-2) was supplied

to the reactor over about 11 minutes to start the reaction, the reaction was carried out at a jacket temperature of 238°C, an internal temperature of 230°C, a reflux ratio of 4.9, and a pressure in a range of 20 to 29 kPa, and extraction of phenol was continued for 3 hours after the transfer of the entire reaction solution (13-2). A mass of the reaction solution after the reaction was 23.44 kg. A carbonyl compound was isolated by purifying a part of the reaction solution (hereinafter, referred to as "reaction solution (13-2)") with a column fractionation device. The isolated carbonyl compound was a mixture of compounds represented by Formulae (I-22a) to (I-22b) (hereinafter, the mixture is referred to as "mixture (I-22)" in some cases).

[Chem. 90]

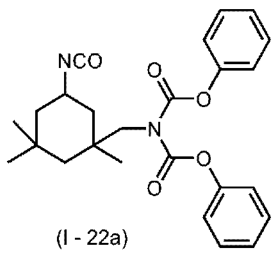

(I - 22a)

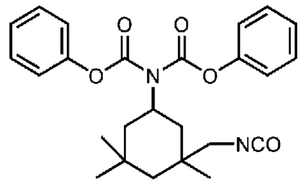

(I - 22b)

In addition, as a result of analysis by NMR and gas chromatography, isophorone diisocyanate (IPDI) was generated at a yield of 75% by mass.

3. Step (13-3): Light boiling separation step

Light boiling separation was carried out by the same operation as that of Synthesis Example 1-1, except that the reaction solution (13-2) was continuously fed at 4.69 kg/hour and the extraction rate of the line 34 in a steady state was 0.80 kg/hour. The amount of the liquid recovered in the storage tank 303 was 3.98 kg, and as a result of analysis by NMR, LC, and gas chromatography, IPDI was recovered at a yield of 82% by mass with respect to the supplied reaction solution (13-2).

4. Step (13-4): High boiling separation step

High boiling separation was carried out by the same operation as that of Synthesis Example 1-1, except that the liquid recovered in the storage tank 303 in step (13-3) was used, the operation temperature was set to 190°C, and the internal pressure was set to 0.3 kPa. The amount of the liquid recovered in the storage tank 402 was 3.18 kg, and the IPDI recovery rate was 120% by mass.

[Synthesis Example 1-14]

(Production of XDI and carbonyl compound (1-23) corresponding to XDI)

1. Step (14-1): Production step of carbamate compound

As a corresponding carbamate was synthesized from xylylenediamine, the temperature of the reaction solution was raised to 120°C, and the internal pressure was set to 1.0 kPa, 12.64 kg of phenol in the solution was extracted, using the same method as that of step (1-1) of Synthesis Example 1-1, except that 3.33 kg of xylylenediamine was used instead of 4-aminomethyl-1,8-octanediamine, and 17.42 kg of diphenyl carbonate was used, 4.45 kg of phenol was mixed with xylylenediamine, and 4.45 kg of phenol was supplied to the reactor.

As a result of analysis of the solution after the reaction (hereinafter, referred to as "reaction solution (14-1)") by liquid chromatography, a carbamate compound corresponding to xylylenediamine was generated at a yield of 98% by mass. A mass of the reaction solution (14-1) was 16.99 kg.

2. Step (14-2): Pyrolysis step of carbamate compound

A pyrolysis reaction was carried out by the same operation as that of Synthesis Example 1-1, except that 16.99 kg of the reaction solution (14-1) and 16.99 kg of diphenyl carbonate were used, the reaction solution (14-1) was supplied

to the reactor over about 11 minutes to start the reaction, the reaction was carried out at a jacket temperature of 238°C, an internal temperature of 230°C, a reflux ratio of 4.5, and a pressure in a range of 20 to 29 kPa, and extraction of phenol was continued for 3 hours after the transfer of the entire reaction solution (14-2). A mass of the reaction solution after the reaction was 28.89 kg. A carbonyl compound was isolated by purifying a part of the reaction solution (hereinafter, referred to as "reaction solution (14-2)") with a column fractionation device. The isolated carbonyl compound was a compound represented by Formula (1-23).

[Chem. 91]

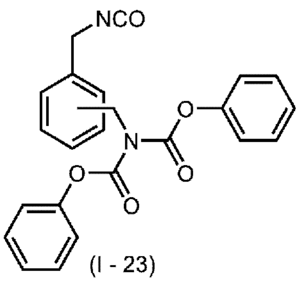

(I - 23)

In addition, as a result of analysis by NMR and gas chromatography, xylylene diisocyanate (XDI) was generated at a yield of 78% by mass.

3. Step (14-3): Light boiling separation step

Light boiling separation was carried out by the same operation as that of Synthesis Example 1-1, except that the reaction solution (14-2) was continuously fed at 5.78 kg/hour and the extraction rate of the line 34 in a steady state was 0.87 kg/hour. The amount of the liquid recovered in the storage tank 303 was 3.98 kg, and as a result of analysis by NMR, LC, and gas chromatography, XDI was recovered at a yield of 88% by mass with respect to the supplied reaction solution (14-2).

4. Step (14-4): High boiling separation step

High boiling separation was carried out by the same operation as that of Synthesis Example 1-1, except that the liquid recovered in the storage tank 303 in step (14-3) was used, the operation temperature was set to 170°C, and the internal pressure was set to 0.3 kPa. The amount of the liquid recovered in the storage tank 402 was 3.87 kg, and the XDI recovery rate was 125% by mass.

[Synthesis Example 1-15]

(Production of MDI and carbonyl compound (I-13) corresponding to MDI)

1. Step (15-1): Production step of carbamate compound

In a state in which the line 14 was closed, 3.33 kg (16.8 mol) of 4,4'-diaminodiphenylmethane was supplied from the storage tank 101 through the line 11 to the reaction container 104 made of SUS with a baffle, 2.53 kg (27.0 mol) of phenol was supplied from the storage tank 102 through the line 12 to the reaction container 104, and stirring was carried out for homogenization. Subsequently, in a state in which the line 16 was closed, 2.53 kg (27.0 mol) of phenol was supplied from the storage tank 102 through the line 15 to the reaction container 105 made of SUS with a baffle, and 11.96 kg (55.6 mol) of diphenyl carbonate was supplied from the storage tank 103 through the line 13 to the reaction container 105. A liquid temperature of the reaction container 105 was adjusted to 65°C, stirring was carried out for homogenization, and then the mixture solution of 4,4'-diaminodiphenylmethane and phenol was supplied from the reaction container 104 through the line 14 so that the internal temperature thereof did not exceed 70°C. After continuing stirring for 2 hours, as the temperature of the reaction solution was raised to 120°C, and the internal pressure was set to about 1 kPa, 7.58 kg of phenol in the solution was extracted to the storage tank 107 through the line 17 and the capacitor (condensing device) A11.

As a result of analysis of the solution after the reaction (hereinafter, referred to as "reaction solution (15-1)") by liquid chromatography, a carbamate compound corresponding to 4,4'-diaminodiphenylmethane was generated at a

yield of 95% by mass. The line 16 was opened, and the reaction solution (15-1) was transferred to the storage tank 106 through the line 16. A mass of the reaction solution (15-1) was 12.77 kg.

2. Step (15-2): Pyrolysis step of carbamate compound

A pyrolysis reaction was carried out by the same operation as that of Synthesis Example 1-1, except that 12.77 kg of the reaction solution (15-1) and 12.77 kg of diphenyl carbonate were used, the reaction solution (15-1) was supplied to the reactor over about 10 minutes to start the reaction, the reaction was carried out at a jacket temperature of 238°C, an internal temperature of 230°C, a reflux ratio of 0.8, a pressure in a range of 11 to 16 kPa, and extraction of phenol was continued for 3 hours after the transfer of the entire reaction solution (15-1). A mass of the reaction solution transferred to the storage tank 205 was 11.75 kg. A carbonyl compound was isolated by purifying a part of the reaction solution (hereinafter, referred to as "reaction solution (15-2)") with a column fractionation device. The isolated carbonyl compound was a compound represented by Formula (1-13).

[Chem. 92]

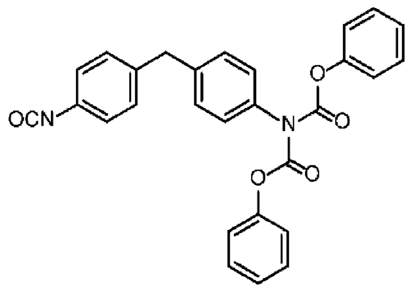

( I -13 )

In addition, as a result of analysis by NMR and gas chromatography, MDI was generated at a yield of 70% by mass.

3. Step (15-3): Light boiling separation step

Light boiling separation was carried out by the same operation as that of Synthesis Example 1-1, except that the reaction solution (15-2) was continuously fed at 2.35 kg/hour and the extraction rate of the line 34 in a steady state was 1.97 kg/hour. The amount of the liquid recovered in the storage tank 303 was 9.87 kg, and as a result of analysis by NMR, LC, and gas chromatography, MDI was recovered at a yield of 79% by mass with respect to the supplied reaction solution (15-2).

4. Step (15-4): High boiling separation step

High boiling separation was carried out by the same operation as that of Synthesis Example 1-1, except that the liquid recovered in the storage tank 303 in step (15-3) was used, the operating temperature was set to 170°C, and the internal pressure was set to 0.3 kPa. The amount of the liquid recovered in the storage tank 402 was 4.54 kg, and the MDI recovery rate was 130% by mass.

[Synthesis Example 1-16]

(Production of TDI and carbonyl compounds (I-24a) to (I-24b) corresponding to TDI)

1. Step (16-1): Production step of carbamate compound

As a corresponding carbamate was synthesized from tolylene-2,4-diamine, the temperature of the reaction solution was raised to 120°C, and the internal pressure was set to 1.0 kPa, 14.49 kg of phenol in the solution was extracted, using the same method as that of step (1-1) of Synthesis Example 1-1, except that 3.33 kg of tolylene-2,4-diamine was used instead of 4-aminomethyl-1,8-octanediamine and 19.41 kg of diphenyl carbonate were used, 5.15 kg of phenol was mixed with xylylenediamine, and 5.15 kg of phenol was supplied to the reactor.

As a result of analysis of the solution after the reaction (hereinafter, referred to as "reaction solution (16-1)") by liquid chromatography, the corresponding carbamate compound was generated at a yield of 96% by mass. A mass

of the reaction solution (16-1) was 18.54 kg.

2. Step (16-2): Pyrolysis step of carbamate compound

A pyrolysis reaction was carried out by the same operation as that of Synthesis Example 1-1, except that 18.54 kg of the reaction solution (16-1) and 18.54 kg of diphenyl carbonate were used, the reaction solution (16-1) was supplied to the reactor over about 11 minutes to start the reaction, the reaction was carried out at a jacket temperature of 238°C, an internal temperature of 230°C, a reflux ratio of 4.7, and a pressure in a range of 20 to 29 kPa, and extraction of phenol was continued for 3 hours after the transfer of the entire reaction solution (16-1). A mass of the reaction solution after the reaction was 11.75 kg. A carbonyl compound was isolated by purifying a part of the reaction solution (hereinafter, referred to as "reaction solution (16-2)") with a column fractionation device. The isolated carbonyl compound was a mixture of compounds represented by Formulae (I-24a) to (I-24b) (hereinafter, the mixture is referred to as "mixture (I-24)" in some cases).

[Chem. 93]

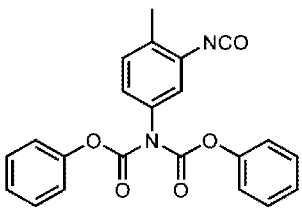

(I - 24a)

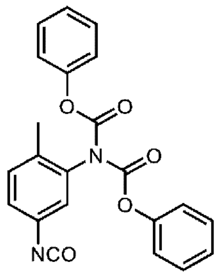

(I - 24b)

In addition, as a result of analysis by NMR and gas chromatography, toluene diisocyanate (TDI) was generated at a yield of 71 % by mass.

3. Step (16-3): Light boiling separation step

Light boiling separation was carried out by the same operation as that of Synthesis Example 1-1, except that the reaction solution (16-2) was continuously fed at 2.35 kg/hour and the extraction rate of the line 34 in a steady state was 0.94 kg/hour. The amount of the liquid recovered in the storage tank 303 was 4.70 kg, and as a result of analysis by NMR, LC, and gas chromatography, TDI was recovered at a yield of 85% by mass with respect to the supplied reaction solution (16-2).

4. Step (16-4): High boiling separation step

High boiling separation was carried out by the same operation as that of Synthesis Example 1-1, except that the liquid recovered in the storage tank 303 in step (16-3) was used, the operation temperature was set to 160°C, and the internal pressure was set to 0.3 kPa. The amount of the liquid recovered in the storage tank 402 was 3.27 kg, and the TDI recovery rate was 119% by mass.

<Production of carbamate compound (III)>

[Synthesis Example 2-1]

(Production of carbamate compounds (III-1a) to (III-24b))

A carbamate compound (III) corresponding to each isocyanate compound was synthesized by adding a hydroxy compound (V) of the type and the blending amount described in the following table to 100.0 g of each isocyanate compound, and reacting the mixture at 120°C for 3 hours.

In addition, carbamate compounds (III-1a) to (III-1c) were obtained as the mixture (III-1) thereof, carbamate compounds (III-2a) to (III-2c) were obtained as the mixture (III-2) thereof, carbamate compounds (III-3a) to (III-3c) were obtained as the mixture (III-3) thereof, carbamate compounds (III-4a) to (III-4c) were obtained as the mixture (III-4)

thereof, carbamate compounds (III-5a) to (III-5b) were obtained as the mixture (III-5) thereof, carbamate compounds (III-7a) to (III-7b) were obtained as the mixture (III-7) thereof, carbamate compounds (III-8a) to (III-8b) were obtained as the mixture (III-8) thereof, carbamate compounds (III-10a) to (III-10b) were obtained as the mixture (III-10) thereof, carbamate compounds (III-22a) to (III-22b) were obtained as the mixture (III-11) thereof, and carbamate compounds (III-24a) to (III-24b) were obtained as the mixture (III-24) thereof. Structures of these carbamate compounds are as described below.

[Table 1]

| Carbamate compound (III) | Raw material | | | |
|---|---|---|---|---|
| | Isocyanate compound (I) | | Hydroxy compound (V) | |
| | Type | g | Type | g |
| (III-1) | TTI | 100.0 | Phenol | 37.5 |
| (III-2) | TTI | 100.0 | 2-methoxyphenol | 49.5 |
| (III-3) | TTI | 100.0 | 4-cumylphenol | 84.6 |
| (III-4) | LTI | 100.0 | Phenol | 35.2 |
| (III-5) | LTI | 100.0 | 2-methoxyphenol | 46.5 |
| (III-7) | LDI | 100.0 | Phenol | 44.3 |
| (III-8) | LDI | 100.0 | 2-methoxyphenol | 58.5 |
| (III-10) | LDI-Et | 100.0 | Phenol | 38.0 |
| (III-20) | HDI | 100.0 | Phenol | 55.9 |
| (III-16) | HMDI | 100.0 | Phenol | 35.8 |
| (III-19) | HXDI | 100.0 | Phenol | 48.4 |
| (III-21) | PDI | 100.0 | Phenol | 61.0 |
| (III-22) | IPDI | 100.0 | Phenol | 42.3 |
| (III-23) | XDI | 100.0 | Phenol | 49.9 |
| (III-13) | MDI | 100.0 | Phenol | 37.6 |
| (III-24) | TDI | 100.0 | Phenol | 54.0 |

(Examples 1 to 62 and Comparative Examples 1 to 6)

(Production of isocyanate compositions A-a1 to A-a46 and A-b1 to A-b6)

Each isocyanate composition was obtained by mixing isocyanate compound (II), carbonyl compound (I), carbamate compound (III), and carbonate (IV) such that types and contents thereof are as shown in each table.

In addition, details of the types of the isocyanate compound (II), the carbonyl compound (I), the carbamate compound (III), and the carbonate (IV) used are as shown below.

(Isocyanate compound (II))

As the isocyanate compound (II), compounds shown below obtained by the above-described synthesis method were used.

TTI: 4-Isocyanatomethyl-1,8-octamethylene diisocyanate
LTI: 2-Isocyanatoethyl-2,6-diisocyanatohexanoate (lysine triisocyanate)
LDT: Lysine methyl ester diisocyanate
LDI-Et: Lysine ethyl ester diisocyanate
HDI: Diisocyanatohexane
HMDI: Methylenebis(cyclohexyl isocyanate)

HXDI: 1,3-Bis(isocyanatomethyl)cyclohexane
PDI: Diisocyanatopentane
IPDI: 3-Isocyanatomethyl-3,5,5-trimethylcyclohexyl isocyanate
XDI: Diisocyanatoxylene
MDI: Diisocyanatodiphenylmethane
TDI: Diisocyanatotoluene

(Carbonyl compound (I))

As the carbonyl compound (I), compounds represented by Formulae (I-1a) to (I-24b) obtained by the above-described synthesis method were used. In addition, for the carbonyl compounds (I-1a) to (I-1c), the mixture (I-1) thereof was used, for the carbonyl compounds (L2a) to (L2c), the mixture (1-2) thereof was used, for the carbonyl compounds (I-3a) to (I-3c), the mixture (1-3) thereof was used, for the carbonyl compounds (I-4a) to (I-4c), the mixture (I-4) thereof was used, for the carbonyl compounds (1-5a) to (1-5b), the mixture (1-5) thereof was used, for the carbonyl compounds (I-7a) to (I-7b), the mixture (I-7) thereof was used, for the carbonyl compounds (1-8a) to (1-8b), the mixture (1-8) thereof was used, for the carbonyl compounds (I-10a) to (I-10b), the mixture (1-10) thereof was used, for the carbonyl compounds (I-22a) to (I-22b), the mixture (1-11) thereof was used, and for the carbonyl compounds (I-24a) to (I-24b), the mixture (1-24) thereof was used.

[Chem. 94]

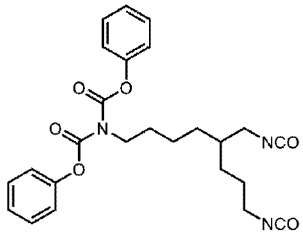
(I - 1a)

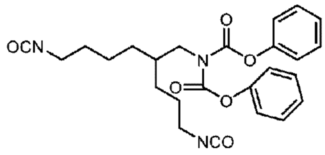
(I - 1b)

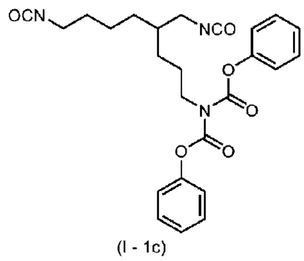
(I - 1c)

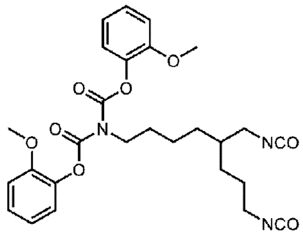
(I - 2a)

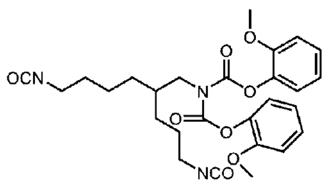
(I - 2b)

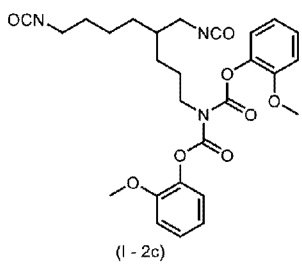
(I - 2c)

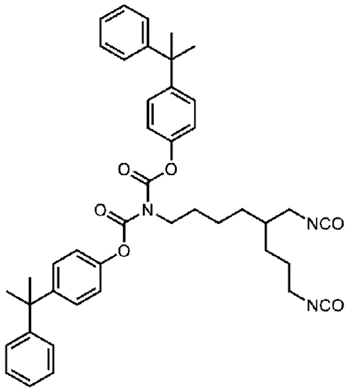
(I - 3a)

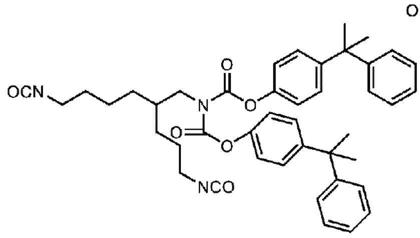
(I - 3b)

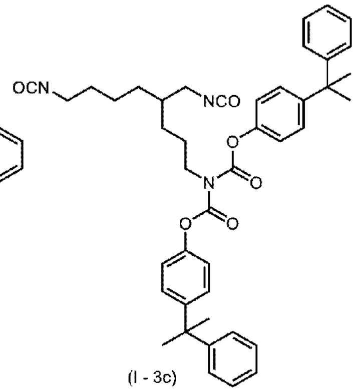
(I - 3c)

[Chem. 95]

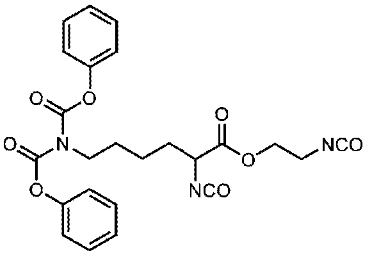
(I - 4a)

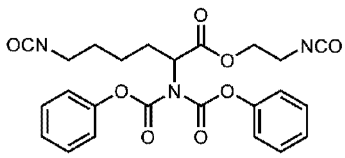
(I - 4b)

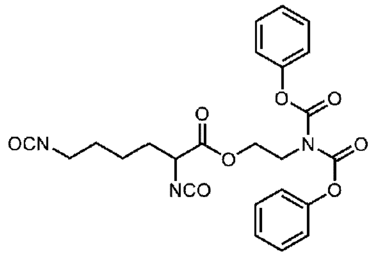
(I - 4c)

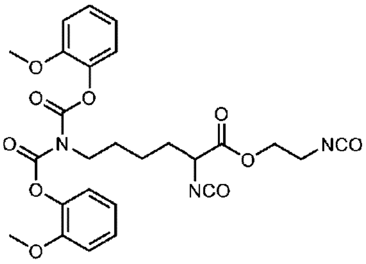
(I - 5a)

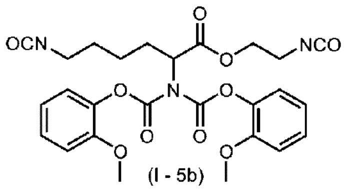
(I - 5b)

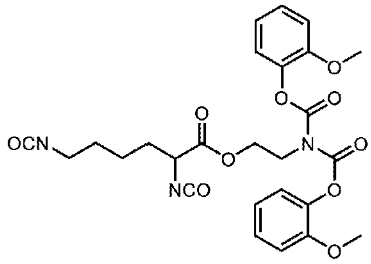
(I - 5c)

[Chem. 96]

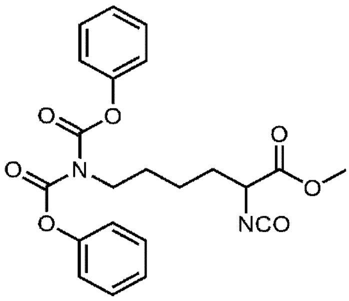

(I - 7a)

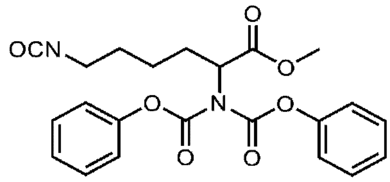

(I - 7b)

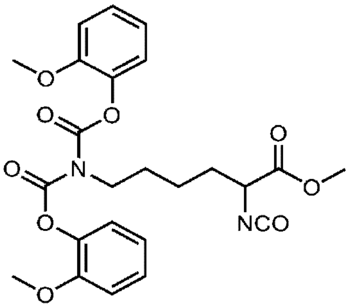

(I - 8a)

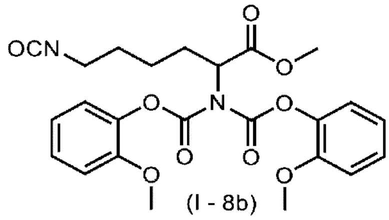

(I - 8b)

[Chem. 97]

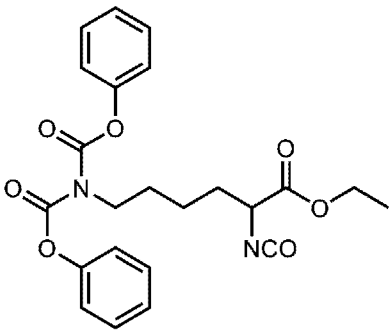

(I - 10a)

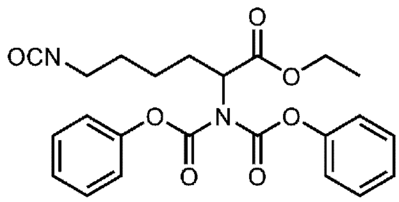

(I - 10b)

[Chem. 98]

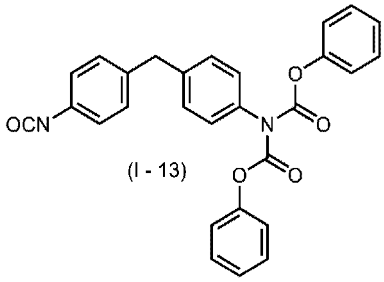

(I - 13)

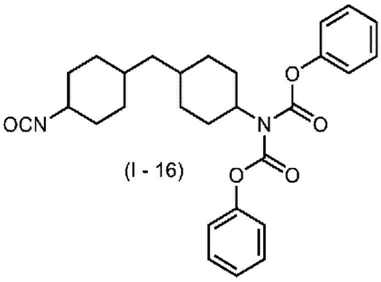

(I - 16)

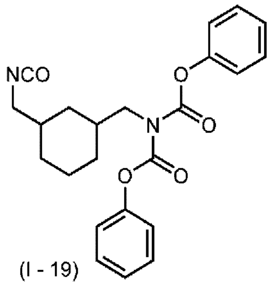

(I - 19)

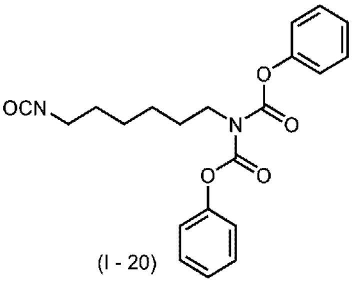

(I - 20)

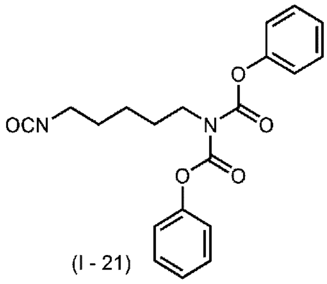

(I - 21)

[Chem. 99]

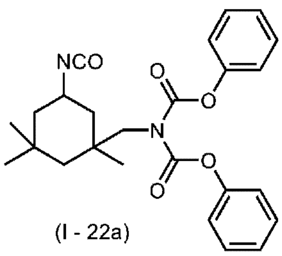

(I - 22a)

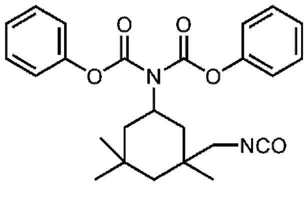

(I - 22b)

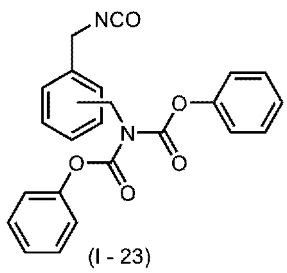

(I - 23)

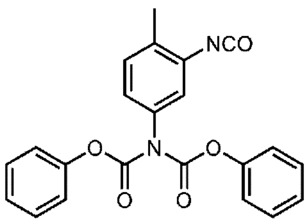

(I - 24a)

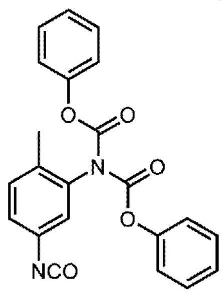

(I - 24b)

(Carbamate compound (III))

As the carbamate compound (III), compounds represented by Formulae (III-1a) to (III-24b), obtained by the above-described synthesis method, were used. In addition, for the carbamate compounds (III-1a) to (III-1c), the mixture (III-1) thereof was used, for the carbamate compounds (III-2a) to (III-2c), the mixture (III-2) thereof was used, for the carbamate compounds (III-3a) to (III-3c), the mixture (III-3) thereof was used, for the carbamate compounds (III-4a) to (III-4c), the mixture (III-4) thereof was used, for the carbamate compounds (III-5a) to (III-5b), the mixture (III-5) thereof was used, for the carbamate compounds (III-7a) to (III-7b), the mixture (III-7) thereof was used, for the carbamate compounds (III-8a) to (III-8b), the mixture (III-8) thereof was used, for the carbamate compounds (III-10a) to (III-10b), the mixture (III-10) thereof was used, for the carbamate compounds (III-22a) to (III-22b), the mixture (lll-11) thereof was used, and for the carbamate compounds (III-24a) to (III-24b), the mixture (III-24) thereof was used.

[Chem. 100]

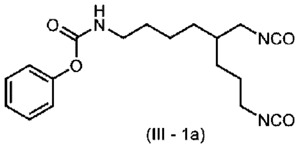
(III - 1a)
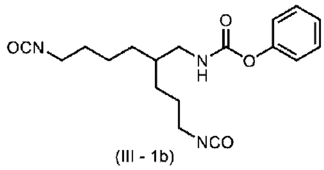
(III - 1b)
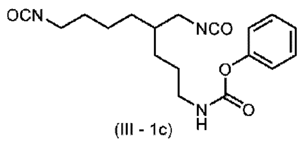
(III - 1c)
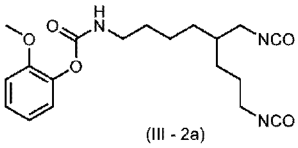
(III - 2a)
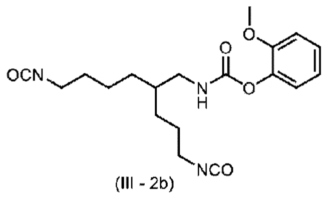
(III - 2b)
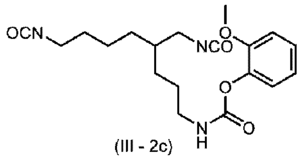
(III - 2c)
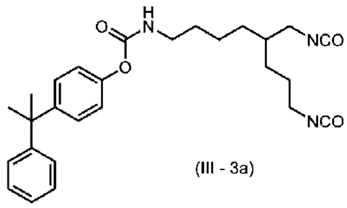
(III - 3a)
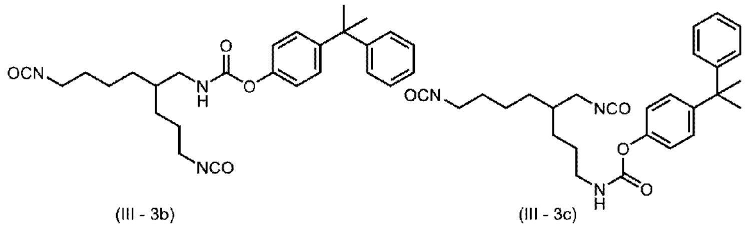
(III - 3b)
(III - 3c)

[Chem. 101]

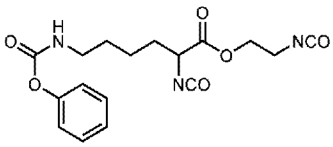
(III - 4a)
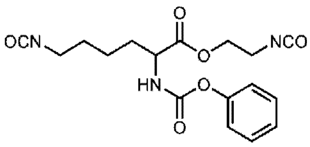
(III - 4b)
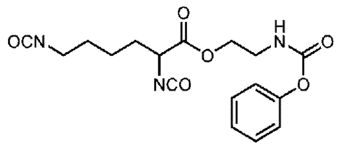
(III - 4c)
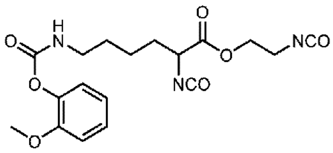
(III - 5a)
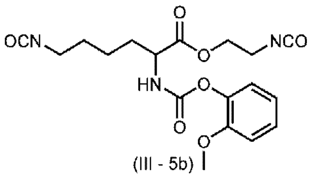
(III - 5b)
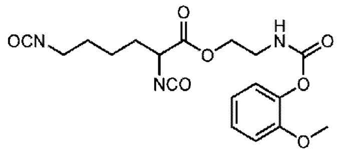
(III - 5c)

[Chem. 102]

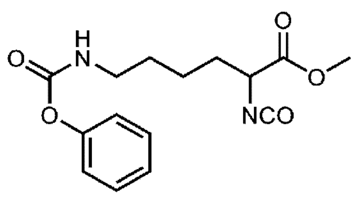
(III - 7a)
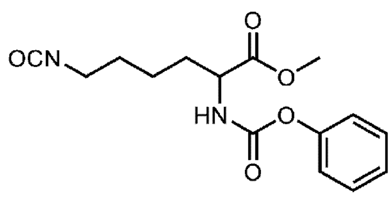
(III- 7b)
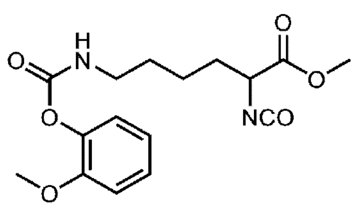
(III - 8a)
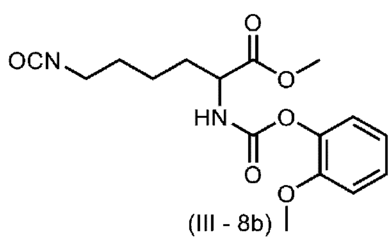
(III - 8b)

[Chem. 103]

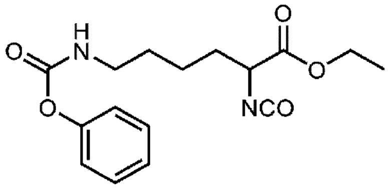

(III - 10a)

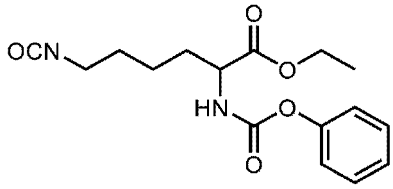

(III - 10b)

[Chem. 104]

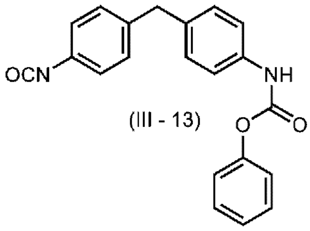

(III - 13)

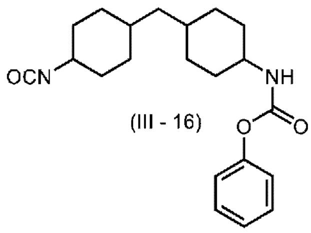

(III - 16)

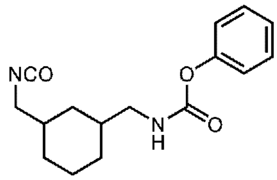

(III - 19)

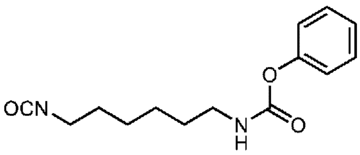

(III - 20)

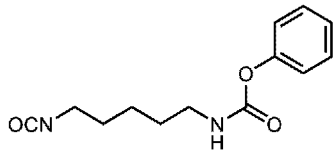

(III - 21)

[Chem. 105]

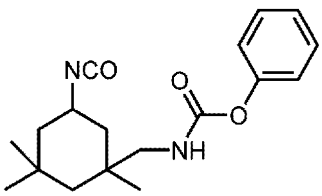

(III - 22a)

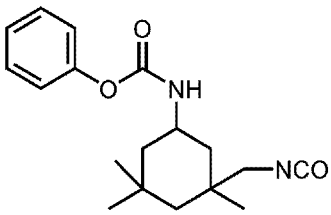

(III - 22b)

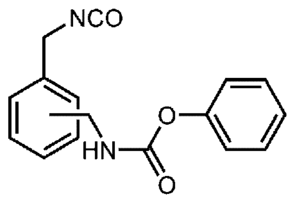

(III - 23)

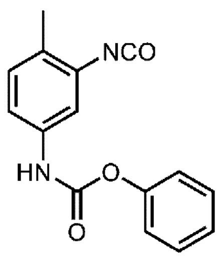

(III - 24a)

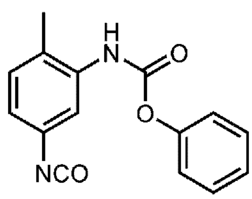

(III - 24b)

(Carbonate (IV))

As the carbonate (IV), compounds shown below were used.

DPC: Diphenyl carbonate

GAC: Bis(2-methoxyphenyl)carbonate
DPCP: Bis(4-cumylphenyl)carbonate

The above-described storage test was implemented on each of the obtained isocyanate compositions, and each evaluation was carried out. Results are shown in each table below.

[Table 2]

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 1 | A-a1 | TTI | 98 | (I-1) | 3.50E+00 | (III-1) | 3.20E+00 | DPC | 4.00E+00 | 1.10E+01 | 19 | 34 | 15 | 0.07 |
| Example 2 | A-a2 | TTI | 97 | (I-1) | 5.50E+00 | (III-1) | 4.70E+03 | DPC | 5.80E+02 | 5.30E+03 | 18 | 37 | 19 | 0.1 |
| Example 3 | A-a3 | TTI | 97 | (I-1) | 7.50E+03 | (III-1) | 4.20E+01 | DPC | 3.70E+00 | 7.50E+03 | 16 | 26 | 10 | 0.08 |
| Example 4 | A-a4 | TTI | 99 | (I-1) | 4.50E+03 | (III-1) | 2.50E+00 | DPC | 6.20E+03 | 1.10E+04 | 10 | 21 | 11 | 0.09 |
| Example 5 | A-a5 | TTI | 98 | (I-1) | 9.70E+03 | (III-1) | 9.60E+03 | DPC | 9.80E+03 | 2.90E+04 | 16 | 32 | 16 | 0.07 |
| Example 6 | A-a6 | TTI | 99 | (I-1) | 9.00E+03 | (III-1) | 1.00E+04 | DPC | 6.20E+03 | 2.50E+04 | 14 | 32 | 18 | 0.07 |
| Example 7 | A-a7 | TTI | 97 | (I-1) | 5.00E+03 | (III-1) | 1.20E+03 | DPC | 9.20E+03 | 1.50E+04 | 19 | 29 | 10 | 0.02 |
| Example 8 | A-a8 | TTI | 98 | (I-1) | 8.40E+03 | (III-1) | 9.10E+03 | DPC | 4.70E+03 | 2.20E+04 | 10 | 30 | 20 | 0.1 |
| Example 9 | A-a9 | TTI | 98 | (I-1) | 5.60E+03 | (III-1) | 9.00E+03 | DPC | 0.00E+00 | 1.50E+04 | 17 | 92 | 75 | 20 |
| Example 10 | A-a10 | TTI | 99 | (I-1) | 3.70E+03 | (III-1) | 0.00E+00 | DPC | 2.90E+03 | 6.70E+03 | 12 | 97 | 85 | 21 |
| Example 11 | A-a11 | TTI | 98 | (I-2) | 5.50E+00 | (III-2) | 6.00E+00 | GAC | 2.90E+00 | 1.40E+01 | 18 | 29 | 11 | 0.02 |
| Example 12 | A-a12 | TTI | 99 | (I-2) | 9.00E+00 | (III-2) | 8.30E+03 | GAC | 6.30E+03 | 1.50E+04 | 13 | 32 | 19 | 0.04 |
| Example 13 | A-a13 | TTI | 99 | (I-2) | 9.60E+02 | (III-2) | 9.40E+03 | GAC | 3.30E+00 | 1.00E+04 | 19 | 39 | 20 | 0.09 |
| Example 14 | A-a14 | TTI | 98 | (I-2) | 6.60E+03 | (III-2) | 4.50E+00 | GAC | 5.10E+03 | 1.20E+04 | 18 | 38 | 20 | 0.02 |

(continued)

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 15 | A-a15 | TTI | 98 | (I-2) | 9.40E+03 | (III-2) | 9.30E+03 | GAC | 9.20E+03 | 2.80E+04 | 14 | 28 | 14 | 0.04 |
| Example 16 | A-a16 | TTI | 99 | (I-2) | 9.00E+03 | (III-2) | 2.00E+03 | GAC | 3.00E+03 | 1.40E+04 | 17 | 34 | 17 | 0.07 |
| Example 17 | A-a17 | TTI | 99 | (I-2) | 3.70E+03 | (III-2) | 5.20E+03 | GAC | 9.90E+03 | 1.90E+04 | 19 | 31 | 12 | 0.07 |
| Example 18 | A-a18 | TTI | 99 | (I-2) | 3.50E+03 | (III-2) | 8.90E+03 | GAC | 6.00E+03 | 1.80E+04 | 13 | 25 | 12 | 0.09 |
| Example 19 | A-a19 | TTI | 98 | (I-2) | 9.00E+03 | (III-2) | 6.40E+02 | GAC | 0.00E+00 | 9.60E+03 | 12 | 87 | 75 | 10 |
| Example 20 | A-a20 | TTI | 98 | (I-2) | 4.50E+02 | (III-2) | 0.00E+00 | GAC | 1.30E+03 | 1.80E+03 | 15 | 108 | 93 | 14 |

[Table 3]

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 21 | A-a21 | TTI | 99 | (I-3) | 6.00E+00 | (III-3) | 2.90E+00 | DPCP | 3.00E+00 | 1.19E+01 | 13 | 24 | 11 | 0.01 |
| Example 22 | A-a22 | TTI | 99 | (I-3) | 8.50E+00 | (III-3) | 8.00E+03 | DPCP | 5.45E+03 | 1.35E+04 | 15 | 30 | 15 | 0.05 |
| Example 23 | A-a23 | TTI | 98 | (I-3) | 8.55E+02 | (III-3) | 8.50E+03 | DPCP | 2.50E+00 | 9.36E+03 | 18 | 34 | 16 | 0.07 |
| Example 24 | A-a24 | TTI | 99 | (I-3) | 6.02E+03 | (III-3) | 5.00E+00 | DPCP | 5.00E+03 | 1.10E+04 | 17 | 35 | 18 | 0.03 |
| Example 25 | A-a25 | TTI | 99 | (I-3) | 9.20E+03 | (III-3) | 9.00E+03 | DPCP | 9.40E+03 | 2.76E+04 | 12 | 24 | 12 | 0.05 |
| Example 26 | A-a26 | TTI | 99 | (I-3) | 8.50E+03 | (III-3) | 2.00E+03 | DPCP | 2.50E+03 | 1.30E+04 | 14 | 34 | 20 | 0.05 |
| Example 27 | A-a27 | TTI | 98 | (I-3) | 2.70E+03 | (III-3) | 4.50E+03 | DPCP | 9.50E+03 | 1.67E+04 | 13 | 25 | 12 | 0.04 |
| Example 28 | A-a28 | TTI | 99 | (I-3) | 4.00E+03 | (III-3) | 9.20E+03 | DPCP | 5.00E+03 | 1.82E+04 | 16 | 33 | 17 | 0.03 |

[Table 4]

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Comparative Example 1 | A-b 1 | TTI | 90 | (I-1) | 6.10E+03 | (III-1) | 8.10E+02 | DPC | 4.80E+03 | 1.20E+04 | 20 | 81 | 61 | 19 |
| Comparative Example 2 | A-b2 | TTI | 99 | (I-1) | 0.00E+00 | (III-1) | 4.60E+03 | DPC | 7.90E+03 | 1.30E+04 | 13 | 106 | 93 | 40 |
| Comparative Example 3 | A-b3 | TTI | 97 | (I-1) | 2.50E+04 | (III-1) | 7.40E+03 | DPC | 7.10E+02 | 3.30E+04 | 15 | 99 | 84 | 33 |
| Comparative Example 4 | A-b4 | TTI | 89 | (I-2) | 2.50E+02 | (III-2) | 3.10E+03 | GAC | 2.60E+03 | 6.00E+03 | 18 | 80 | 62 | 16 |
| Comparative Example 5 | A-b5 | TTI | 97 | (I-2) | 0.00E+00 | (III-2) | 3.10E+03 | GAC | 2.60E+03 | 5.70E+03 | 14 | 87 | 73 | 10 |
| Comparative Example 6 | A-b6 | TTI | 99 | (I-2) | 3.00E+04 | (III-2) | 6.40E+02 | GAC | 1.20E+02 | 3.10E+04 | 19 | 99 | 80 | 15 |

[Table 5]

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 29 | A-a29 | LTI | 98 | (I-4) | 5.50E+00 | (III-4) | 3.60E+00 | DPC | 4.40E+00 | 1.40E+01 | 15 | 30 | 15 | 0.04 |
| Example 30 | A-a30 | LTI | 97 | I1-4) | 3.00E+00 | (III-4) | 9.00E+03 | DPC | 6.70E+03 | 1.60E+04 | 11 | 26 | 15 | 0.02 |
| Example 31 | A-a31 | LTI | 99 | (I-4) | 9.10E+03 | (III-4) | 2.10E+03 | DPC | 2.50E+00 | 1.10E+04 | 19 | 38 | 19 | 0.04 |
| Example 32 | A-a32 | LTI | 98 | (I-4) | 1.60E+02 | (III-4) | 4.10E+00 | DPC | 7.30E+03 | 7.50E+03 | 14 | 33 | 19 | 0.08 |
| Example 33 | A-a33 | LTI | 97 | (I-4) | 9.80E+03 | (III-4) | 9.60E+03 | DPC | 9.10E+03 | 2.90E+04 | 12 | 22 | 10 | 0.04 |
| Example 34 | A-a34 | LTI | 99 | (I-4) | 9.70E+03 | (III-4) | 7.70E+02 | DPC | 2.50E+03 | 1.30E+04 | 17 | 37 | 20 | 0.04 |
| Example 35 | A-a35 | LTI | 97 | (I-4) | 5.20E+03 | (III-4) | 5.40E+03 | DPC | 9.60E+03 | 2.00E+04 | 17 | 32 | 15 | 0.1 |
| Example 36 | A-a36 | LTI | 97 | (I-4) | 8.30E+03 | (III-4) | 9.70E+03 | DPC | 5.60E+02 | 1.90E+04 | 10 | 23 | 13 | 0.03 |
| Example 37 | A-a37 | LTI | 99 | (I-5) | 2.50E+01 | (III-5) | 6.10E+02 | GAC | 1.50E+02 | 7.80E+02 | 15 | 28 | 13 | 0.02 |
| Example 38 | A-a38 | LDI | 97 | (I-7) | 2.50E+00 | (III-7) | 4.00E+00 | DPC | 3.00E+00 | 9.50E+00 | 11 | 28 | 17 | 0.1 |
| Example 39 | A-a39 | LDI | 99 | (I-7) | 2.10E+00 | (III-7) | 3.20E+03 | DPC | 4.70E+03 | 7.90E+03 | 19 | 34 | 15 | 0.07 |

(continued)

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 40 | A-a40 | LDI | 97 | (I-7) | 3.40E+03 | (III-7) | 5.80E+03 | DPC | 3.30E+00 | 9.20E+03 | 15 | 31 | 16 | 0.02 |
| Example 41 | A-a41 | LDI | 99 | (I-7) | 5.10E+02 | (III-7) | 4.00E+00 | DPC | 6.00E+03 | 6.50E+03 | 10 | 22 | 12 | 0.07 |
| Example 42 | A-a42 | LDI | 99 | (I-7) | 9.50E+03 | (III-7) | 9.60E+03 | DPC | 9.10E+03 | 2.80E+04 | 13 | 24 | 11 | 0.05 |
| Example 43 | A-a43 | LDI | 99 | (I-7) | 9.10E+03 | (III-7) | 7.50E+03 | DPC | 3.10E+03 | 2.00E+04 | 15 | 27 | 12 | 0.08 |
| Example 44 | A-a44 | LDI | 98 | (I-7) | 6.60E+03 | (III-7) | 7.60E+02 | DPC | 9.70E+03 | 1.70E+04 | 15 | 27 | 12 | 0.07 |
| Example 45 | A-a45 | LDI | 98 | (I-7) | 7.20E+02 | (III-7) | 9.00E+03 | DPC | 7.60E+03 | 1.70E+04 | 10 | 20 | 10 | 0.03 |
| Example 46 | A-a46 | LDI | 99 | (I-8) | 1.50E+02 | (III-8) | 3.30E+03 | GAC | 2.20E+02 | 3.70E+03 | 12 | 22 | 10 | 0.05 |

[Table 6]

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 47 | A-a47 | LDI-Et | 97 | (I-10) | 2.10E+00 | (III-10) | 3.50E+00 | DPC | 3.20E+00 | 8.80E+00 | 12 | 22 | 10 | 0.05 |
| Example 48 | A-a48 | LDI-Et | 99 | (I-10) | 2.50E+00 | (III-10) | 3.20E+03 | DPC | 4.20E+03 | 7.40E+03 | 18 | 30 | 12 | 0.05 |
| Example 49 | A-a49 | LDI-Et | 97 | (I-10) | 3.40E+03 | (III-10) | 5.00E+03 | DPC | 2.50E+00 | 8.40E+03 | 16 | 31 | 15 | 0.04 |
| Example 50 | A-a50 | LDI-Et | 99 | (I-10) | 5.00E+02 | (III-10) | 5.00E+00 | DPC | 7.00E+03 | 7.51E+03 | 14 | 25 | 11 | 0.06 |
| Example 51 | A-a51 | LDI-Et | 99 | (I-10) | 9.20E+03 | (III-10) | 9.50E+03 | DPC | 9.20E+03 | 2.79E+04 | 12 | 25 | 13 | 0.03 |
| Example 52 | A-a52 | LDI-Et | 99 | (I-10) | 9.00E+03 | (III-10) | 7.00E+03 | DPC | 3.20E+03 | 1.92E+04 | 11 | 25 | 14 | 0.02 |
| Example 53 | A-a53 | LDI-Et | 98 | (I-10) | 6.50E+03 | (III-10) | 7.00E+02 | DPC | 9.00E+03 | 1.62E+04 | 12 | 24 | 12 | 0.01 |
| Example 54 | A-a54 | LDI-Et | 98 | (I-10) | 5.00E+02 | (III-10) | 9.00E+03 | DPC | 6.50E+03 | 1.60E+04 | 13 | 26 | 13 | 0.08 |
| Example 55 | A-a55 | HDI | 98 | (I-20) | 9.00E+03 | (III-20) | 3.50E+03 | DPC | 9.30E+03 | 2.20E+04 | 14 | 33 | 19 | 0.03 |
| Example 56 | A-a56 | HMDI | 97 | (I-16) | 8.70E+03 | (III-16) | 4.40E+02 | DPC | 9.70E+01 | 9.20E+03 | 13 | 26 | 13 | 0.07 |
| Example 57 | A-a57 | HXDI | 98 | (I-19) | 2.10E+03 | (III-19) | 5.40E+03 | DPC | 1.40E+03 | 8.90E+03 | 14 | 27 | 13 | 0.07 |

(continued)

| Isocyanate composition | | Composition | | | | | | | | | Evaluation | | | |
|---|---|---|---|---|---|---|---|---|---|---|---|---|---|---|
| | | Isocyanate compound (II) | | Carbonyl compound (I) | | Carbamate compound (III) | | Carbonate (IV) | | (I) + (III) + (IV) Content (ppm by mass) | Immediately after production | After storage test | | |
| | | Type | Content (% by mass) | Type | Content (ppm by mass) | Type | Content (ppm by mass) | Type | Content (ppm by mass) | | Hazen color number | Hazen color number | Δ Hazen color number (before and after storage) | Amount of denaturation (% by mass) |
| Example 58 | A-a58 | PDI | 97 | (I-21) | 3.40E+03 | (III-21) | 1.30E+03 | DPC | 4.50E+03 | 9.20E+03 | 12 | 22 | 10 | 0.07 |
| Example 59 | A-a59 | IPDI | 98 | (I-22) | 1.40E+03 | (III-22) | 8.90E+03 | DPC | 1.80E+03 | 1.20E+04 | 11 | 23 | 12 | 0.06 |
| Example 60 | A-a60 | XDI | 99 | (I-23) | 7.60E+03 | (III-23) | 4.80E+03 | DPC | 6.00E+03 | 1.80E+04 | 20 | 36 | 16 | 0.08 |
| Example 61 | A-a61 | MDI | 98 | (I-13) | 8.50E+03 | (III-I3) | 9.20E+03 | DPC | 6.30E+03 | 2.40E+04 | 22 | 36 | 14 | 0.05 |
| Example 62 | A-a62 | TDI | 99 | (I-24) | 7.00E+03 | (III-24) | 6.60E+03 | DPC | 5.50E+03 | 1.90E+04 | 25 | 38 | 13 | 0.05 |

From each of the tables above, in isocyanate compositions A-a1 to A-a62 (Examples 1 to 62) containing the isocyanate compound (II) and the carbonyl compound (I) in specific amounts, coloring was sufficiently suppressed, and storage stability was excellent.

In addition, in isocyanate compositions A-a1 to A-a8, A-a11 to A-a18, and A-a21 to A-a62 (Examples 1 to 8, 11 to 18, and 21 to 62) further containing a carbamate compound (III) and a carbonate (IV), in addition to the isocyanate compound (II) and the carbonyl compound (I), a change in color difference before and after the storage was smaller, the amount of denaturation was smaller, and the storage stability was particularly excellent.

On the other hand, in isocyanate compositions A-b1 and A-b4 (Comparative Examples 1 and 4) in which a content of the isocyanate compound (II) was less than 97% by mass with respect to a total mass of the isocyanate composition, the change in color difference before and after storage was large, the amount of denaturation was large, and the storage stability was poor.

In addition, in isocyanate compositions A-b2, A-b3, A-b5, and A-b6 (Comparative Examples 2, 3, 5, and 6) in which the carbonyl compound (I) was not contained, or the content of the carbonyl compound (I) was more than $1.0 \times 10^4$ ppm by mass with respect to the total mass of the isocyanate composition, the change in color difference before and after storage was large, the amount of denaturation was also large, and the storage stability was poor.

[Industrial Applicability]

According to the carbonyl compound and the method for producing thereof of the present embodiment, it is possible to provide a novel carbonyl compound. The method for producing an isocyanate compound of the present embodiment is a method using the carbonyl compound, and thus it is possible to prevent a by-product from being fixed to a device at the time of production of the isocyanate compound and to improve a yield of the isocyanate compound.

In addition, according to the isocyanate composition of the present embodiment, it is possible to provide an isocyanate composition in which coloring is sufficiently suppressed and the storage stability is excellent.

[Reference Signs List]

11, 12, 13, 14, 15, 16, 17, 21, 22, 23, 24, 25, 26, 27, 28, 31, 32, 33, 34, 35, 36, 41, 42, 43: Line
A11, A21, A31, A41: Capacitor
A32: Reboiler
101, 102, 103, 106, 107, 202, 204, 205, 302, 303, 402, 403: Storage tank
104, 105, 201: Reaction container made of SUS with a baffle
203, 301: Multi-stage distillation column
401: Thin-film distillation device

## Claims

1. A carbonyl compound represented by General Formula (I),

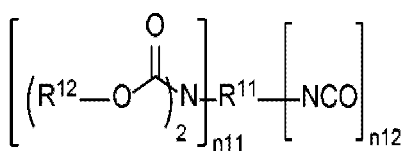

(I)

(in General Formula (1), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less).

2. The carbonyl compound according to Claim 1,

wherein $R^{11}$ is a di- or higher valent and tetra- or lower valent aliphatic hydrocarbon group having 1 or more and 20 or less carbon atoms, or a di- or higher valent and tri- or lower valent aromatic hydrocarbon group having 6 or more and 20 or less carbon atoms, which may have 1 or more and 4 or less ester groups or nitrogen atoms, and $R^{12}$ is a monovalent aromatic hydrocarbon group having 6 or more and 20 or less carbon atoms, which may

contain an oxygen atom.

**3.** The carbonyl compound according to Claim 1 or 2,
wherein $R^{11}$ is a di- or higher valent and tetra- or lower valent aliphatic hydrocarbon group having 5 or more and 15 or less carbon atoms, or a di- or higher valent and tri- or lower valent aromatic hydrocarbon group having 6 or more and 15 or less carbon atoms, which may have 1 or more and 2 or less ester groups, $R^{12}$ is a monovalent aromatic hydrocarbon group having 6 or more and 15 or less carbon atoms, which may contain an oxygen atom, $n^{11}$ is an integer of 1 or more and 4 or less, $n^{12}$ is an integer of 0 or more and 3 or less, and a sum of $n^{11}$ and $n^{12}$ is an integer of 2 or more and 4 or less.

**4.** A method for producing the carbonyl compound according to any one of Claims 1 to 3, the production method comprising:

mixing one or more compounds selected from the group consisting of an isocyanate compound and a carbamate compound with one or more compounds selected from the group consisting of a carbonate and a hydroxy compound, and
heating the mixture to synthesize the carbonyl compound.

**5.** The production method according to Claim 4,

wherein the isocyanate compound is a compound represented by General Formula (II)

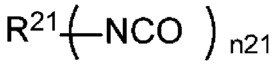

( II )

(in General Formula (II), $R^{21}$ is an n21-valent organic group, and satisfies a relational formula: $R^{21} = R^{11}$, and n21 is an integer of 2 or more and 8 or less, and satisfies a relational formula: n21 = n11 + n12).

**6.** The production method according to Claim 4 or 5,

wherein the carbamate compound is a compound represented by General Formula (III),

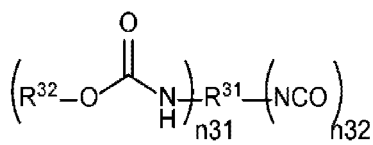

( III )

(in General Formula (III), $R^{31}$ is an (n31 + n32)-valent organic group, and satisfies a relational formula: $R^{31} = R^{11}$, $R^{32}$ is a monovalent organic group, and satisfies a relational formula: $R^{32} = R^{12}$, n31 is an integer of 1 or more and 8 or less, n32 is an integer of 0 or more and 7 or less, and a sum of n31 and n32 is an integer of 2 or more and 8 or less, and satisfies a relational formula: n31 + n32 = n11 + n12).

**7.** The production method according to any one of Claims 4 to 6,

wherein the carbonate is a compound represented by General Formula (IV),

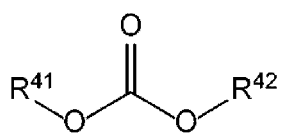

( IV )

(in General Formula (IV), $R^{41}$ and $R^{42}$ are each independently a monovalent organic group, and satisfy a relational formula: $R^{41} = R^{42} = R^{12}$).

**8.** The production method according to any one of Claims 4 to 7,

wherein the hydroxy compound is a compound represented by General Formula (V),

$R^{51}$-OH (V)

(in General Formula (V), $R^{51}$ is a monovalent organic group, and satisfies a relational formula: $R^{51} = R^{12}$).

**9.** A method for producing an isocyanate compound, comprising:

performing distillation purification on a reaction solution including an isocyanate compound represented by General Formula (II) in the presence of the carbonyl compound according to any one of Claims 1 to 3; and continuously recovering the isocyanate compound as a gas-phase component,

$$R^{21}\left(-NCO\right)_{n21} \quad (II)$$

(in General Formula (II), $R^{21}$ is an n21-valent organic group, and satisfies a relational formula: $R^{21} = R^{11}$, and n21 is an integer of 2 or more and 8 or less, and satisfies a relational formula: n21 = n11 + n12).

**10.** An isocyanate composition comprising: with respect to a total mass of the isocyanate composition,

97% by mass or more of an isocyanate compound; and
2.0 ppm by mass or more and $1.0 \times 10^4$ ppm by mass or less of a carbonyl compound represented by General Formula (I),
wherein the isocyanate compound and the carbonyl compound are different compounds from each other,

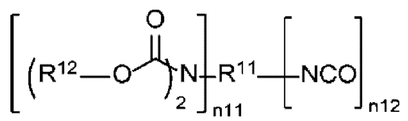

(I)

(in General Formula (I), $R^{11}$ is an (n11 + n12)-valent organic group, $R^{12}$ is a monovalent organic group, n11 is an integer of 1 or more and 8 or less, n12 is an integer of 0 or more and 7 or less, and a sum of n11 and n12 is an integer of 2 or more and 8 or less).

**11.** The isocyanate composition according to Claim 10,

wherein $R^{11}$ is a di- or higher valent and tetra- or lower valent aliphatic hydrocarbon group having 1 or more and 20 or less carbon atoms, or a di- or higher valent and tri- or lower valent aromatic hydrocarbon group having 6 or more and 20 or less carbon atoms, which may have 1 or more and 4 or less ester groups or nitrogen atoms, and $R^{12}$ is a monovalent aromatic hydrocarbon group having 6 or more and 20 or less carbon atoms, which may contain an oxygen atom.

**12.** The isocyanate composition according to Claim 10 or 11,

wherein the isocyanate compound is a compound represented by General Formula (II),

$$R^{21}\left(-NCO\right)_{n21} \quad (II)$$

(in General Formula (II), $R^{21}$ is an n21-valent organic group, and satisfies a relational formula: $R^{21} = R^{11}$, and n21 is an integer of 2 or more and 8 or less, and satisfies a relational formula: n21 = n11 + n12).

**13.** The isocyanate composition according to any one of Claims 10 to 12, further comprising:
2.0 ppm by mass or more and $1.0 \times 10^4$ ppm by mass or less of one or more compounds selected from the group consisting of a carbamate compound and a carbonate with respect to the total mass of the isocyanate composition.

**14.** The isocyanate composition according to Claim 13,

wherein the carbamate compound is a compound represented by General Formula (III),

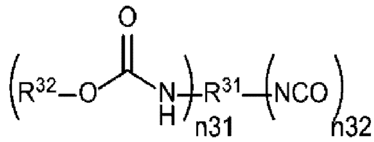

( III )

(in General Formula (III), $R^{31}$ is an (n31 + n32)-valent organic group, and satisfies a relational formula: $R^{31} = R^{11}$, $R^{32}$ is a monovalent organic group, and satisfies a relational formula: $R^{32} = R^{12}$, n31 is an integer of 1 or more and 8 or less, n32 is an integer of 0 or more and 7 or less, and a sum of n31 and n32 is an integer of 2 or more and 8 or less, and satisfies a relational formula: n31 + n32 = n11 + n12).

**15.** The isocyanate composition according to Claim 13 or 14,

wherein the carbonate is a compound represented by General Formula (IV),

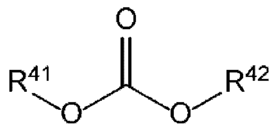

( IV )

(in General Formula (IV), $R^{41}$ and $R^{42}$ are each independently a monovalent organic group, and satisfy a relational formula: $R^{41} = R^{42} = R^{12}$).

*FIG. 1*

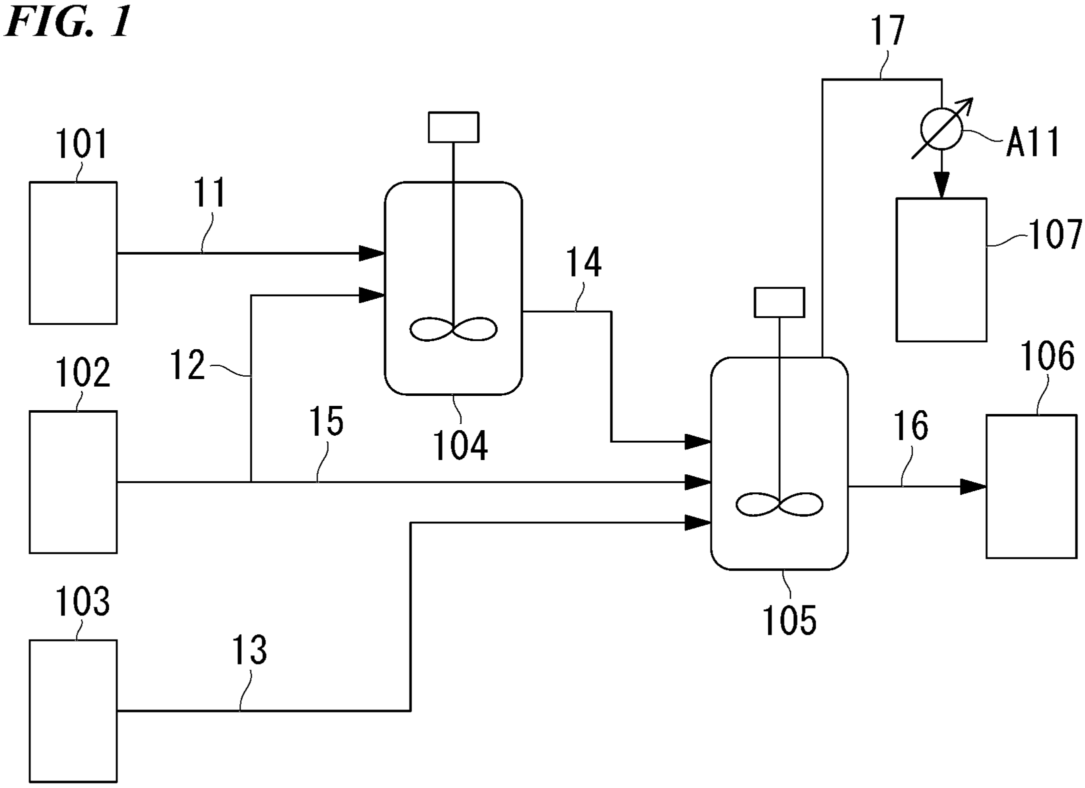
17
101
A11
11
107
14
12
102
15
104
106
16
103
105
13

*FIG. 2*

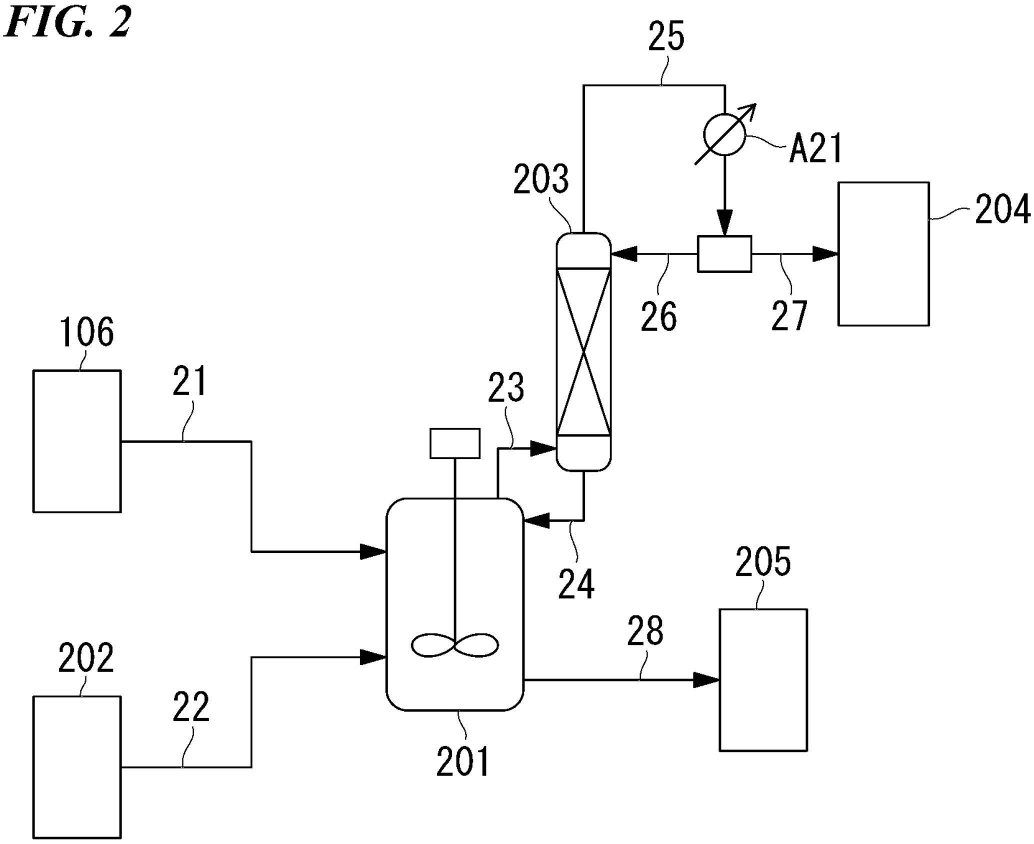
25
A21
203
204
26
27
106
21
23
24
205
28
202
22
201

FIG. 3

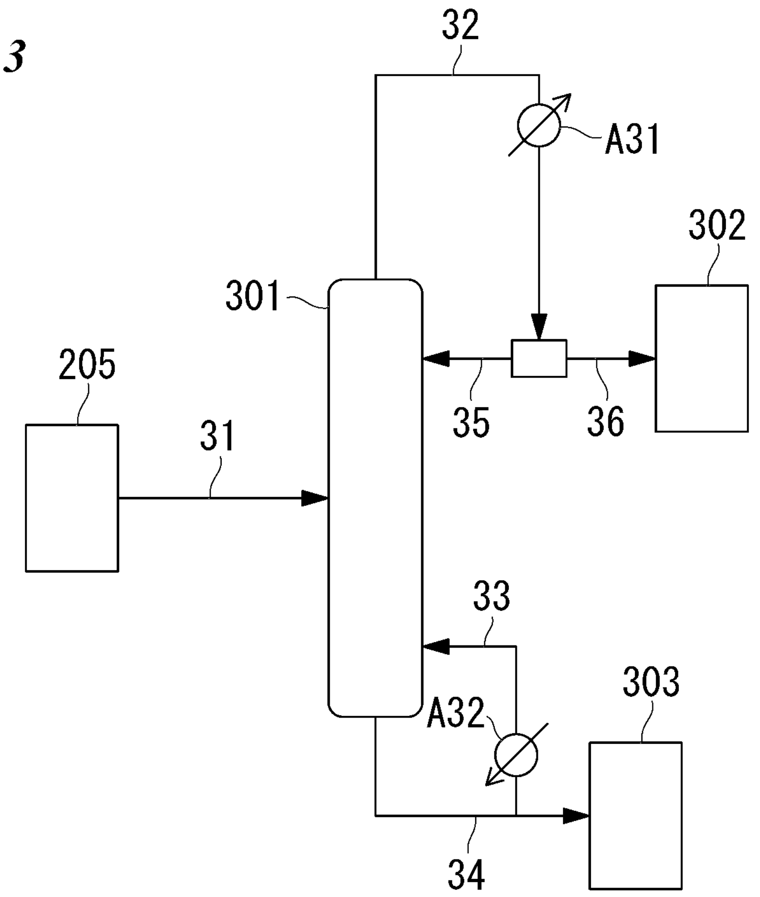
32
A31
302
301
205
35
36
31
33
303
A32
34

FIG. 4

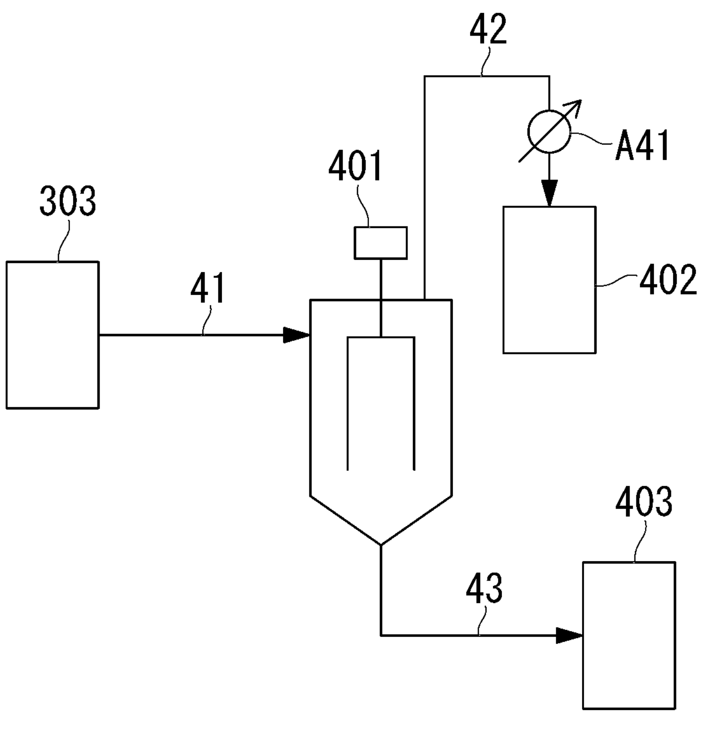
42
A41
401
303
402
41
403
43

## *FIG. 5*

(I-1a)

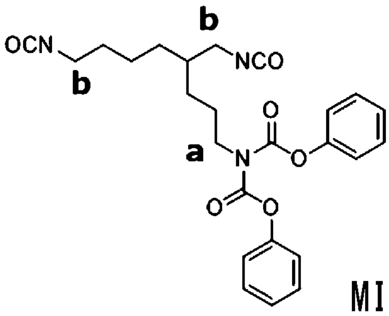

(I-1b)

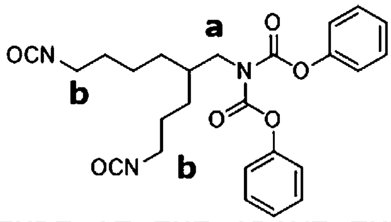

(I-1c)

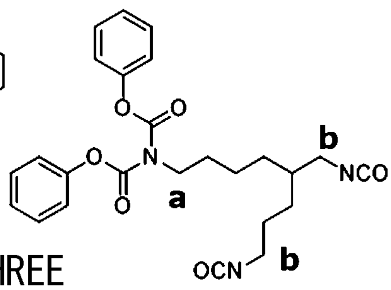

MIXTURE OF THE ABOVE THREE
CARBONYL COMPOUNDS

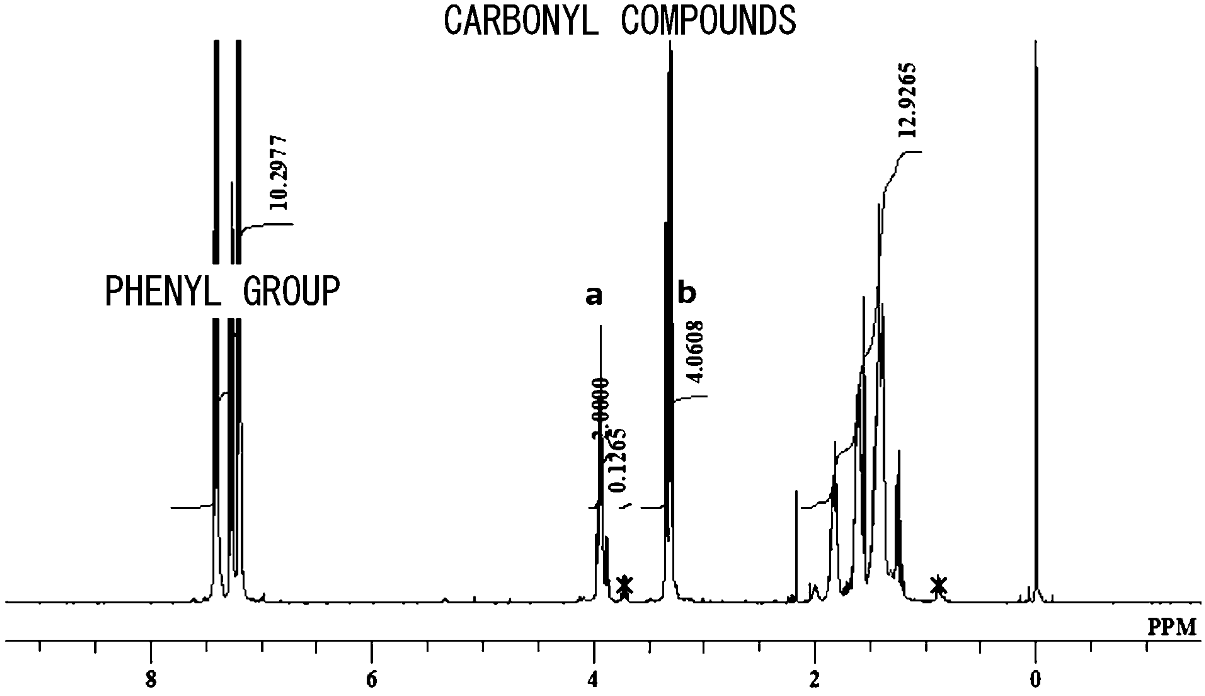

*FIG. 6A*

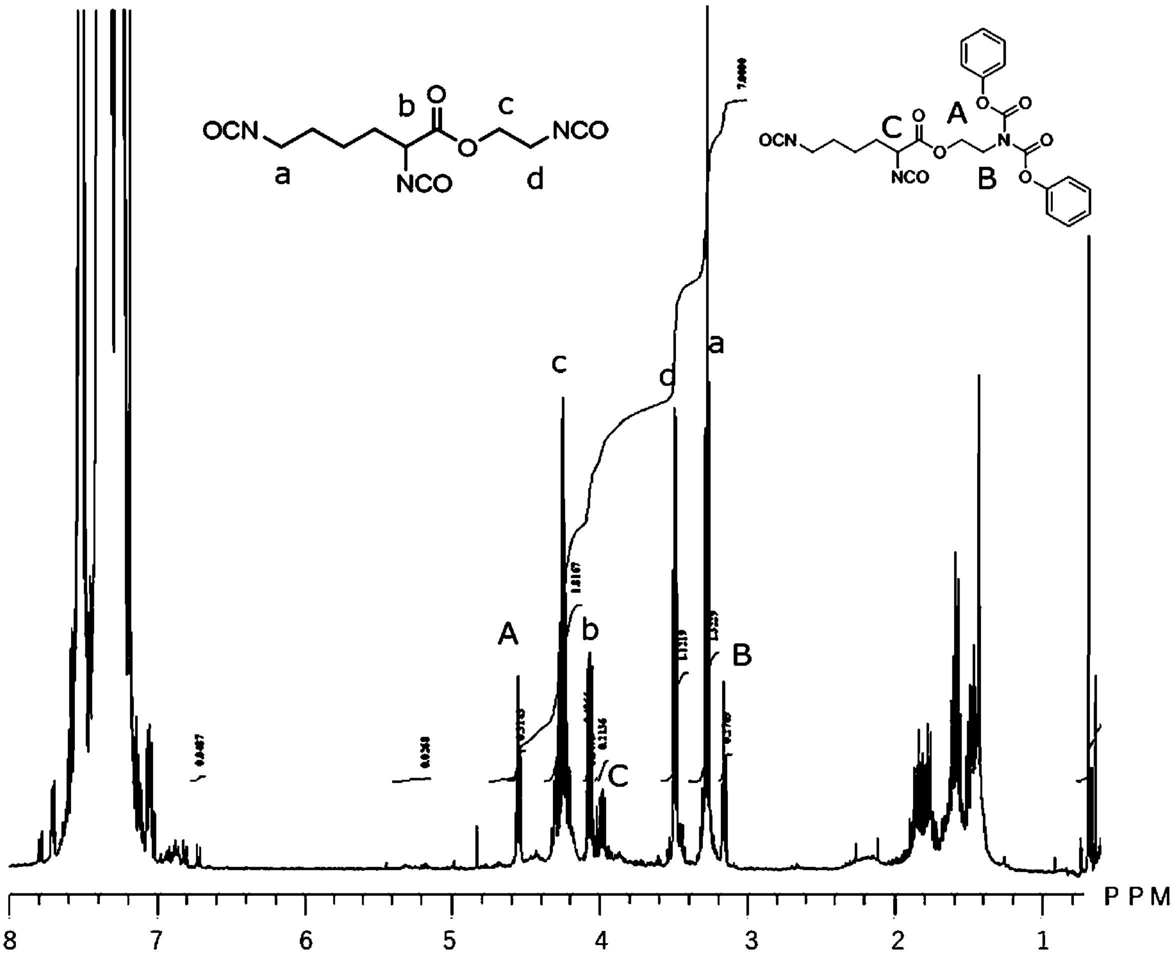

*FIG. 6B*

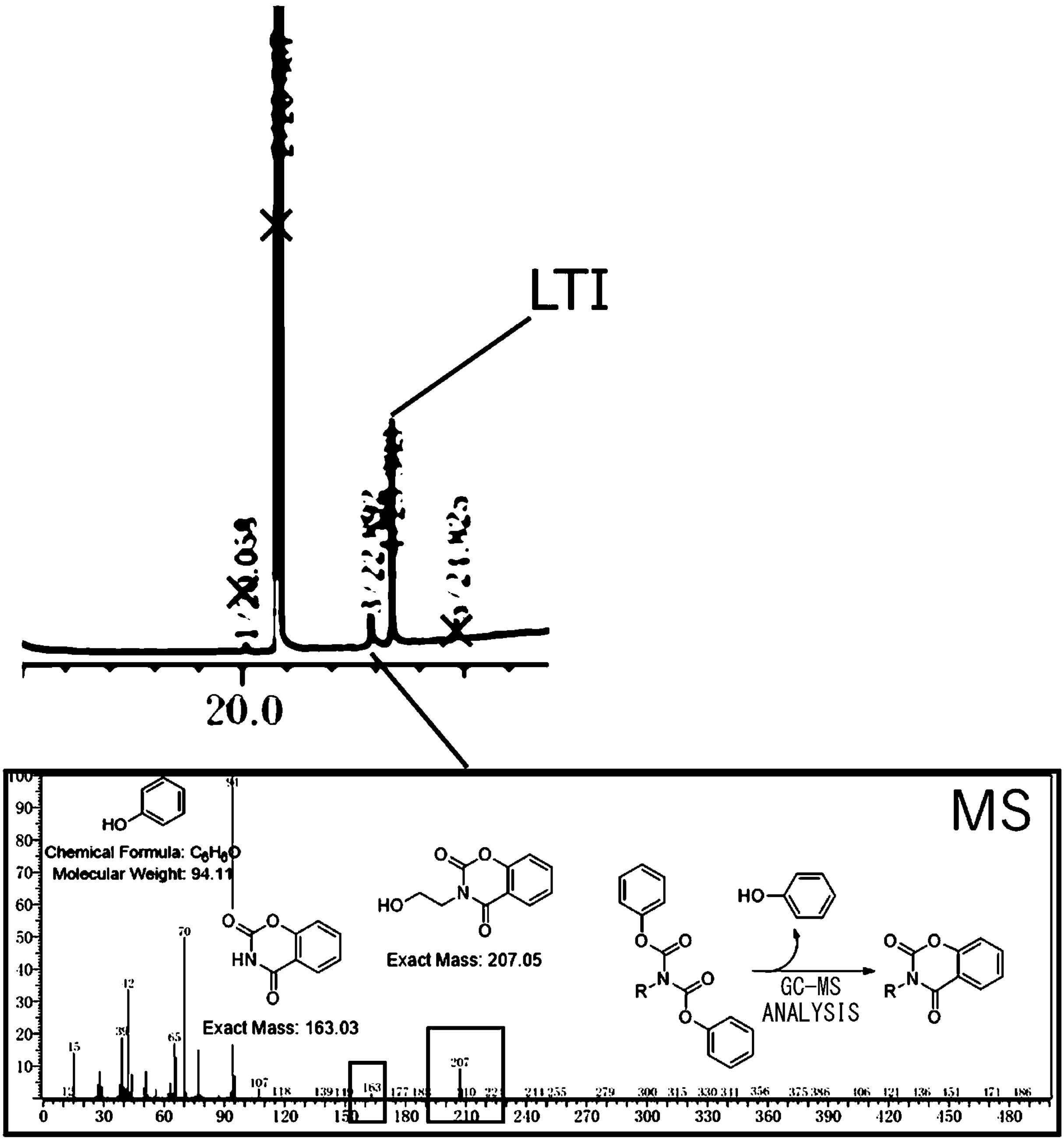
LTI
20.0
MS
Chemical Formula: C6H6O
Molecular Weight: 94.11
Exact Mass: 163.03
Exact Mass: 207.05
GC-MS
ANALYSIS

**INTERNATIONAL SEARCH REPORT**

International application No.
**PCT/JP2022/041614**

**A. CLASSIFICATION OF SUBJECT MATTER**

***C07C 265/14***(2006.01)i; ***C07C 269/00***(2006.01)i; ***C07C 269/08***(2006.01)i; ***C07C 271/52***(2006.01)i
FI: C07C271/52 CSP; C07C269/00; C07C269/08; C07C265/14

According to International Patent Classification (IPC) or to both national classification and IPC

**B. FIELDS SEARCHED**

Minimum documentation searched (classification system followed by classification symbols)

C07C265/00; C07C269/00; C07C271/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2003-002834 A (TANABE SEIYAKU CO., LTD.) 08 January 2003 (2003-01-08) p. 47, compound 4 | 1 |
| Y | | 4-8 |
| A | | 9-15 |
| X | ENGLUND, Ethan A. et al. An Efficient Synthesis of a Probe for Protein Function: 2,3-Diaminopropionic Acid with Orthogonal Protecting Groups, Organic Letters, 2004, 6(2), pp. 213-215 scheme 1, compound 8 | 1 |
| Y | | 4-8 |
| A | | 9-15 |
| X | JP 2008-526761 A (ALANTOS PHARMACEUTICALS, INC.) 24 July 2008 (2008-07-24) p. 211, product of step D | 1 |
| Y | | 4-8 |
| A | | 9-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 January 2023** | **07 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/041614**

**C. DOCUMENTS CONSIDERED TO BE RELEVANT**

| Category* | Citation of document, with indication, where appropriate, of the relevant passages | Relevant to claim No. |
|---|---|---|
| X | WO 2015/030106 A1 (KYOTO UNIVERSITY) 05 March 2015 (2015-03-05) p. 51, compound 14a, p. 57, compound 14b | 1 |
| Y | | 4-8 |
| A | | 9-15 |
| X | CN 112661930 A (WANHUA CHEMICAL GROUP CO., LTD.) 16 April 2021 (2021-04-16) paragraphs [0092], [0093], compound a | 1-3 |
| Y | | 4-8 |
| A | | 9-15 |
| X | CN 113024563 A (CHINA PHARMACEUTICAL UNIVERSITY) 25 June 2021 (2021-06-25) paragraph [0556], compound 14 | 1 |
| Y | | 4-8 |
| A | | 9-15 |

Form PCT/ISA/210 (second sheet) (January 2015)

**INTERNATIONAL SEARCH REPORT**
**Information on patent family members**

International application No.
**PCT/JP2022/041614**

| Patent document cited in search report | Publication date (day/month/year) | Patent family member(s) | Publication date (day/month/year) |
|---|---|---|---|
| JP 2003-002834 A | 08 January 2003 | (Family: none) | |
| JP 2008-526761 A | 24 July 2008 | US 2006/0173183 A1<br>p. 124, product of step D<br>EP 1843820 A1<br>CN 101137414 A | |
| WO 2015/030106 A1 | 05 March 2015 | (Family: none) | |
| CN 112661930 A | 16 April 2021 | (Family: none) | |
| CN 113024563 A | 25 June 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4324879 A
- US 4412073 A
- JP S57047319 A
- JP S63057577 A
- US 3976622 A
- US 4176132 A
- US 4290969 A
- US 4837359 A
- US 4983762 A
- US 5641851 A
- GB 994890 A
- JP H07304724 A
- JP H02228317 A
- JP H08291129 A
- JP 2012506465 W
- JP 3071008 B
- JP 4137941 B


### Non-patent literature cited in the description


- **DRITTER JAHRGANG.** 186. A. W. Hofmann: Ueber die aromatischen Cyanate. *Berchte der Deutechen Chemischen Gesellschaft,* vol. 3, 653-658
- **DYER E et al.** Thermal Degradation of Alkyl N-Phenylcarbomates. *Journal of American Chemical Society,* 1959, vol. 81, 2138-2143
- **ULRICH H et al.** 2+2] Cycloaddition Reactions of Unsymmetrically substituted Carbodiimides. *Journal of Heterocyclic Chemistry,* 1987, vol. 24, 1121-1123
- **SCHWETLICK K et al.** Kinetics and Catalysis of Consecutive Isocyanate Reactions. Formation of Carbamates, Allophanates and Isocyanurates. *Journal of the Chemical Society, Perkin transactions II,* 1995, vol. 2, 395-402
- Organic Chemistry and Biochemical Naming Method. Nankodo Co., Ltd, 1992
- *Chemistry Field,* 1980